(19) Europäisches Patentamt / European Patent Office / Office européen des brevets

(11) **EP 3 710 869 B1**

(12) **FASCICULE DE BREVET EUROPEEN**

(45) Date de publication et mention de la délivrance du brevet:
**27.10.2021 Bulletin 2021/43**

(21) Numéro de dépôt: **18799791.1**

(22) Date de dépôt: **15.11.2018**

(51) Int Cl.:
*G01V 3/08* (2006.01)          *A61B 5/06* (2006.01)
*G01B 7/004* (2006.01)        *G01B 21/04* (2006.01)
*A61B 5/00* (2006.01)          *A61B 34/20* (2016.01)

(86) Numéro de dépôt international:
**PCT/EP2018/081309**

(87) Numéro de publication internationale:
**WO 2019/096877 (23.05.2019 Gazette 2019/21)**

(54) **PROCÉDÉ DE COMPENSATION D'UN LOCALISATEUR MAGNÉTIQUE, LOCALISATEUR ET PROGRAMME D'ORDINATEUR**

VERFAHREN ZUR KOMPENSATION EINES MAGNETISCHEN LOKALISATORS, LOKALISATOR UND COMPUTERPROGRAMM

METHOD FOR COMPENSATING A MAGNETIC LOCATOR, LOCATOR AND COMPUTER PROGRAM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.11.2017 FR 1760773**

(43) Date de publication de la demande:
**23.09.2020 Bulletin 2020/39**

(73) Titulaire: **MINMAXMEDICAL**
**38610 Gières (FR)**

(72) Inventeurs:
- **HUGUEL, Loïc**
  **38950 Saint Martin Le Vinoux (FR)**
- **ROUSSEAU, Sandra**
  **38000 Grenoble (FR)**
- **CHAVE, Mickaël**
  **38120 Saint Egreve (FR)**

(74) Mandataire: **Regimbeau**
**Parc d'affaires Cap Nord A**
**2, allée Marie Berhaut**
**CS 71104**
**35011 Rennes Cedex (FR)**

(56) Documents cités:
**US-A1- 2003 200 052     US-A1- 2005 246 122**
**US-A1- 2011 004 430**

- **VAALS J J VAN ET AL: "OPTIMIZATION OF EDDY-CURRENT COMPENSATION*", JOURNAL OF MAGNETIC RESONANCE, ACADEMIC PRESS, ORLANDO, FL, US, vol. 90, no. 1, 15 octobre 1990 (1990-10-15), pages 52-70, XP000175881, ISSN: 1090-7807**

EP 3 710 869 B1

**Description**

L'invention concerne un procédé de compensation d'un localisateur magnétique en présence d'au moins un matériau perturbateur.

Un localisateur magnétique comporte généralement un dispositif d'émission, dit émetteur, ayant une ou plusieurs bobines émettrices rigidement liées les unes aux autres, et un dispositif de réception, dit récepteur, ayant une ou plusieurs bobines réceptrices rigidement liées les unes aux autres. L'analyse conjointe des champs magnétiques émis par les bobines émettrices et des champs magnétiques mesurés par les bobines réceptrices permet de déterminer par une unité de traitement la position et/ou l'orientation du dispositif de réception par rapport au dispositif d'émission. Cette unité de traitement comporte un modèle de champ permettant de calculer la position et/ou l'orientation du récepteur par calcul d'une prédiction des mesures en fonction d'un critère de minimisation d'une erreur calculée par rapport aux mesures.

Le document US2011/004430A1 décrit un procédé de compensation d'un localisateur magnétique selon le préambule de la revendication 1.

Le problème de l'invention est qu'il peut exister un matériau perturbateur du champ magnétique entre l'émetteur et le récepteur. En particulier, certains matériaux conducteurs de l'électricité peuvent donner naissance à des courants de Foucault, lorsque ces matériaux sont placés dans un champ magnétique. Les courants de Foucault qui circulent alors dans ce matériau conducteur engendrent à leur tour un champ magnétique perturbateur.

Ainsi, la présence d'un matériau perturbateur fausse le calcul de la prédiction par l'unité de traitement.

L'invention vise à résoudre ce problème en proposant un procédé et un dispositif de compensation d'un localisateur magnétique en présence d'au moins un matériau perturbateur, qui permettent de calculer la bonne position et/ou orientation du récepteur correspondant le plus possible à sa position réelle et/ou à son orientation réelle, malgré le champ magnétique perturbateur induit par le matériau.

A cet effet, un premier objet de l'invention est un procédé de compensation d'un localisateur magnétique en présence d'au moins un matériau perturbateur de champ magnétique, selon la revendication 1.

Un deuxième objet de l'invention est un procédé de compensation d'un localisateur magnétique en présence d'au moins un matériau perturbateur de champ magnétique, selon la revendication 2.

Un troisième objet de l'invention est un procédé de compensation d'un localisateur magnétique en présence d'au moins un matériau perturbateur de champ magnétique, selon la revendication 3.

Un quatrième objet de l'invention est un procédé de compensation d'un localisateur magnétique en présence d'au moins un matériau perturbateur de champ magnétique, selon la revendication 4.

Les revendications 5 à 9 concernent des modes de réalisation de ces procédés de compensation.

On décrit ci-dessous d'autres modes de réalisation.

Suivant un autre mode de réalisation de l'invention, à chaque itération

- on initialise la prédiction Hi aux valeurs initiales prescrites,
- le paramètre $\beta$ est alors ajouté comme variable d'état de l'algorithme de minimisation de Levenberg-Marquardt,
- puis on calcule les $\Delta_i$ en fonction de Hi et Ipi et $\beta$ selon les équations :

$$\mathrm{Hp_i} = \mathrm{H_i}.(1+\Delta_i.(j+ \beta.\omega_i)),$$

les $\Delta_i$ étant une solution au système d'équations suivant :

$$\mathcal{R}(\mathrm{H_i}) - \mathcal{R}(\mathrm{Ip_i}) + \Delta_i.(\mathcal{R}(\mathrm{H_i}).\beta. \omega_i - \mathcal{I}(\mathrm{H_i})) = 0$$

et

$$\mathcal{I}(\mathrm{H_i}) - \mathcal{I}(\mathrm{Ip_i}) + \Delta_i.(\mathcal{R}(\mathrm{H_i}) + \mathcal{I}(\mathrm{H_i}).\beta. \omega_i) = 0$$

pour i allant de 1 à N, où $\mathcal{R}(\mathrm{H_i})$ est la partie réelle de la prédiction Hi, $\mathcal{I}(\mathrm{H_i})$ est la partie imaginaire de la prédiction Hi, $\mathcal{R}(\mathrm{Ip_i})$ est la partie réelle de la mesure Ipi, $\mathcal{I}(\mathrm{Ip_i})$ est la partie imaginaire de la mesure Ipi,

- puis on calcule l'erreur Ei,
- puis on calcule le critère C suivant l'équation

$$C = \sum_{i=0}^{N} \left| E_i \right|^2 ,$$

- puis on calcule par le modèle de champ la prédiction Hi qui correspond au critère C,

cette prédiction Hi calculée étant utilisée pour l'itération suivante jusqu'à ce que le critère C calculé devienne inférieur à une borne positive non nulle η, prescrite.

Suivant un autre mode de réalisation de l'invention, à chaque itération

- on initialise la prédiction Hi aux valeurs initiales prescrites,
- le paramètre β est alors ajouté comme variable d'état de l'algorithme de minimisation de Levenberg-Marquardt,
- puis on calcule les $\Delta_i$ comme minimisant un vecteur C.$\Delta$-D sur ses coordonnées en fonction de Hi et Ipi et β selon les équations :

$$Hp_i = H_i.(1+\Delta_i.(j+ \beta.\omega_i)),$$

$Hpi = Hi.(1+\Delta_i.(j+ \beta.\omega_i))$,
où $\Delta$ est un vecteur $\Delta$ ayant $\Delta_i$ comme coordonnées,
D est un vecteur, dont des premières et deuxièmes coordonnées sont formées de respectivement:

$$-(\mathcal{R}(H_i) - \mathcal{R}(Ip_i))$$

et

$$-(\mathcal{I}(H_i) - \mathcal{I}(Ip_i)),$$

C est une matrice, ayant comme coefficients correspondants aux $\Delta_i$ respectivement

$$\mathcal{R}(H_i).\beta.\ \omega_i - \mathcal{I}(H_i)$$

et

$$\mathcal{R}(H_i) + \mathcal{I}(H_i).\beta.\ \omega_i$$

et les coefficients 0 ailleurs,
pour i allant de 1 à N, où

$\mathcal{R}(H_i)$ est la partie réelle de la prédiction Hi,

$\mathcal{I}(H_i)$ est la partie imaginaire de la prédiction $H_i$,

$\mathcal{R}(Ip_i)$ est la partie réelle de la mesure $Ip_i$,

$\mathcal{I}(Ip_i)$ est la partie imaginaire de la mesure $Ip_i$,

- puis on calcule l'erreur $E_i$,
- puis on calcule le critère C suivant l'équation

$$C = \sum_{i=0}^{N} \left| E_i \right|^2 ,$$

- puis on calcule par le modèle de champ la prédiction $H_i$ qui correspond au critère C,

cette prédiction $H_i$ calculée étant utilisée pour l'itération suivante jusqu'à ce que le critère C calculé devienne inférieur à une borne positive non nulle η, prescrite.

Un cinquième objet de l'invention est un localisateur magnétique suivant la revendication 10.

Un sixième objet de l'invention est un localisateur magnétique suivant la revendication 11.

Un septième objet de l'invention est un localisateur magnétique suivant la revendication 12.

Un huitième objet de l'invention est un localisateur magnétique suivant la revendication 13.

La revendication 14 concerne un mode de réalisation de ces localisateurs magnétiques.

Un neuvième objet de l'invention est un programme d'ordinateur, comportant des instructions de code qui conduisent le localisateur magnétique selon l'une quelconque des revendications 10-14 à exécuter les étapes du procédé de compensation du localisateur magnétique en présence d'au moins un matériau perturbateur de champ magnétique, tel que décrit ci-dessus, lorsque le programme d'ordinateur est exécuté sur un calculateur.

L'invention sera mieux comprise à la lecture de la description qui va suivre, donnée uniquement à titre d'exemple non limitatif en référence aux dessins annexés, sur lesquels :

- la figure 1 représente schématiquement un synoptique modulaire d'un localisateur magnétique suivant un mode de réalisation de l'invention,
- la figure 2 représente schématiquement un matériau perturbateur de champ magnétique,
- les figures 3, 4 et 5 représentent schématiquement trois diagrammes de phases de champ magnétique pour trois matériaux différents,
- les figures 6 et 7 sont des diagrammes de Fresnel représentant la mesure perturbée et un modèle perturbé Hpi, utilisés suivant un mode de réalisation de l'invention,
- la figure 8 est un synoptique modulaire d'une unité de traitement du localisateur suivant un mode de réalisation de l'invention,
- la figure 9 est un synoptique modulaire d'une unité de traitement du localisateur suivant la figure 8, utilisant un premier algorithme,
- la figure 10 est un synoptique modulaire d'une unité de traitement du localisateur suivant la figure 8, utilisant un deuxième algorithme,
- la figure 11 est un synoptique modulaire d'une unité de traitement du localisateur suivant la figure 8, utilisant un troisième algorithme.

On décrit ci-dessous en référence aux figures un procédé de compensation d'un localisateur magnétique et un dispositif de compensation d'un localisateur magnétique.

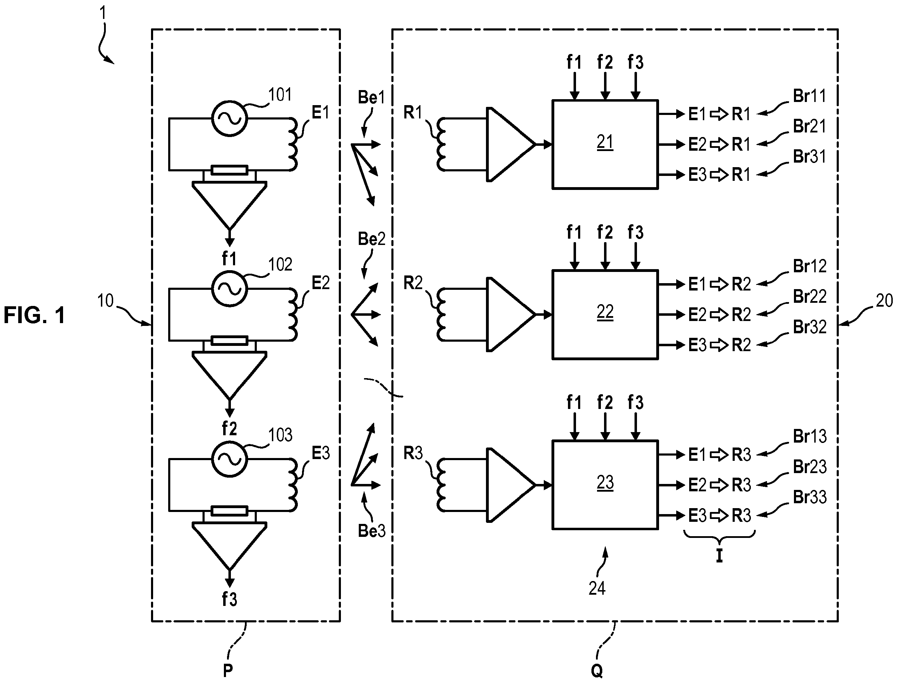

A la figure 1, un localisateur magnétique 1 ou dispositif 1 de localisation magnétique comprend un ou plusieurs émetteurs 10 et un ou plusieurs récepteurs 20.

L'émetteur 10 comprend une bobine émettrice ou une pluralité (nombre Ne) de bobines émettrices, par exemple E1, E2, E3, et un générateur ou une pluralité de générateurs, par exemple 101, 102, 103, d'au moins un signal d'émission, relié aux bobines émettrices E1, E2, E3, pour asservir les bobines émettrices avec leur signal d'émission respectif. Les bobines émettrices sont rigidement fixées à un même support mécanique de l'émetteur 10, afin d'occuper une position connue et d'avoir une orientation connue et peuvent être par exemple orientées selon des axes distincts. L'émetteur 10 a par exemple une position connue et une orientation connue. On considère ici, à titre d'exemple non limitatif, le cas d'un système dans lequel l'émetteur 10 comporte 3 bobines d'émission E1, E2, E3 orientées selon trois axes distincts, par exemple selon les trois axes d'un repère orthonormal. Ces trois axes des moments magnétiques respectifs des bobines E1, E2, E3 peuvent être confondus avec l'axe de l'enroulement des bobines dans le cas d'un bobinage orthogonal à l'axe d'enroulement. Ils peuvent cependant en être différents si le bobinage n'est pas à 90°, par exemple à 45°. Lorsqu'une tension est imposée aux bornes d'une bobine émettrice E1, E2, E3, un courant circule dans la bobine émettrice E1, E2, E3 qui génère alors un champ magnétique Be1, Be2, Be3 proportionnel au courant qui la parcoure et dont la forme dépend des caractéristiques de la bobine (orientation, moment magnétique, forme....). Une source de tension peut, par exemple, être utilisée comme générateur 101, 102, 103 pour imposer une tension aux bornes de la bobine émettrice E1, E2, E3 entraînant la création d'un courant.

Lorsque chaque générateur 101, 102, 103 est mis en marche et relié à respectivement la bobine émettrice E1, E2, E3, chaque bobine émettrice E1, E2, E3 émet un champ magnétique, respectivement Be1, Be2, Be3, d'émission en réponse au signal d'émission qui lui est envoyé par le générateur 101, 102, 103. Le signal d'émission et le champ magnétique, respectivement Be1, Be2, Be3, d'émission sont par exemple sinusoïdaux et ont par exemple une fréquence déterminée, respectivement f1, f2, f3. Les fréquences déterminées peuvent être identiques les unes aux autres ou différentes les unes des autres. Les fréquences déterminées peuvent être par exemple de 100 Hz à 50 kHz.

Le récepteur 20 comprend une bobine réceptrice ou plusieurs (nombre Nr) de bobines réceptrices, par exemple R1, R2, R3, et un dispositif 24 de mesure, qui est relié aux bobines réceptrices R1, R2, R3. Le nombre Nr de bobines réceptrices peut être différent de ou égal au nombre Ne de bobines émettrices. Les bobines réceptrices sont rigidement fixées à un même support mécanique du récepteur 120 et peuvent être par exemple orientées selon des axes distincts. Le récepteur 20 a par exemple une position connue et une orientation connue. On considère ici, à titre d'exemple, le cas d'un système dans lequel le récepteur 20 comporte 3 bobines de réception R1, R2, R3 orientées selon trois axes distincts, par exemple selon les trois axes d'un repère orthonormal. Ces trois axes des moments magnétiques respectifs des bobines R1, R2, R3 peuvent être confondus avec l'axe de l'enroulement des bobines dans le cas d'un bobinage orthogonal à l'axe d'enroulement. Ils peuvent cependant en être différents si le bobinage n'est pas à 90°, par exemple à 45°. En présence d'un champ magnétique variable, une tension (mesure) proportionnelle à la variation du flux du champ magnétique apparaît dans la bobine réceptrice. En mesurant la tension aux bornes de la bobine réceptrice, par exemple grâce à un voltmètre ou autre moyen similaire, ou en mesurant le courant (mesure) parcourant la bobine réceptrice par exemple par un ampèremètre ou autre moyen similaire, il est possible de déterminer le champ magnétique auquel est soumis la bobine réceptrice, à condition de connaître les caractéristiques de la bobine réceptrice, qui comprennent notamment le moment magnétique de la bobine réceptrice.

Le dispositif 24 de mesure mesure pour chaque bobine réceptrice R1, R2, R3 une pluralité de champs magnétiques de réception induits respectivement par la pluralité de champs magnétiques Be1, Be2, Be3 d'émission et appelés mesures Ipi pour i allant de 1 à N et comporte par exemple à cet effet des modules de mesure 21, 22, 23 reliés respectivement aux bobines réceptrices R1, R2, R3. On a N= Nr.Ne couples possibles de bobines émettrice et de bobines réceptrices, ce qui permet de constituer N mesures Ipi par le dispositif 24 de mesure. Suivant un mode de réalisation, N est plus grand que 1. Les mesures Ipi sont par exemple complexes, en ayant chacune un gain et une phase. Les mesures $Ip_i$ sont par exemple fournies sur une sortie de démodulation du dispositif 24 de mesure. Dans le cas où le signal d'émission est sinusoïdal avec une fréquence déterminée, la mesure correspondante $Ip_i$ est également sinusoïdal avec la même fréquence déterminée. Ainsi, dans l'exemple de la figure 1, le dispositif 24 de mesure mesure par le module de mesure 21 relié à la bobine réceptrice R1 :

- le champ magnétique Br11 de réception (noté E1→R1), qui est induit dans la bobine réceptrice R1 par le champ magnétique Be1 d'émission de la bobine émettrice E1,
- le champ magnétique Br21 de réception (noté E2→R1), qui est induit dans la bobine réceptrice R1 par le champ magnétique Be2 d'émission de la bobine émettrice E2,
- le champ magnétique Br31 de réception (noté E3→R1), qui est induit dans la bobine réceptrice R1 par le champ magnétique Be3 d'émission de la bobine émettrice E1.

Le dispositif 24 de mesure mesure par le module de mesure 23 relié à la bobine réceptrice R2 :

- le champ magnétique Br12 de réception (noté E1→R2), qui est induit dans la bobine réceptrice R2 par le champ magnétique Be1 d'émission de la bobine émettrice E1,
- le champ magnétique Br22 de réception (noté E2→R2), qui est induit dans la bobine réceptrice R2 par le champ magnétique Be2 d'émission de la bobine émettrice E2,
- le champ magnétique Br32 de réception (noté E3→R2), qui est induit dans la bobine réceptrice R2 par le champ magnétique Be3 d'émission de la bobine émettrice E1.

Le dispositif 24 de mesure mesure par le module de mesure 22 relié à la bobine réceptrice R3 :

- le champ magnétique Brl3 de réception (noté E1→R3), qui est induit dans la bobine réceptrice R3 par le champ magnétique Be1 d'émission de la bobine émettrice E1,
- le champ magnétique Br23 de réception (noté E2→R3), qui est induit dans la bobine réceptrice R3 par le champ magnétique Be2 d'émission de la bobine émettrice E2,
- le champ magnétique Br33 de réception (noté E3→R3), qui est induit dans la bobine réceptrice R3 par le champ magnétique Be3 d'émission de la bobine émettrice E1.

L'émetteur 10 et le récepteur 20 sont reliés à une unité 25 de traitement, (pouvant comprendre par exemple un microprocesseur) reliée d'une part au générateur 101, 102, 103 du dispositif 10 d'émission et d'autre part au dispositif 24 de mesure du dispositif 20 de réception. L'unité 25 de traitement est configurée pour traiter les signaux d'émission émis et les mesures Ipi reçues, et permet ainsi de retrouver à partir de ces signaux d'émission émis et de ces mesures Ip$_i$ la position P et l'orientation Q du récepteur 20 relativement à l'émetteur 10. L'unité 25 de traitement reçoit des informations concernant les caractéristiques des signaux d'émission appliqués aux bobines émettrices ainsi que des informations concernant les caractéristiques des signaux parcourant les bobines réceptrices (représentatifs du champ magnétique de réception par la bobine réceptrice et donc des mesures Ip$_i$). A partir de l'intensité et de la phase des champs magnétiques de réception captés par les bobines réceptrices, l'unité 25 de de traitement détermine les coordonnées spatiales de position P et d'orientation Q de l'ensemble de bobines réceptrices par rapport à l'ensemble des bobines émettrices, par exemple à l'aide d'un algorithme de minimisation, par exemple de type Levenberg-Marquardt, permettant de minimiser l'erreur entre les champs mesurés (champs magnétiques de réception ou mesures Ipi) et les champs magnétiques théoriques modélisés à partir de connaissances a priori de l'émetteur et du récepteur.

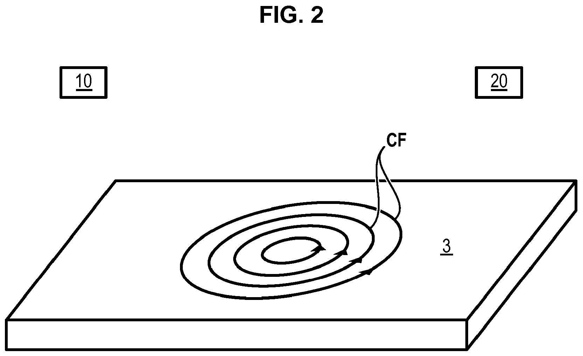

Un matériau perturbateur 3 de champ magnétique peut être présent à proximité du localisateur 1, par exemple entre l'émetteur 10 et le récepteur 20 ou à proximité de ceux-ci, ainsi que représenté à la figure 2, où seulement une bobine émettrice de l'émetteur 10 et une bobine réceptrice du récepteur 20 ont été représentées à titre d'exemple. Le matériau perturbateur 3 peut être par exemple un matériau conducteur de l'électricité, par exemple métallique. Tout matériau conducteur 3 de l'électricité placé dans un champ magnétique variable donne naissance à des courants de Foucault dans le matériau conducteur 3, symbolisés par les boucles de courant CF à la figure 2. Les courants de Foucault CF qui circulent alors dans ce conducteur 3 donne naissance à leur tour à un champ magnétique perturbateur dont la phase dépend de l'impédance du conducteur 3. Mais cette phase est constante pour une fréquence donnée. Le matériau perturbateur 3 peut être par exemple une prothèse métallique, comme par exemple une tête fémorale de prothèse de hanche, une tige fémorale de prothèse de hanche, une plaque métallique, par exemple de cuivre, une autre bobine métallique (autres que les bobines émettrices et les bobines réceptrices) ou autres.

Si le champ magnétique induit par le perturbateur n'est pas en phase avec le signal de l'émetteur alors il est possible de détecter la perturbation et de la compenser en partie. La méthode présentée permet de compenser un seul perturbateur, elle ne compense pas les perturbations en phase avec le signal.

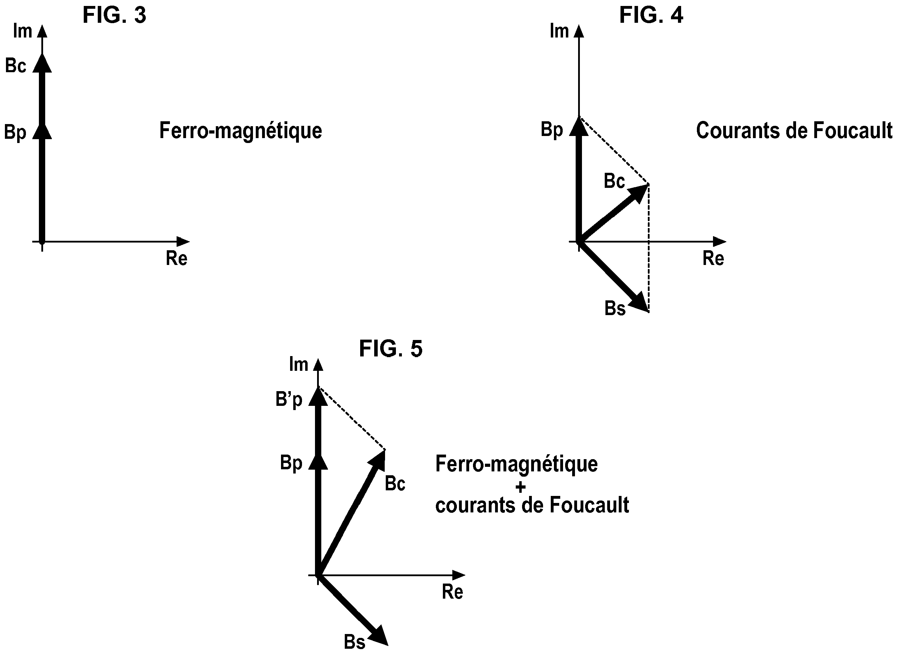

On peut résumer les situations par les trois diagrammes de phases des figures 3, 4 et 5 (partie réelle Re des champs magnétiques en abscisses, partie imaginaire Im des champs magnétiques en ordonnées), avec Bp le champ magnétique initial sans perturbation (par exemple le champ magnétique d'émission Be1 ou Be2 ou Be3), Bs le champ magnétique émis par les courants de Foucault CF du matériau perturbateur 3 de champ magnétique et Bc le champ magnétique résultant, égal à la somme de Bp et de Bs.

A la figure 3, dans le cas d'un matériau ferromagnétique (comme par exemple les ferrites), le matériau génère une perturbation ferro-magnétique, où il n'existe pas de courants de Foucault et il n'y a pas de déphasage entre le champ initial Bp et le champ résultant Bc.

A la figure 4, dans le cas d'un matériau perturbateur 3 de champ magnétique, qui est conducteur de l'électricité (comme par exemple une plaque de cuivre et/ou d'argent et/ou d'aluminium), les courants de Foucault CF induits par le matériau 3 créent un champ secondaire Bs qui a pour effet de diminuer le champ initial Bp et d'introduire un déphasage. Le champ résultant Bc est déphasé et plus faible en amplitude par rapport au champ initial Bp.

A la figure 5, dans le cas d'un matériau perturbateur 3 de champ magnétique, qui est mixte (à la fois ferromagnétique et conducteur de l'électricité), pour des fréquences relativement faibles, le caractère ferro-magnétique fait que l'amplitude du champ résultant Bc est plus forte que celle du champ initial Bp. Les courants de Foucault CF induits par le matériau 3 introduisent en même temps un déphasage. A mesure que la fréquence augmente, l'effet du ferromagnétisme diminue, celui des courants de Foucault CF induits par le matériau 3 CF augmente, l'amplitude du champ résultant Bc diminue de plus en plus, le déphasage augmente puis décroît selon une loi propre au matériau 3.

L'invention permet de compenser la perturbation Bs due aux courants de Foucault CF induits par le matériau 3. On prendra comme hypothèse que les perturbations ferromagnétiques sont nulles.

Le matériau perturbateur 3 de champ magnétique peut être un objet métallique conducteur en une certaine position de l'espace relative à une bobine émettrice. Cela implique que si l'objet métallique se déplace, il constitue un nouveau perturbateur dont les caractéristiques sont différentes.

Le localisateur magnétique 1 met en oeuvre le procédé de compensation de la présence du matériau perturbateur 3 de champ magnétique, décrit ci-dessous.

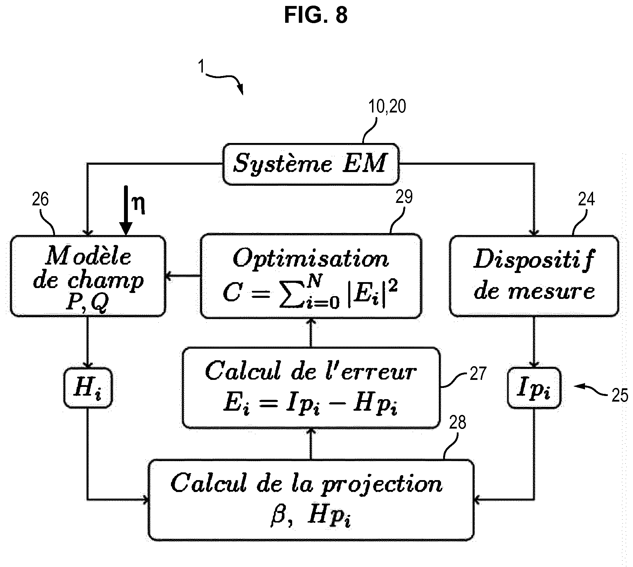

A la figure 8, le localisateur 1 comporte l'unité 25 de traitement comportant un module 26 de modélisation de champ permettant de calculer une position P et/ou une orientation Q du récepteur 20 par calcul d'une prédiction Hi des mesures en fonction d'un critère C de minimisation d'une erreur Ei calculée par rapport aux mesures Ip$_i$. Ce module 26 peut comporter par exemple un filtre de Kalman pour calculer la prédiction H$_i$. Le module 26 dispose d'une modélisation physique du localisateur 1, qui prédit en l'absence de perturbation et en fonction de la position P et/ou de l'orientation Q du récepteur 20 les N mesures, pour fournir les prédictions H$_i$. Le module 26 cherche la position P et/ou de l'orientation Q (ou état du capteur P, Q) et calcule la prédiction H$_i$ qui vérifient le critère C. Le critère C est considéré par le module 26 comme représentant l'écart (pouvant être par exemple une norme de l'erreur prise au sens de L2, pouvant être la somme des carrés des erreurs Ei sur i allant de 1 à N) entre la prédiction H$_i$ et les mesures Ip$_i$. Le critère C peut être calculé comme étant par exemple une norme de l'erreur Ei prise au sens de L$^2$. Suivant un mode de réalisation, le critère C peut être calculé comme étant la somme des carrés des erreurs Ei sur i allant de 1 à N. Suivant un mode de réalisation, le critère C peut être calculé selon l'équation suivante

$$C = \sum_{i=0}^{N} \left| H_i(P,Q) - IP_i \right|^2$$

Suivant un mode de réalisation, l'erreur $E_i$ est calculée par un module 27 de calcul d'erreur comme étant la différence entre les mesures $Ip_i$ et un modèle perturbé Hpi des mesures, selon l'équation

$$E_i = Ip_i - Hp_i.$$

A un instant donné, on dispose de N mesures complexes $Ip_i$ et de N prédictions complexes $H_i$ issues du modèle 26, pour i allant de 1 à N. Chacune de ces mesures $Ip_i$ est effectuée à une certaine fréquence ou pulsation (égale à la fréquence multipliée par $2\pi$), qui est notée ci-dessous $\omega_i$.

Les mesures $Ip_i$ sont par exemple rangées dans un vecteur Ip ayant comme coordonnées ces mesures $Ip_i$, c'est-à-dire

$$Ip = \begin{pmatrix} Br11 \\ Br21 \\ ... \\ \dfrac{BrN_r1}{Br21} \\ ... \\ ... \\ BrN_eN_r \end{pmatrix} = \begin{pmatrix} E1 \rightarrow R1 \\ E1 \rightarrow R2 \\ ... \\ \dfrac{E1 \rightarrow R_{N_r}}{E2 \rightarrow R1} \\ ... \\ ... \\ E_{N_e} \rightarrow R_{N_r} \end{pmatrix}$$

Les prédictions $H_i$ sont par exemple rangées dans un vecteur H(P,Q) ayant comme coordonnées ces prédictions $H_i$, c'est-à-dire

$$H(P,Q) = \begin{pmatrix} H(Br11) \\ H(Br21) \\ ... \\ \dfrac{H(BrN_r1)}{H(Br21)} \\ ... \\ ... \\ H(BrN_eN_r) \end{pmatrix} = \begin{pmatrix} H(E1 \rightarrow R1) \\ H(E1 \rightarrow R2) \\ ... \\ \dfrac{H(E1 \rightarrow R_{N_r})}{H(E2 \rightarrow R1)} \\ ... \\ ... \\ H(E_{N_e} \rightarrow R_{N_r}) \end{pmatrix}$$

En l'absence de matériau perturbateur 3 de champ magnétique par courant de Foucault $P_i=0$, on suppose que le modèle 26 est convenablement calibré et qu'il suit correctement la mesure (alors appelée $I_i$ en l'absence de matériau perturbateur 3 de champ magnétique) en fonction du temps t, selon l'équation $H_i(t) = I_i(t) + \varepsilon_i$ avec $\varepsilon_i$ négligeable ($\varepsilon_i = 0$ par la suite).

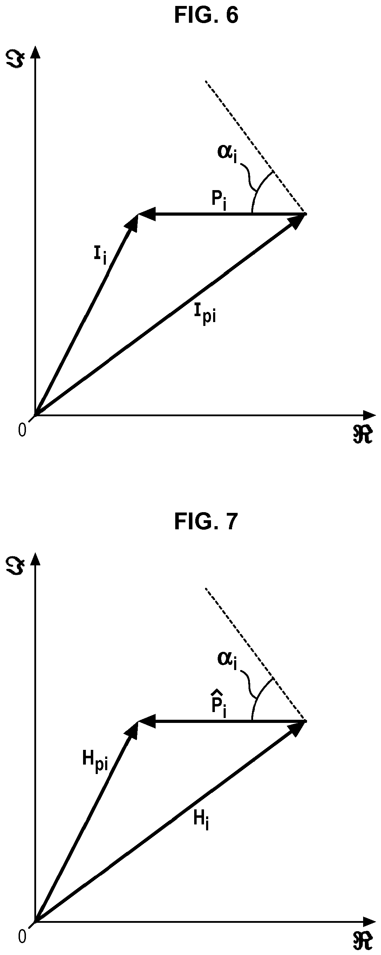

Lorsqu'un matériau perturbateur 3 de champ magnétique est présent, une perturbation $P_i$, non négligeable, s'ajoute à la mesure $I_i$ et on a alors $Ip_i= I_i+P_i$ et le modèle perturbé $Hp_i$ est égal à $Hp_i= H_i+P_i$. La figure 6 est un diagramme de Fresnel représentant la mesure perturbée $Ip_i$ par rapport à une mesure non perturbée $I_i$. La figure 7 est un diagramme de Fresnel représentant la prédiction non perturbée $H_i$ par rapport au modèle perturbé $Hp_i$.

On ne corrige pas la mesure $Ip_i$ qui elle est exacte mais on modélise la perturbation $P_i$ en calculant un modèle perturbé $Hp_i$ de telle sorte que le critère C soit minimal. Suivant un mode de réalisation, l'unité 25 de traitement comporte un module 29 de calcul du critère C de minimisation d'une erreur Ei calculée par rapport aux mesures $Ip_i$. Suivant un mode de réalisation,

$$C = \sum_{i=0}^{N} \left| HP_i - IP_i \right|^2$$

Suivant un mode de réalisation, on calcule comme modèle perturbé Hpi une projection de la prédiction $H_i$ sur les mesures $Ip_i$ en fonction d'un paramètre $\beta$ caractéristique du matériau perturbateur 3 de champ magnétique, et par exemple uniquement en fonction du paramètre $\beta$. Cette projection permet de réduire l'effet du matériau perturbateur 3 de champ magnétique par courants de Foucault.

Suivant un mode de réalisation, on minimise le critère $C = E^T.E$ avec $E = Ip-Hp(H,Ip,\beta)$.

Le vecteur $Hp(H,Ip,\beta)$ est une fonction de projection de H sur Ip et est calculé en fonction du vecteur $H(P, Q)$ et du vecteur Ip.

Le vecteur $Hp(H,Ip,\beta)$ a comme coordonnées ce modèle perturbé $Hp_i$, pour i allant de 1 à N, c'est-à-dire

$$Hp(H,Ip,\beta) = \begin{pmatrix} Hp(Br11) \\ Hp(Br21) \\ ... \\ \dfrac{Hp(BrN_r1)}{Hp(Br21)} \\ ... \\ ... \\ Hp(BrN_eN_r) \end{pmatrix} = \begin{pmatrix} Hp(E1 \rightarrow R1) \\ Hp(E1 \rightarrow R2) \\ ... \\ \dfrac{Hp(E1 \rightarrow R_{N_r})}{Hp(E2 \rightarrow R1)} \\ ... \\ ... \\ Hp(E_{N_e} \rightarrow R_{N_r}) \end{pmatrix}$$

Le modèle perturbé $Hp_i$ des mesures est calculé par un module 28 de calcul de modèle perturbé à partir des mesures $Ip_i$ et de la prédiction $H_i$ par les équations suivantes :

$$P_i = \rho_i \frac{jI_i}{|I_i|} e^{j\alpha_i}$$

$$I_i = H_i = Ip_i - P_i$$

où $P_i$ est la perturbation apportée aux mesures $Ip_i$ par le matériau perturbateur 3 de champ magnétique, $\rho_i$ est l'intensité de la perturbation $P_i$, $\alpha_i$ est un angle de déphasage provoqué par le matériau perturbateur 3 de champ magnétique dans

la perturbation $P_i$.

Les angles de déphasage $\alpha_i$ sont liés par la relation caractéristique du matériau perturbateur 3 de champ magnétique selon l'équation

$$\alpha_i = -\arctan(\beta. \omega_i).$$

où $\beta$ est un paramètre caractéristique du matériau perturbateur 3 de champ magnétique.

Le paramètre $\beta$ est calculé comme étant identique pour toutes les mesures $Ip_i$ et représente un rapport d'une inductance $L_p$ modélisant le matériau perturbateur 3 sur une résistance $R_p$ modélisant le matériau perturbateur 3 dans une certaine position relative à l'émetteur 10, l'impédance du matériau perturbateur 3 étant $Zp = R_p + j.L_p.\omega_i$.

La perturbation $P_i$ et/ou l'intensité $\rho_i$ de la perturbation $P_i$ et/ou l'angle de déphasage $\alpha_i$ et/ou le paramètre $\beta$ et/ou le modèle perturbe $Hp_i$ est calculé de manière à minimiser l'erreur $Ei$.

On modélise la fonction de projection vecteur $Hp(H,Ip,\beta)$ pour identifier le paramètre $\beta$.

Pour i allant de 1 à N, N équations sont à résoudre.

En supposant $I_i = H_i$ et $Ip_i = Hp_i$, on obtient

$$H_i + P_i - Ip_i = 0$$

puis on obtient le vecteur Hp de modèle projeté dont les coordonnées sont Hpi donnée par l'équation suivante :

$$Hp_i = H_i.(1+\Delta_i.(j+ \beta.\omega_i))$$

avec

$$\Delta_i = \frac{\rho_i}{|H_i| \cdot \sqrt{\beta^2 \cdot \omega_i^2 + 1}}$$

$\Delta_i$ est appelé distance de projection.

La perturbation $P_i$ et/ou l'intensité $\rho_i$ de la perturbation $P_i$ et/ou l'angle de déphasage $\alpha_i$ et/ou le paramètre $\beta$ et/ou le modèle perturbe $Hp_i$ et/ou la distance $\Delta_i$ de projection peuvent être calculés par l'unité 25 de traitement par un procédé de calcul à itérations successives selon l'une des figures 8 à 11. Par exemple, suivant un mode de réalisation, à chaque itération

- on initialise les prédictions $H_i$ du module 26 à des valeurs données,
- puis on calcule par le module 28 par l'un des procédés de calcul ou algorithmes décrits le modèle perturbé $Hp_i$, en fonction de $H_i$ et $Ip_i$, par exemple selon les équations

$$\Delta_i = [\mathcal{R}(H_i).\mathcal{I}(Ip_i) - \mathcal{I}(H_i).\mathcal{R}(Ip_i)]/[(\mathcal{I}(H_i)^2 + \mathcal{R}(H_i)^2] ,$$

$$\beta = [-\mathcal{R}(H_i)^2 - \mathcal{I}(H_i)^2 + \mathcal{R}(H_i).\mathcal{R}(Ip_i) + \mathcal{I}(H_i).\mathcal{I}(Ip_i)]/[(\mathcal{R}(H_i).\mathcal{I}(Ip_i) - \mathcal{I}(H_i).\mathcal{R}(Ip_i)).\omega_i] ,$$

$$Hp_i = H_i.(1+\Delta_i.(j+ \beta.\omega_i)) ,$$

- puis on calcule par le module 27 l'erreur Ei selon l'équation Ei = Ipi - Hpi,

- puis on calcule par le module 29 le critère C suivant l'équation

$$C = \sum_{i=0}^{N} |HP_i - IP_i|^2$$

, ou suivant l'équation

$$C = \sum_{i=0}^{N} |E_i|^2$$

- puis on calcule par le modèle du module 26 la prédiction $H_i$ qui correspond au critère C à l'aide de l'algorithme de minimisation, par exemple de type Levenberg-Marquardt, du modèle. Cet algorithme de minimisation cherche par le module 26 la position P et/ou l'orientation Q du récepteur 20 qui minimise l'écart entre la prédiction $H_i$ selon le critère C et les mesures $Ip_i$. La prédiction $H_i$ ainsi calculée par le modèle 26 est utilisée pour l'itération suivante. On poursuit les itérations jusqu'à ce que, par exemple le critère C calculé devienne inférieur à une borne positive non nulle η, prescrite.

Dans le cas où le matériau perturbateur 3 se déplace par rapport à l'émetteur 10 et/ou au récepteur 20, le paramètre β varie dans le temps en fonction du déplacement du matériau perturbateur 3.

Chaque module 26, 27, 28, 29 de l'unité 25 de traitement et/ou chaque étape du procédé peut être mis en œuvre par des moyens informatiques, par exemple par un ou plusieurs calculateur(s), un ou plusieurs ordinateur(s), un ou plusieurs microprocesseur(s) et un ou plusieurs programme(s) d'ordinateur. Le ou les programme(s) d'ordinateur comporte des instructions de code pour la mise en oeuvre du procédé de compensation.

Suivant un mode de réalisation, le localisateur magnétique 1 comprenant :

- au moins un émetteur 10 comprenant une pluralité (Ne) de bobines émettrices et un générateur apte à générer au moins un signal d'émission, relié aux bobines émettrices, pour que chaque bobine émettrice soit apte à émettre au moins un champ magnétique d'émission à au moins une pulsation déterminée $\omega_i$ en réponse au signal d'émission qui lui est envoyé par le générateur,
- au moins un récepteur comprenant une ou plusieurs (Nr) bobines réceptrices et un dispositif de mesure, qui est apte à être relié à la ou aux bobines réceptrices et qui est apte à mesurer pour chaque bobine réceptrice une pluralité de champs magnétiques de réception induits respectivement par la pluralité de champs d'émission et appelés mesures Ipi pour i allant de 1 à N,
- une unité 25 de traitement comportant un modèle de champ permettant de calculer une position P et/ou une orientation Q du récepteur par calcul de la prédiction Hi des mesures en fonction du C calculé en fonction de l'erreur $E_i$, elle-même calculée par rapport aux mesures Ipi,

caractérisé en ce que

l'unité 25 de traitement est configurée pour que l'erreur Ei soit calculée par itérations successives depuis des valeurs initiales prescrites de la prédiction Hi comme étant la différence entre les mesures Ipi et un modèle perturbé Hpi des mesures, selon l'équation

$$E_i = Ip_i - Hp_i,$$

le modèle perturbé Hpi des mesures vérifiant les équations suivantes

$$Hp_i = H_i + P_i,$$

$$P_i = \rho_i \frac{jH_i}{|H_i|} e^{j\alpha_i} \; ,$$

$$\alpha_i = -\arctan(\beta \cdot \omega_i),$$

où Pi est une perturbation apportée aux mesures Ipi par le matériau perturbateur 3 de champ magnétique, $\rho_i$ est l'intensité de la perturbation, $\alpha_i$ est un angle de déphasage provoqué par le matériau perturbateur 3 de champ magnétique, β étant un paramètre du matériau perturbateur 3 de champ magnétique,
le paramètre β étant identique pour toutes les mesures Ipi,

l'unité 25 de traitement étant configurée pour effectuer le calcul de manière à minimiser le critère C.

L'unité 25 de traitement est ainsi configurée pour compenser la présence du matériau perturbateur 3 de champ magnétique.

Le localisateur magnétique 1 comprend ainsi dans son unité 25 de traitement des moyens de compensation de la présence du matériau perturbateur 3 de champ magnétique.

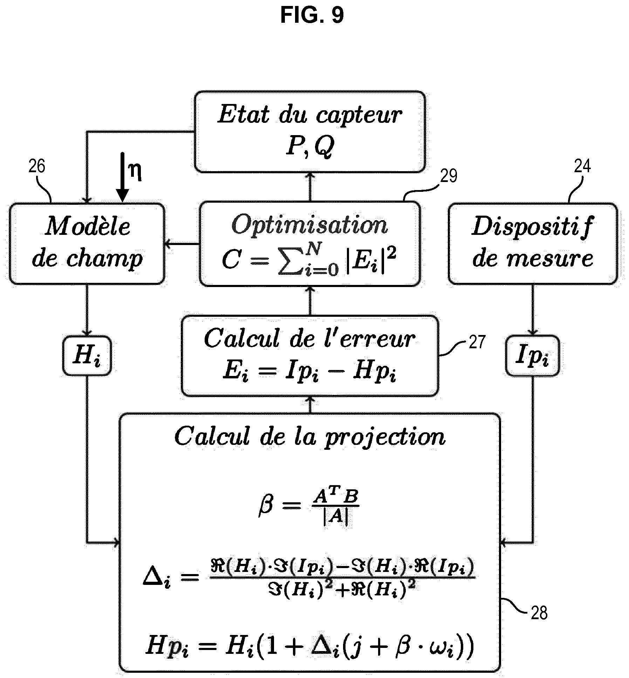

On décrit ci-dessous un premier algorithme pour effectuer le calcul, en référence à la figure 9.

Suivant un mode de réalisation, en partant de l'équation Hi -Ipi $+\Delta_i.H_i.(j+\beta.\omega_i)) = 0$ , on obtient sur les parties réelles et les parties imaginaires de celle-ci un système de deux équations, qui présente une solution unique pour $\Delta_i$ et $\beta$, à savoir la solution

$$\Delta_i = [\mathscr{R}(H_i).\mathscr{I}(Ip_i) - \mathscr{I}(H_i).\mathscr{R}(Ip_i)]/[(\mathscr{I}(H_i)^2 + \mathscr{R}(H_i)^2]$$

et

$$\beta = [-\mathscr{R}(H_i)^2-\mathscr{I}(H_i)^2+\mathscr{R}(H_i).\mathscr{R}(Ip_i)+\mathscr{I}(H_i).\mathscr{I}(Ip_i)]/[(\mathscr{R}(H_i).\mathscr{I}(Ip_i)-\mathscr{I}(H_i).\mathscr{R}(Ip_i)).\omega_i]$$

Suivant un mode de réalisation, le premier algorithme A de calcul prévoit que $\beta$ est calculé comme étant une solution au système d'équations suivant, provenant des équations précédentes donnant Hpi, à savoir

$$\Delta_i.\mathscr{R}(H_i).\beta.\,\omega_i = \mathscr{R}(Ip_i) - \mathscr{R}(H_i) + \Delta_i.\mathscr{I}(H_i)$$

et

$$\Delta_i.\mathscr{I}(H_i).\beta.\,\omega_i = \mathscr{I}(Ip_i) - \mathscr{I}(H_i) - \Delta_i.\mathscr{R}(H_i)$$

pour i allant de 1 à N, où

$\mathscr{R}(H_i)$ est la partie réelle de la prédiction Hi et est également noté $\Re\big(H_i\big)$,

$\mathscr{I}(H_i)$ est la partie imaginaire de la prédiction Hi et est également noté $\Im\big(H_i\big)$,

$\mathscr{R}(Ip_i)$ est la partie réelle de la mesure Ipi et est également noté $\Re\big(IP_i\big)$,

$\mathscr{I}(Ip_i)$ est la partie imaginaire de la mesure Ipi et est également noté $\Im\big(IP_i\big)$.

Suivant un mode de réalisation, ce premier algorithme A de calcul peut prévoir que le paramètre $\beta$ est calculé comme minimisant un vecteur A.$\beta$ - B sur ses coordonnées, où

A est un vecteur, dont des premières et deuxièmes coordonnées sont formées de respectivement :

$$\Delta_i.\mathscr{R}(H_i).\omega_i$$

et

$$\Delta_i.\mathscr{I}(H_i).\omega_i \,,$$

,

B est un vecteur, dont des premières et deuxièmes coordonnées sont formées de respectivement:

$$\mathscr{R}(Ip_i) - \mathscr{R}(H_i) + \Delta_i.\mathscr{I}(H_i)$$

et

$$\mathcal{I}(\mathrm{Ip_i}) - \mathcal{I}(\mathrm{H_i}) - \Delta_i.\mathcal{R}(\mathrm{H_i}).$$

.

Dans un mode de réalisation, le paramètre $\beta$ peut être calculé comme minimisant la somme des carrés des coordonnées du vecteur A.$\beta$ - B pour i allant de 1 à N.

Dans un autre mode de réalisation, le paramètre $\beta$ peut être calculé comme étant une solution minimale à l'équation vectorielle A.$\beta$ = B, par

$$\beta = \frac{A^T \cdot B}{\left|A\right|^2}$$

L'opérateur $^T$ désigne la transposition.

Dans un autre mode de réalisation, on utilise une matrice de poids notée W telle que :

$$(W.A). \beta = (W.B)$$

avec W=P.I

et P=($p_1$, $p_2$, $p_3$, ..., $p_N$).

Le paramètre $\beta$ peut alors être calculé comme étant

$$\beta = \frac{(W \cdot A)^T \cdot B}{\left|(W \cdot A)\right|^2}$$

Par exemple, les $p_i$ peuvent être égaux à une puissance de la norme des Hi , c'est-à-dire $P = (|H_0|^k,|H_1|^k,...,|H_N|^k)$.

Ainsi, plus k est grand et plus on prend en compte les grandes mesures, par rapport aux petites. k=2 semble être un bon compromis. On évite ainsi que les petites valeurs des coordonnées des vecteurs A et B conduisent à des phases erronées et dans l'estimation du $\beta$ on peut les rejeter d'avantage.

Dans un mode de réalisation, ce premier algorithme A de calcul, peut être mis en œuvre par les étapes suivantes :

-   calcul de $\Delta_i$ selon l'équation

$$\Delta_i = [\mathcal{R}(\mathrm{H_i}).\mathcal{I}(\mathrm{Ip_i}) - \mathcal{I}(\mathrm{H_i}).\mathcal{R}(\mathrm{Ip_i})]/[(\mathcal{I}(\mathrm{H_i})^2 + \mathcal{R}(\mathrm{H_i})^2],$$

,

$$\beta = \frac{A^T \cdot B}{\left|A\right|^2} \qquad \beta = \frac{(W \cdot A)^T \cdot B}{\left|(W \cdot A)\right|^2}$$

-   calcul de $\beta$ selon l'équation                    ou                    ,

-   calcul de Hpi selon l'équation $Hp_i = H_i.(1+\Delta_i.(j+ \beta.\omega_i))$, ainsi que représenté à la figure 9.

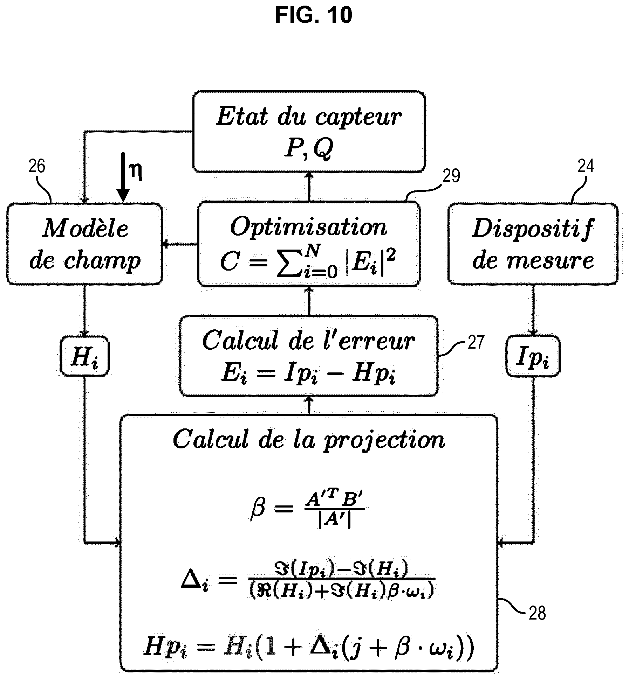

On décrit ci-dessous un deuxième algorithme de calcul, en référence à la figure 10.

Suivant un mode de réalisation, en partant de l'équation

$$\mathrm{H_i} -\mathrm{Ip_i} +\Delta_i.\mathrm{H_i}.(j+\beta.\omega_i)) = 0 \ ,$$

on obtient sur les parties réelles et les parties imaginaires de celle-ci un système de deux équations, qui présente une solution unique pour $\Delta_i$ et $\beta$, à savoir la solution

$$\Delta_i = [\mathscr{I}(Ip_i) - \mathscr{I}(H_i)]/[\mathscr{R}(H_i) + \mathscr{I}(H_i).\beta.\omega_i]$$

Suivant un mode de réalisation, le deuxième algorithme B de calcul prévoit que $\beta$ est calculé comme étant une solution à l'équation suivante, provenant de l'équation précédente donnant $\Delta_i$, à savoir

$$\beta. \omega_i.[\mathscr{R}(H_i).\mathscr{I}(Ip_i) - \mathscr{R}(Ip_i).\mathscr{I}(H_i)] + \mathscr{R}(H_i)^2 + \mathscr{I}(H_i)^2 - (\mathscr{R}(H_i).\mathscr{R}(Ip_i) + \mathscr{I}(H_i).(Ip_i)) = 0$$

pour i allant de 1 à N.

Suivant un mode de réalisation, ce deuxième algorithme B de calcul peut prévoir que le paramètre $\beta$ est calculé comme minimisant un vecteur A'.$\beta$ + B' sur ses coordonnées, où

A' est un vecteur, dont les coordonnées sont formées de respectivement :

$$\omega_i.[\mathscr{R}(H_i).\mathscr{I}(Ip_i) - \mathscr{R}(Ip_i).\mathscr{I}(H_i)] ,$$

B' est un vecteur, dont les coordonnées sont formées de respectivement:

$$\mathscr{R}(H_i)^2 + \mathscr{I}(H_i)^2 - (\mathscr{R}(H_i).\mathscr{R}(Ip_i) + \mathscr{I}(H_i).(Ip_i)).$$

Dans un mode de réalisation, le paramètre $\beta$ peut être calculé comme minimisant la somme des carrés des coordonnées du vecteur A'.$\beta$ + B' pour i allant de 1 à N.

Dans un autre mode de réalisation, le paramètre $\beta$ peut être calculé comme étant une solution minimale à l'équation vectorielle A'.$\beta$ = -B', par

$$\beta = \frac{A'^T \cdot B'}{|A'|^2}$$

Dans un mode de réalisation, ce deuxième algorithme B de calcul peut être mis en œuvre par les étapes suivantes :

- calcul des vecteurs A' et B' selon les coordonnées indiquées ci-dessus,
- calcul de $\beta$ selon l'équation $\beta = \dfrac{A'^T \cdot B'}{|A'|^2}$ ou selon l'équation $\beta = \dfrac{(W \cdot A')^T \cdot B'}{|(W \cdot A')|^2}$ si on utilise la matrice de poids précitée W,
- calcul de $\Delta_i$ selon l'équation

$$\Delta_i = [\mathscr{I}(Ip_i) - \mathscr{I}(H_i)]/[\mathscr{R}(H_i) + \mathscr{I}(H_i).\beta.\omega_i]$$

et calcul de Hpi selon l'équation Hpi = $H_i$.(1+$\Delta_i$.(j+ $\beta.\omega_i$)), ainsi que représenté à la figure 10.

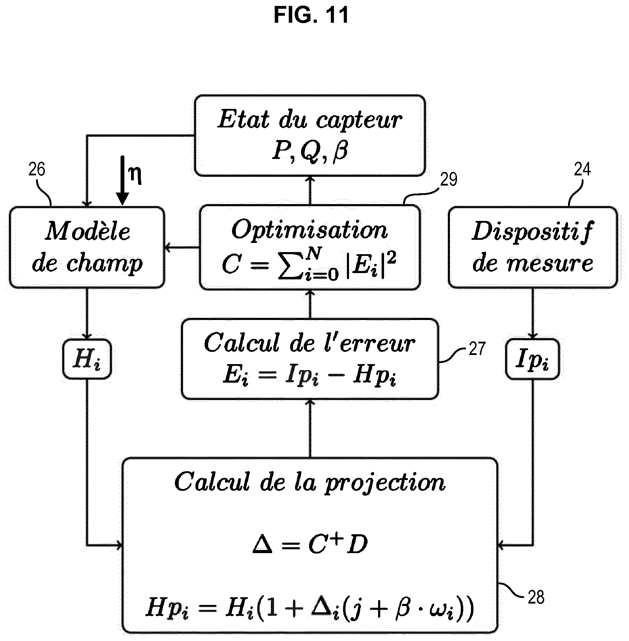

On décrit ci-dessous un troisième algorithme C de calcul, en référence à la figure 11.

Suivant un mode de réalisation, en partant de l'équation

$$H_i - Ip_i + \Delta_i.H_i.(j + \beta.\omega_i)) = 0 ,$$

on obtient sur les parties réelles et les parties imaginaires de celle-ci un système de deux équations, à savoir

$$\mathcal{R}(H_i) - \mathcal{R}(Ip_i) + \Delta_i.(\mathcal{R}(H_i).\beta.\,\omega_i - \mathcal{I}(H_i)) = 0$$

et

$$\mathcal{I}(H_i) - \mathcal{I}(Ip_i) + \Delta_i.(\mathcal{R}(H_i) + \mathcal{I}(H_i).\beta.\,\omega_i) = 0$$

Suivant un mode de réalisation, le troisième algorithme C de calcul prévoit que $\beta$ est calculé comme étant une solution à ce système d'équations. Par exemple, l'algorithme de Levenberg-Marquardt qui fait la résolution inverse calcule en plus le paramètre $\beta$ comme étant une variable d'état.

Suivant un mode de réalisation, ce troisième algorithme C de calcul peut prévoir qu'un vecteur $\Delta$ ayant $\Delta_i$ comme coordonnées est calculé comme minimisant un vecteur $C.\Delta - D$ sur ses coordonnées, où

D est un vecteur, dont des premières et deuxièmes coordonnées sont formées de respectivement:

$$- (\mathcal{R}(H_i) - \mathcal{R}(Ip_i))$$

et

$$-(\mathcal{I}(H_i) - \mathcal{I}(Ip_i)),$$

,

C est une matrice, ayant comme coefficients correspondants aux $\Delta_i$ respectivement

$$\mathcal{R}(H_i).\beta.\,\omega_i - \mathcal{I}(H_i)$$

et

$$\mathcal{R}(H_i) + \mathcal{I}(H_i).\beta.\,\omega_i\ ,$$

, c'est-à-dire que le vecteur $C.\Delta - D$ est égal à :

$$\begin{pmatrix} (\Re(H_0)\cdot\beta\cdot\omega_0 - \Im(H_0)) & 0 & ... & 0 \\ (\Re(H_0)+\Im(H_0)\cdot\beta\cdot\omega_0) & 0 & ... & 0 \\ 0 & (\Re(H_1)\cdot\beta\cdot\omega_1 - \Im(H_1)) & ... & 0 \\ 0 & (\Re(H_1)+\Im(H_1)\cdot\beta\cdot\omega_1) & ... & 0 \\ ... & ... & ... & ... \\ 0 & 0 & ... & (\Re(H_N)\cdot\beta\cdot\omega_N - \Im(H_N)) \\ 0 & 0 & ... & (\Re(H_N)+\Im(H_N)\cdot\beta\cdot\omega_N) \end{pmatrix} \begin{pmatrix} \Delta_0 \\ \Delta_1 \\ ... \\ \Delta_N \end{pmatrix} - \begin{pmatrix} \Re(IP_0) - \Re(H_0) \\ \Im(IP_0) - \Im(H_0) \\ \Re(IP_1) - \Re(H_1) \\ \Im(IP_1) - \Im(H_1) \\ ... \\ ... \\ \Re(IP_N) - \Re(H_N) \\ \Im(IP_N) - \Im(H_N) \end{pmatrix}$$

Dans un autre mode de réalisation, le paramètre $\beta$ peut être calculé comme étant une solution à l'équation vectorielle $C.\Delta = D$. Par exemple, on calcule une matrice $C^+$ qui est la pseudo-inverse de C, pour calculer le vecteur $\Delta$ come étant

$$\Delta = C^+.D.$$

Dans un mode de réalisation, ce troisième algorithme C de calcul peut être mis en œuvre par les étapes suivantes :

- estimation du paramètre $\beta$ par l'algorithme de Levenberg-Marquardt,

- calcul de la matrice C et du vecteur D, calcul du vecteur $\Delta$ vérifiant l'équation C.$\Delta$ =D, par exemple par $\Delta$ =C$^+$.D
- calcul de Hpi selon l'équation Hpi = $H_i$.(1+$\Delta_i$.(j+ $\beta.\omega_i$)), ainsi que représenté à la figure 11.

On décrit ci-dessous une variante du procédé et du dispositif de compensation suivant l'invention. Suivant cette variante, chaque bobine émettrice émet plusieurs fréquences. On émet ainsi plusieurs fréquences par axe de bobine émettrice. On peut ainsi considérer que le matériau perturbateur 3 de champ magnétique constitue plusieurs matériaux perturbateurs 3 de champ magnétique et que les distances de projection sont liées pour les différentes fréquences. On peut considérer par exemple un localisateur 1 ayant une bobine émettrice émettant à K fréquences différentes et J bobines réceptrices, avec K supérieur ou égal à 2 et J supérieur ou égal à 2. On a donc N=K.J mesures Ipi. On a donc N équations de la forme :

$$Ip_i = H_i.(1+\Delta_i.(j+ \beta.\omega_i)).$$

On peut décomposer l'indice i appartenant à [1, N] en deux indices j appartenant à [1, J] et k appartenant à [1, K]. L'équation précédente devient alors :

$$Ip_{jk} = H_{jk}.(1+\Delta_{jk}.(j+ \beta.\omega_{jk})).$$

Dans toutes les équations, la lettre j n'apparaissant pas en indice est le nombre complexe dont le carré est égal à -1, ainsi que cela est connu de l'homme du métier.
On pose le changement de variable :

$$\Delta_{jk} = \rho_j \frac{\omega_{jk}}{1+\left(\beta \cdot \omega_{jk}\right)^2}$$

$\rho_j$ ne dépend que de j.
Les équations à minimiser deviennent :

$$F_{(\rho_j,\beta)} = \begin{cases} R_{jk} = \Re(H_{jk}) - \Re(Ip_{jk}) + \rho_j \frac{\omega_{jk}}{1+\left(\beta \cdot \omega_{jk}\right)^2} \cdot \left(\Re(H_{jk}) \cdot \beta \cdot \omega_{jk} - \Im(H_{jk})\right) = 0 \\[4mm] I_{jk} = \Im(H_{jk}) - \Im(Ip_{jk}) + \rho_j \frac{\omega_{jk}}{1+\left(\beta \cdot \omega_{jk}\right)^2} \cdot \left(\Im(H_{jk}) \cdot \beta \cdot \omega_{jk} + \Re(H_{jk})\right) = 0 \end{cases}$$

On a donc un système de 2JK équations à J+1 inconnues à minimiser.
Suivant un mode de réalisation, 2.J.K $\geq$ J+1.
Si on résout deux par deux, on obtient :

$$v_{jk} = \Re(H_{jk}) \cdot \Re(Ip_{jk}) + \Im(H_{jk}) \cdot \Im(Ip_{jk})$$

$$u_{jk} = \left(\Re(H_{jk}) \cdot \Im(Ip_{jk}) - \Im(H_{jk}) \cdot \Re(Ip_{jk})\right) \cdot \omega_{jk}$$

$$u_{jk} \cdot \rho_j = \left|H_{jk}\right|^2 + \left|Ip_{jk}\right|^2 - 2 \cdot v_{jk}$$

$$u_{jk} \cdot \beta = v_{jk} - \left|H_{jk}\right|^2$$

La solution aux moindres carrés s'exprime par :

$$\rho_j = \frac{\sum\limits_{k=1}^{K} u_{jk} \cdot \left( \left|H_{jk}\right|^2 + \left|Ip_{jk}\right|^2 - 2 \cdot v_{jk} \right)}{\sum\limits_{k=1}^{K} u_{jk}^2}$$

et

$$\beta = \frac{\sum\limits_{j=1}^{J} \sum\limits_{k=1}^{K} u_{jk} \cdot \left( v_{jk} - \left|H_{jk}\right|^2 \right)}{\sum\limits_{j=1}^{J} \sum\limits_{k=1}^{K} u_{jk}^2}$$

Si on a L émetteurs 10 avec L supérieur ou égal à 2, on aura un troisième indice 1 appartenant à [1, L] et on cherche un paramètre $\beta_1$ par émetteur $10_1$, selon les équations suivantes :

$$\rho_{jl} = \frac{\sum\limits_{k=1}^{K} u_{jkl} \cdot \left( \left|H_{jkl}\right|^2 + \left|Ip_{jkl}\right|^2 - 2 \cdot v_{jkl} \right)}{\sum\limits_{k=1}^{K} u_{jkl}^2}$$

et

$$\beta_l = \frac{\sum\limits_{j=1}^{J} \sum\limits_{k=1}^{K} u_{jkl} \cdot \left( v_{jkl} - \left|H_{jkl}\right|^2 \right)}{\sum\limits_{j=1}^{J} \sum\limits_{k=1}^{K} u_{jkl}^2}$$

On a dans ce cas 2JKL mesures pour L(J+1) inconnues. Suivant un mode de réalisation, $2.J.K.L \geq L.(J+1)$.
On remarque que l'augmentation du nombre de fréquences par axes n'augmente pas le nombre d'inconnues et donc accroît la redondance.

**Revendications**

1.  Procédé de compensation d'un localisateur magnétique (1) en présence d'au moins un matériau perturbateur de champ magnétique, ledit localisateur magnétique (1) comprenant :

    - au moins un émetteur (10) comprenant au moins une (Ne) bobine émettrice (El, E2, E3) et au moins un générateur (101, 102, 103) d'au moins un signal d'émission, relié à la au moins une bobine émettrice (E1, E2, E3), pour que la au moins une bobine émettrice (El, E2, E3) émette au moins un champ magnétique (Bel, Be2, Be3) d'émission à au moins une pulsation déterminée $\omega_i$ en réponse au signal d'émission qui lui est envoyé par le générateur (101, 102, 103),
    - au moins un récepteur (20) comprenant au moins une (Nr) bobine réceptrice (R1, R2, R3) et un dispositif (24) de mesure, qui est relié à la au moins une bobine réceptrice (R1, R2, R3) et qui fournit au moins une mesure Ipi d'un champ magnétique de réception (Br11, Br21, Br31 ; Br12, Br22, Br32 ; Br13, Br23, Br33) induit respectivement par le champ magnétique (Bel, Be2, Be3) d'émission dans chaque bobine réceptrice (R1, R2, R3), de manière à fournir une ou plusieurs mesures Ipi pour i allant de 1 à N, avec N = Nr.Ne, où Ne est un premier nombre de bobine émettrice (EI, E2, E3), Nr est un deuxième nombre de bobine réceptrice (R1, R2, R3), Ne ≥ 1, Nr ≥ 1,

- une unité (25) de traitement comportant un modèle de champ permettant de calculer une position (P) et/ou une orientation (Q) du récepteur (20) par calcul d'une prédiction Hi de la ou des mesures en fonction d'un critère (C) calculé en fonction d'une erreur $E_i$, elle-même calculée par rapport à la ou aux mesures Ipi,

l'erreur Ei étant calculée par itérations successives depuis des valeurs initiales prescrites de la prédiction Hi comme étant la différence entre la ou les mesures Ipi et un modèle perturbé Hpi de la ou des mesures, selon l'équation

$$E_i = Ip_i - Hp_i,$$

le modèle perturbé Hpi de la ou des mesures vérifiant les équations suivantes

$$Hp_i = H_i + P_i,$$

$$P_i = \rho_i \frac{jH_i}{|H_i|} e^{j\alpha_i} \ ,$$

$$\alpha_i = -\arctan(\beta. \omega_i),$$

où $P_i$ est la perturbation apportée à la ou aux mesures Ipi par le matériau perturbateur (3) de champ magnétique, $\rho_i$ est l'intensité de la perturbation, $\alpha_i$ est un angle de déphasage provoqué par le matériau perturbateur (3) de champ magnétique, $\beta$ étant un paramètre du matériau perturbateur (3) de champ magnétique,

le paramètre $\beta$ étant identique pour toutes les mesures Ipi,

le calcul étant effectué de manière à minimiser le critère (C),

**caractérisé en ce que**

à chaque itération

- on initialise la prédiction Hi aux valeurs initiales prescrites,
- puis on calcule $\Delta_i$, le paramètre $\beta$ comme minimisant le vecteur A.$\beta$ - B sur ses coordonnées, et le modèle perturbé Hpi en fonction de Hi et Ipi selon les équations

$$\Delta_i = [\mathcal{R}(H_i).\mathcal{I}(Ip_i) - \mathcal{I}(H_i).\mathcal{R}(Ip_i)]/[(\mathcal{I}(H_i)^2 + \mathcal{R}(H_i)^2] \ ,$$

$$Hp_i = H_i.(1+\Delta_i.(j+ \beta.\omega_i)) \ ,$$

où A est un vecteur, dont des premières et deuxièmes coordonnées sont formées de respectivement :

$$A_{2i} = \Delta_i.\mathcal{R}(H_i).\omega_i$$

et

$$A_{2i+1} = \Delta_i.\mathcal{I}(H_i).\omega_i \ ,$$

B est un vecteur, dont des premières et deuxièmes coordonnées sont formées de respectivement:

$$B_{2i} = \mathscr{R}(\mathrm{Ip_i}) - \mathscr{R}(\mathrm{H_i}) + \Delta_i.\mathscr{I}(\mathrm{H_i}) \text{ et } B_{2i+1} = \mathscr{I}(\mathrm{Ip_i}) - \mathscr{I}(\mathrm{H_i}) - \Delta_i.\mathscr{R}(\mathrm{H_i}),$$

pour i allant de 1 à N, où

$\mathscr{R}(\mathrm{H_i})$ est la partie réelle de la prédiction Hi,

$\mathscr{I}(\mathrm{H_i})$ est la partie imaginaire de la prédiction Hi,

$\mathscr{R}(\mathrm{Ip_i})$ est la partie réelle de la mesure $\mathrm{Ip}_i$,

$\mathscr{I}(\mathrm{Ip_i})$ est la partie imaginaire de la mesure Ipi,

- puis on calcule l'erreur $E_i$,
- puis on calcule le critère C suivant l'équation

$$C = \sum_{i=0}^{N} \left| E_i \right|^2,$$

- puis on calcule par le modèle de champ la prédiction Hi qui correspond au critère C,

cette prédiction Hi calculée étant utilisée pour l'itération suivante jusqu'à ce que le critère C calculé devienne inférieur à une borne positive non nulle η, prescrite.

2. Procédé de compensation d'un localisateur magnétique (1) en présence d'au moins un matériau perturbateur de champ magnétique, ledit localisateur magnétique (1) comprenant :

- au moins un émetteur (10) comprenant au moins une (Ne) bobine émettrice (El, E2, E3) et au moins un générateur (101, 102, 103) d'au moins un signal d'émission, relié à la au moins une bobine émettrice (E1, E2, E3), pour que la au moins une bobine émettrice (E1, E2, E3), émette au moins un champ magnétique (Be1, Be2, Be3) d'émission à au moins une pulsation déterminée $\omega_i$ en réponse au signal d'émission qui lui est envoyé par le générateur (101, 102, 103),
- au moins un récepteur (20) comprenant au moins une (Nr) bobine réceptrice (R1, R2, R3) et un dispositif (24) de mesure, qui est relié à la au moins une bobine réceptrice (R1, R2, R3) et qui fournit au moins une mesure Ipi d'un champ magnétique de réception (Br11, Br21, Br31 ; Br12, Br22, Br32 ; Br13, Br23, Br33) induit respectivement par le champ magnétique (Be1, Be2, Be3) d'émission dans chaque bobine réceptrice (R1, R2, R3), de manière à fournir une ou plusieurs mesures Ipi pour i allant de 1 à N, avec N = Nr.Ne, où Ne est un premier nombre de bobine émettrice (El, E2, E3), Nr est un deuxième nombre de bobine réceptrice (R1, R2, R3), Ne $\geq$ 1, Nr $\geq$ 1,
- une unité (25) de traitement comportant un modèle de champ permettant de calculer une position (P) et/ou une orientation (Q) du récepteur (20) par calcul d'une prédiction Hi de la ou des mesures en fonction d'un critère (C ) calculé en fonction d'une erreur $E_i$, elle-même calculée par rapport à la ou aux mesures Ipi,

l'erreur Ei étant calculée par itérations successives depuis des valeurs initiales prescrites de la prédiction Hi comme étant la différence entre les mesures Ipi et un modèle perturbé Hpi de la ou des mesures, selon l'équation

$$E_i = \mathrm{Ip_i} - \mathrm{Hp_i},$$

le modèle perturbé Hpi de la ou des mesures vérifiant les équations suivantes

$$\mathrm{Hp_i} = \mathrm{H_i} + \mathrm{P_i},$$

$$P_i = \rho_i \frac{jH_i}{|H_i|} e^{j\alpha_i} \quad ,$$

$$\alpha_i = -\arctan(\beta. \omega_i ),$$

où Pi est la perturbation apportée à la ou aux mesures Ipi par le matériau perturbateur (3) de champ magnétique, $\rho_i$ est l'intensité de la perturbation, $\alpha_i$ est un angle de déphasage provoqué par le matériau perturbateur (3) de champ magnétique, $\beta$ étant un paramètre du matériau perturbateur (3) de champ magnétique,
le paramètre $\beta$ étant identique pour toutes les mesures Ipi,
le calcul étant effectué de manière à minimiser le critère (C ),
**caractérisé en ce que**
à chaque itération

- on initialise la prédiction Hi aux valeurs initiales prescrites,
- puis on calcule $\Delta_i$, le paramètre $\beta$ et le modèle perturbé Hpi en fonction de $H_i$ et Ipi selon les équations

$$\Delta_i = [\mathcal{R}(H_i).\mathcal{I}(Ip_i) - \mathcal{I}(H_i).\mathcal{R}(Ip_i)]/[(\mathcal{I}(H_i)^2 + \mathcal{R}(H_i)^2] \ ,$$

$$\beta = \frac{A^T \cdot B}{|A|^2}$$

$$Hp_i = H_i.(1+\Delta_i.(j+ \beta.\omega_i)) \ ,$$

où A est un vecteur, dont des premières et deuxièmes coordonnées sont formées de respectivement :

$$A_{2i} = \Delta_i.\mathcal{R}(H_i).\omega_i$$

et

$$A_{2i+1} = \Delta_i.\mathcal{I}(H_i).\omega_i \ ,$$

B est un vecteur, dont des premières et deuxièmes coordonnées sont formées de respectivement:

$$B_{2i} = \mathcal{R}(Ip_i) - \mathcal{R}(H_i) + \Delta_i.\mathcal{I}(H_i)$$

et

$$B_{2i+1} = \mathcal{I}(Ip_i) - \mathcal{I}(H_i) - \Delta_i.\mathcal{R}(H_i),$$

pour i allant de 1 à N, où

$\mathcal{R}(H_i)$ est la partie réelle de la prédiction Hi,

$\mathcal{I}(H_i)$ est la partie imaginaire de la prédiction Hi,

$\mathcal{R}(Ip_i)$ est la partie réelle de la mesure Ipi,

$\mathcal{I}(\mathrm{Ip}i)$ est la partie imaginaire de la mesure Ipi.

- puis on calcule l'erreur E$_i$,
- puis on calcule le critère C suivant l'équation

$$C = \sum_{i=0}^{N} \left| E_i \right|^2 \; ,$$

- puis on calcule par le modèle de champ la prédiction Hi qui correspond au critère C,

cette prédiction Hi calculée étant utilisée pour l'itération suivante jusqu'à ce que le critère C calculé devienne inférieur à une borne positive non nulle η, prescrite.

3. Procédé de compensation d'un localisateur magnétique (1) en présence d'au moins un matériau perturbateur de champ magnétique, ledit localisateur magnétique (1) comprenant :

- au moins un émetteur (10) comprenant au moins une (Ne) bobine émettrice (E1, E2, E3) et au moins un générateur (101, 102, 103) d'au moins un signal d'émission, relié à la au moins une bobine émettrice (E1, E2, E3), pour que la au moins une bobine émettrice (E1, E2, E3) émette au moins un champ magnétique (Be1, Be2, Be3) d'émission à au moins une pulsation déterminée $\omega_i$ en réponse au signal d'émission qui lui est envoyé par le générateur (101, 102, 103),
- au moins un récepteur (20) comprenant au moins une (Nr) bobine réceptrice (R1, R2, R3) et un dispositif (24) de mesure, qui est relié à la au moins une bobine réceptrice (R1, R2, R3) et qui fournit au moins une mesure Ipi d'un champ magnétique de réception (Br11, Br21, Br31 ; Br12, Br22, Br32 ; Br13, Br23, Br33) induit respectivement par le champ magnétique (Be1, Be2, Be3) d'émission dans chaque bobine réceptrice (R1, R2, R3), de manière à fournir une ou plusieurs mesures Ipi pour i allant de 1 à N, avec N = Nr.Ne, où Ne est un premier nombre de bobine émettrice (EI, E2, E3), Nr est un deuxième nombre de bobine réceptrice (R1, R2, R3), Ne ≥ 1, Nr ≥ 1,
- une unité (25) de traitement comportant un modèle de champ permettant de calculer une position (P) et/ou une orientation (Q) du récepteur (20) par calcul d'une prédiction Hi de la ou des mesures en fonction d'un critère (C ) calculé en fonction d'une erreur E$_i$, elle-même calculée par rapport à la ou aux mesures Ipi,

l'erreur Ei étant calculée par itérations successives depuis des valeurs initiales prescrites de la prédiction Hi comme étant la différence entre la ou les mesures Ipi et un modèle perturbé Hpi de la ou des mesures, selon l'équation

$$\mathrm{E}i = \mathrm{Ip}i - \mathrm{Hp}i,$$

le modèle perturbé Hpi de la ou des mesures vérifiant les équations suivantes

$$\mathrm{Hp}i = \mathrm{H}_i + \mathrm{P}_i,$$

$$P_i = \rho_i \frac{jH_i}{\left| H_i \right|} e^{j\alpha_i} \; ,$$

$$\alpha_i = -\arctan(\beta . \, \omega_i ),$$

où Pi est la perturbation apportée à la ou aux mesures Ipi par le matériau perturbateur (3) de champ magnétique, $\rho_i$ est l'intensité de la perturbation, $\alpha_i$ est un angle de déphasage provoqué par le matériau perturbateur (3) de champ magnétique, β étant un paramètre du matériau perturbateur (3) de champ magnétique,

le paramètre β étant identique pour toutes les mesures Ipi,

le calcul étant effectué de manière à minimiser le critère (C ),

**caractérisé en ce que**

à chaque itération

- on initialise la prédiction Hi aux valeurs initiales prescrites,
- puis on calcule le paramètre β comme minimisant le vecteur A'.β+B' sur ses coordonnées, puis les $\Delta_i$ et le modèle perturbé Hpi en fonction de Hi et Ipi selon les équations :

$$\Delta_i = [\mathcal{I}(Ip_i) - \mathcal{I}(H_i)]/[\mathcal{R}(H_i).+ \mathcal{I}(H_i).\beta.\omega_i]$$

$$Hp_i = H_i.(1+\Delta_i.(j+ \beta.\omega_i))$$

où A' est un vecteur, dont les coordonnées sont formées de respectivement :

$$\omega_i.[\mathcal{R}(H_i).\mathcal{I}(Ip_i) - \mathcal{R}(Ip_i).\mathcal{I}(H_i)] \; ,$$

B' est un vecteur, dont les coordonnées sont formées de respectivement:

$$\mathcal{R}(H_i)^2 + \mathcal{I}(H_i)^2 - (\mathcal{R}(H_i).\mathcal{R}(Ip_i)+\mathcal{I}(H_i).(Ip_i)),$$

pour i allant de 1 à N, où

$\mathcal{R}(H_i)$ est la partie réelle de la prédiction Hi,

$\mathcal{I}(H_i)$ est la partie imaginaire de la prédiction Hi,

$\mathcal{R}(Ip_i)$ est la partie réelle de la mesure Ipi,

$\mathcal{I}(Ip_i)$ est la partie imaginaire de la mesure Ipi,

- puis on calcule l'erreur $E_i$,
- puis on calcule le critère C suivant l'équation

$$C = \sum_{i=0}^{N}\left|E_i\right|^2 \; ,$$

- puis on calcule par le modèle de champ la prédiction Hi qui correspond au critère C,

cette prédiction Hi calculée étant utilisée pour l'itération suivante jusqu'à ce que le critère C calculé devienne inférieur à une borne positive non nulle η, prescrite.

4. Procédé de compensation d'un localisateur magnétique (1) en présence d'au moins un matériau perturbateur de champ magnétique, ledit localisateur magnétique (1) comprenant :

- au moins un émetteur (10) comprenant au moins une (Ne) bobine émettrice (E1, E2, E3) et au moins un générateur (101, 102, 103) d'au moins un signal d'émission, relié à la au moins une bobine émettrice (E1, E2, E3), pour que la au moins une bobine émettrice (E1, E2, E3) émette au moins un champ magnétique (Be1, Be2, Be3) d'émission à au moins une pulsation déterminée $\omega_i$ en réponse au signal d'émission qui lui est envoyé par le générateur (101, 102, 103),
- au moins un récepteur (20) comprenant au moins une (Nr) bobine réceptrice (R1, R2, R3) et un dispositif (24)

de mesure, qui est relié à la au moins une bobine réceptrice (R1, R2, R3) et qui fournit au moins une mesure $Ip_i$ d'un champ magnétique de réception (Br11, Br21, Br31 ; Br12, Br22, Br32 ; Br13, Br23, Br33) induit respectivement par le champ magnétique (Be1, Be2, Be3) d'émission dans chaque bobine réceptrice (R1, R2, R3), de manière à fournir une ou plusieurs mesures Ipi pour i allant de 1 à N, avec N = Nr.Ne, où Ne est un premier nombre de bobine émettrice (E1, E2, E3), Nr est un deuxième nombre de bobine réceptrice (R1, R2, R3), Ne $\geq$ 1, Nr $\geq$ 1,
- une unité (25) de traitement comportant un modèle de champ permettant de calculer une position (P) et/ou une orientation (Q) du récepteur (20) par calcul d'une prédiction Hi de la ou des mesures en fonction d'un critère (C ) calculé en fonction d'une erreur $E_i$, elle-même calculée par rapport à la ou aux mesures Ipi,

l'erreur Ei étant calculée par itérations successives depuis des valeurs initiales prescrites de la prédiction Hi comme étant la différence entre la ou les mesures Ipi et un modèle perturbé Hpi de la ou des mesures, selon l'équation

$$E_i = Ip_i - Hp_i,$$

le modèle perturbé Hpi de la des mesures vérifiant les équations suivantes

$$Hp_i = H_i + P_i,$$

$$P_i = \rho_i \frac{jH_i}{|H_i|} e^{j\alpha_i} \quad,$$

$$\alpha_i = -\arctan(\beta. \omega_i),$$

où Pi est la perturbation apportée à la ou aux mesures Ipi par le matériau perturbateur (3) de champ magnétique, $\rho_i$ est l'intensité de la perturbation, $\alpha_i$ est un angle de déphasage provoqué par le matériau perturbateur (3) de champ magnétique, $\beta$ étant un paramètre du matériau perturbateur (3) de champ magnétique,
le paramètre $\beta$ étant identique pour toutes les mesures Ipi,
le calcul étant effectué de manière à minimiser le critère (C ),
**caractérisé en ce que**
à chaque itération

- on initialise la prédiction Hi aux valeurs initiales prescrites,
- puis on calcule le paramètre $\beta$ puis les $\Delta_i$ et le modèle perturbé Hpi en fonction de Hi et Ipi selon les équations :

$$\beta = \frac{A'^T \cdot B'}{|A'|^2}$$

$$\Delta_i = [\mathcal{I}(Ip_i) - \mathcal{I}(H_i)]/[\mathcal{R}(H_i).+ \mathcal{I}(H_i).\beta.\omega_i]$$

$$Hp_i = H_i.(1+\Delta_i.(j+ \beta.\omega_i))$$

où A' est un vecteur, dont les coordonnées sont formées de respectivement :

$$\omega_i.[\mathcal{R}(H_i).\mathcal{I}(Ip_i) - \mathcal{R}(Ip_i).\mathcal{I}(H_i)] \quad,$$

B' est un vecteur, dont les coordonnées sont formées de respectivement:

$$\mathcal{R}(H_i)^2 + \mathcal{I}(H_i)^2 - (\mathcal{R}(H_i).\mathcal{R}(Ip_i)+\mathcal{I}(H_i).(Ip_i)),$$

pour i allant de 1 à N, où

$\mathcal{R}(H_i)$ est la partie réelle de la prédiction Hi,

$\mathcal{I}(H_i)$ est la partie imaginaire de la prédiction Hi,

$\mathcal{R}(Ip_i)$ est la partie réelle de la mesure Ipi,

$\mathcal{I}(Ip_i)$ est la partie imaginaire de la mesure Ipi,

- puis on calcule l'erreur $E_i$,
- puis on calcule le critère C suivant l'équation

$$C = \sum_{i=0}^{N} \left| E_i \right|^2 ,$$

- puis on calcule par le modèle de champ la prédiction Hi qui correspond au critère C,

cette prédiction Hi calculée étant utilisée pour l'itération suivante jusqu'à ce que le critère C calculé devienne inférieur à une borne positive non nulle η, prescrite.

5. Procédé de compensation suivant l'une quelconque des revendications 1 à 4, **caractérisé en ce que** le modèle de champ permettant de calculer une position (P) et/ou une orientation (Q) du récepteur (20) par calcul de la prédiction Hi de la ou des mesures en fonction du critère (C ) utilise un algorithme de minimisation de Levenberg-Marquardt.

6. Procédé de compensation suivant l'une quelconque des revendications précédentes, **caractérisé en ce que** les pulsations déterminées $\omega_i$ sont distinctes l'une de l'autre.

7. Procédé de compensation suivant l'une quelconque des revendications précédentes, **caractérisé en ce que** l'émetteur (10) comporte une pluralité Ne de bobines émettrices (El, E2, E3) émettant respectivement une pluralité Ne de champs magnétiques (Bel, Be2, Be3) d'émission, avec Ne ≥ 2,
le dispositif (24) de mesure mesurant pour chaque bobine réceptrice (R1, R2, R3) une pluralité Ne de champs magnétiques de réception (Br11, Br21, Br31 ; Br12, Br22, Br32 ; Brl3, Br23, Br33) induits respectivement par la pluralité Ne de champs magnétiques (Bel, Be2, Be3) d'émission dans la bobine réceptrice (R1, R2, R3) et formant les mesures Ipi pour i allant de 1 à N.

8. Procédé de compensation suivant l'une quelconque des revendications précédentes, **caractérisé en ce que** le localisateur (1) comporte une bobine émettrice (El, E2, E3) émettant à K pulsations différentes $\omega_{jk}$ et J bobines réceptrices (R1, R2, R3), le dispositif (24) de mesure fournissant les mesures $Ip_{jk}$ pour l'indice j allant de 1 à J et l'indice k allant de 1 à K,
l'unité (25) de traitement calcule

$$\rho_j = \frac{\sum_{k=1}^{K} u_{jk} \cdot \left( \left| H_{jk} \right|^2 + \left| Ip_{jk} \right|^2 - 2 \cdot v_{jk} \right)}{\sum_{k=1}^{K} u_{jk}^2}$$

et

$$\beta = \frac{\sum_{j=1}^{J}\sum_{k=1}^{K} u_{jk} \cdot \left(v_{jk} - \left|H_{jk}\right|^2\right)}{\sum_{j=1}^{J}\sum_{k=1}^{K} u_{jk}^2}$$

avec

$$v_{jk} = \Re(H_{jk}) \cdot \Re(Ip_{jk}) + \Im(H_{jk}) \cdot \Im(Ip_{jk})$$

$$u_{jk} = \left(\Re(H_{jk}) \cdot \Im(Ip_{jk}) - \Im(H_{jk}) \cdot \Re(Ip_{jk})\right) \cdot \omega_{jk}$$

$$Ip_{jk} = H_{jk}.(1 + \Delta_{jk}.(j + \beta.\omega_{jk}))$$

$$\Delta_{jk} = \rho_j \frac{\omega_{jk}}{1 + \left(\beta \cdot \omega_{jk}\right)^2}$$

où $H_{jk}$ est la prédiction, $\rho_j$ est l'intensité de la perturbation, $Hp_{jk}$ est le modèle perturbé et $Hp_{jk}$ - $Ip_{jk}$ =0.

9. Procédé de compensation suivant l'une quelconque des revendications précédentes, **caractérisé en ce que** le localisateur (1) comporte L bobines émettrices (EI, E2, E3) ou L émetteurs (10), qui émettent à K pulsations différentes $\omega_{jkl}$, et J bobines réceptrices (R1, R2, R3), le dispositif (24) de mesure fournissant les mesures $Ip_{jkl}$ pour l'indice j allant de 1 à J, l'indice k allant de 1 à K et l'indice 1 allant de 1 à L, l'unité (25) de traitement calcule

$$\rho_{jl} = \frac{\sum_{k=1}^{K} u_{jkl} \cdot \left(\left|H_{jkl}\right|^2 + \left|Ip_{jkl}\right|^2 - 2 \cdot v_{jkl}\right)}{\sum_{k=1}^{K} u_{jkl}^2}$$

et

$$\beta_l = \frac{\sum_{j=1}^{J}\sum_{k=1}^{K} u_{jkl} \cdot \left(v_{jkl} - \left|H_{jkl}\right|^2\right)}{\sum_{j=1}^{J}\sum_{k=1}^{K} u_{jkl}^2}$$

avec

$$v_{jkl} = \Re(H_{jkl}) \cdot \Re(Ip_{jkl}) + \Im(H_{jkl}) \cdot \Im(Ip_{jkl})$$

$$u_{jkl} = \left(\Re(H_{jkl}) \cdot \Im(Ip_{jkl}) - \Im(H_{jkl}) \cdot \Re(Ip_{jkl})\right) \cdot \omega_{jkl}$$

$$Ip_{jkl} = H_{jkl}.(1+\Delta_{jkl}.(j+ \beta.\omega_{jkl}))$$

$$\Delta_{jkl} = \rho_j \frac{\omega_{jkl}}{1+\left(\beta \cdot \omega_{jkl}\right)^2}$$

où $H_{jkl}$ est la prédiction, $\rho_{jl}$ est l'intensité de la perturbation, $Hp_{jkl}$ est le modèle perturbé et $Hp_{jkl}$ - $Ip_{jkl}$ =0.

10. Localisateur magnétique (1) comprenant :

- au moins un émetteur (10) comprenant au moins une (Ne) bobine émettrice (El, E2, E3) et au moins un générateur (101, 102, 103) apte à générer au moins un signal d'émission, relié à la au moins une bobine émettrice (El, E2, E3), pour que la au moins une bobine émettrice (El, E2, E3) soit apte à émettre au moins un champ magnétique (Bel, Be2, Be3) d'émission à au moins une pulsation déterminée $\omega_i$ en réponse au signal d'émission qui lui est envoyé par le générateur (101, 102, 103),
- au moins un récepteur (20) comprenant au moins une (Nr) bobine réceptrice (R1, R2, R3) et un dispositif (24) de mesure, qui est apte à être relié à la au moins une bobine réceptrice (R1, R2, R3) et qui est apte à fournir au moins une mesure Ipi d'un champ magnétique de réception (Br11, Br21, Br31 ; Br12, Br22, Br32 ; Brl3, Br23, Br33) induit respectivement par le champ magnétique (Bel, Be2, Be3) d'émission dans chaque bobine réceptrice (R1, R2, R3), pour fournir une ou plusieurs mesures Ipi pour i allant de 1 à N, avec N = Nr.Ne, où Ne est un premier nombre de bobine émettrice (El, E2, E3), Nr est un deuxième nombre de bobine réceptrice (R1, R2, R3), Ne $\geq$ 1, Nr $\geq$ 1,
- une unité (25) de traitement comportant un modèle de champ permettant de calculer une position (P) et/ou une orientation (Q) du récepteur (20) par calcul d'une prédiction Hi de la ou des mesures en fonction d'un critère (C ) calculé en fonction d'une erreur $E_i$, elle-même calculée par rapport à la ou aux mesures Ipi, l'unité (25) de traitement étant configurée pour que l'erreur Ei soit calculée par itérations successives depuis des valeurs initiales prescrites de la prédiction Hi comme étant la différence entre la ou les mesures Ipi et un modèle perturbé Hpi de la ou des mesures, selon l'équation

$$E_i = Ip_i - Hp_i,$$

le modèle perturbé Hpi des mesures vérifiant les équations suivantes

$$Hp_i = H_i + P_i,$$

$$P_i = \rho_i \frac{jH_i}{|H_i|} e^{j\alpha_i} \quad ,$$

$$\alpha_i = -\arctan(\beta. \omega_i ),$$

où Pi est une perturbation apportée à la ou aux mesures Ipi par un matériau perturbateur (3) de champ magnétique, $\rho_i$ est l'intensité de la perturbation, $\alpha_i$ est un angle de déphasage provoqué par le matériau perturbateur (3) de champ magnétique, $\beta$ étant un paramètre du matériau perturbateur (3) de champ magnétique,
le paramètre $\beta$ étant identique pour toutes les mesures Ipi,
l'unité (25) de traitement étant configurée pour effectuer le calcul de manière à minimiser le critère (C ), **caractérisé en ce que**
l'unité (25) de traitement est configurée pour mettre en œuvre les étapes suivantes à chaque itération :

- on initialise la prédiction Hi aux valeurs initiales prescrites,
- puis on calcule $\Delta_i$, le paramètre $\beta$ comme minimisant le vecteur A.$\beta$ - B sur ses coordonnées, et le modèle perturbé Hpi en fonction de Hi et Ipi selon les équations

$$\Delta_i = [\mathcal{R}(H_i).\mathcal{I}(Ip_i) - \mathcal{I}(H_i).\mathcal{R}(Ip_i)]/[(\mathcal{I}(H_i)^2 + \mathcal{R}(H_i)^2] \ ,$$

$$Hp_i = H_i.(1+\Delta_i.(j+ \beta.\omega_i)) \ ,$$

où A est un vecteur, dont des premières et deuxièmes coordonnées sont formées de respectivement :

$$A_{2i} = \Delta_i.\mathcal{R}(H_i).\omega_i$$

et

$$A_{2i+1} = \Delta_i.\mathcal{I}(H_i).\omega_i \ ,$$

B est un vecteur, dont des premières et deuxièmes coordonnées sont formées de respectivement:

$$B_{2i} = \mathcal{R}(Ip_i) - \mathcal{R}(H_i) + \Delta_i.\mathcal{I}(H_i)$$

et

$$B_{2i+1} = \mathcal{I}(Ip_i) - \mathcal{I}(H_i) - \Delta_i.\mathcal{R}(H_i),$$

pour i allant de 1 à N, où

$\mathcal{R}(H_i)$ est la partie réelle de la prédiction Hi,

$\mathcal{I}(H_i)$ est la partie imaginaire de la prédiction Hi,

$\mathcal{R}(Ip_i)$ est la partie réelle de la mesure Ipi,

$\mathcal{I}(Ip_i)$ est la partie imaginaire de la mesure Ipi,

- puis on calcule l'erreur $E_i$,
- puis on calcule le critère C suivant l'équation

$$C = \sum_{i=0}^{N} \left| E_i \right|^2 \ ,$$

- puis on calcule par le modèle de champ la prédiction Hi qui correspond au critère C,

cette prédiction Hi calculée étant utilisée pour l'itération suivante jusqu'à ce que le critère C calculé devienne inférieur à une borne positive non nulle $\eta$, prescrite.

**11.** Localisateur magnétique (1) comprenant :

- au moins un émetteur (10) comprenant au moins une (Ne) bobine émettrice (El, E2, E3) et au moins un générateur (101, 102, 103) apte à générer au moins un signal d'émission, relié à la au moins une bobine émettrice (E1, E2, E3), pour que la au moins une bobine émettrice (E1, E2, E3) soit apte à émettre au moins un champ magnétique (Be1, Be2, Be3) d'émission à au moins une pulsation déterminée $\omega_i$ en réponse au signal d'émission qui lui est envoyé par le générateur (101, 102, 103),
- au moins un récepteur (20) comprenant au moins une (Nr) bobine réceptrice (R1, R2, R3) et un dispositif (24)

de mesure, qui est apte à être relié à la au moins une bobine réceptrice (R1, R2, R3) et qui est apte à fournir au moins une mesure Ipi d'un champ magnétique de réception (Br11, Br21, Br31 ; Br12, Br22, Br32 ; Br13, Br23, Br33) induit respectivement par le champ magnétique (Be1, Be2, Be3) d'émission dans chaque bobine réceptrice (R1, R2, R3), pour fournir une ou plusieurs mesures $Ip_i$ pour i allant de 1 à N, avec N = Nr.Ne, où Ne est un premier nombre de bobine émettrice (E1, E2, E3), Nr est un deuxième nombre de bobine réceptrice (R1, R2, R3), Ne $\geq$ 1, Nr $\geq$ 1,

- une unité (25) de traitement comportant un modèle de champ permettant de calculer une position (P) et/ou une orientation (Q) du récepteur (20) par calcul d'une prédiction $H_i$ de la ou des mesures en fonction d'un critère (C) calculé en fonction d'une erreur $E_i$, elle-même calculée par rapport à la ou aux mesures $Ip_i$,

l'unité (25) de traitement étant configurée pour que l'erreur $E_i$ soit calculée par itérations successives depuis des valeurs initiales prescrites de la prédiction Hi comme étant la différence entre la ou les mesures $Ip_i$ et un modèle perturbé $Hp_i$ de la ou des mesures, selon l'équation

$$E_i = Ip_i - Hp_i,$$

le modèle perturbé $Hp_i$ de la ou des mesures vérifiant les équations suivantes

$$Hp_i = H_i + P_i,$$

$$P_i = \rho_i \frac{jH_i}{|H_i|} e^{j\alpha_i} \quad,$$

$$\alpha_i = -\arctan(\beta.\, \omega_i),$$

où $P_i$ est une perturbation apportée à la ou aux mesures $Ip_i$ par un matériau perturbateur (3) de champ magnétique, $\rho_i$ est l'intensité de la perturbation, $\alpha_i$ est un angle de déphasage provoqué par le matériau perturbateur (3) de champ magnétique, $\beta$ étant un paramètre du matériau perturbateur (3) de champ magnétique,

le paramètre $\beta$ étant identique pour toutes les mesures $Ip_i$,

**caractérisé en ce que**

l'unité (25) de traitement est configurée pour effectuer le calcul de manière à minimiser le critère (C),

l'unité (25) de traitement étant configurée pour mettre en œuvre les étapes suivantes à chaque itération :

- on initialise la prédiction $H_i$ aux valeurs initiales prescrites,
- puis on calcule $\Delta_i$, le paramètre $\beta$ et le modèle perturbé Hpi en fonction de $H_i$ et Ipi selon les équations

$$\Delta_i = [\mathscr{R}(H_i).\mathscr{I}(Ip_i) - \mathscr{I}(H_i).\mathscr{R}(Ip_i)]/[(\mathscr{I}(H_i)^2 + \mathscr{R}(H_i)^2] \;,$$

$$\beta = \frac{A^T \cdot B}{|A|^2}$$

$$Hp_i = H_i.(1 + \Delta_i.(j + \beta.\omega_i)) \;,$$

où A est un vecteur, dont des premières et deuxièmes coordonnées sont formées de respectivement :

$$A_{2i} = \Delta_i.\mathscr{R}(H_i).\omega_i$$

et

$$A_{2i+1} = \Delta_i.\mathcal{I}(H_i).\omega_i \ ,$$

B est un vecteur, dont des premières et deuxièmes coordonnées sont formées de respectivement:

$$B_{2i} \ = \ \mathcal{R}(Ip_i) \ - \ \mathcal{R}(H_i) \ + \ \Delta_i.\mathcal{I}(H_i)$$

et

$$B_{2i+1} \ = \ \mathcal{I}(Ip_i) \ - \ \mathcal{I}(H_i) \ - \ \Delta_i.\mathcal{R}(H_i),$$

pour i allant de 1 à N, où

$\mathcal{R}(H_i)$ est la partie réelle de la prédiction Hi,

$\mathcal{I}(H_i)$ est la partie imaginaire de la prédiction Hi,

$\mathcal{R}(Ip_i)$ est la partie réelle de la mesure Ipi,

$\mathcal{I}(Ip_i)$ est la partie imaginaire de la mesure Ipi.

- puis on calcule l'erreur $E_i$,
- puis on calcule le critère C suivant l'équation

$$C = \sum_{i=0}^{N} \left| E_i \right|^2 \ ,$$

- puis on calcule par le modèle de champ la prédiction Hi qui correspond au critère C,

cette prédiction Hi calculée étant utilisée pour l'itération suivante jusqu'à ce que le critère C calculé devienne inférieur à une borne positive non nulle η, prescrite.

12. Localisateur magnétique (1) comprenant :

- au moins un émetteur (10) comprenant au moins une (Ne) bobine émettrice (El, E2, E3) et au moins un générateur (101, 102, 103) apte à générer au moins un signal d'émission, relié à la au moins une bobine émettrice (El, E2, E3), pour que la au moins une bobine émettrice (El, E2, E3) soit apte à émettre au moins un champ magnétique (Bel, Be2, Be3) d'émission à au moins une pulsation déterminée $\omega_i$ en réponse au signal d'émission qui lui est envoyé par le générateur (101, 102, 103),
- au moins un récepteur (20) comprenant au moins une (Nr) bobine réceptrice (R1, R2, R3) et un dispositif (24) de mesure, qui est apte à être relié à la au moins une bobine réceptrice (R1, R2, R3) et qui est apte à fournir au moins une mesure Ipi d'un champ magnétique de réception (Br11, Br21, Br31 ; Br12, Br22, Br32 ; Brl3, Br23, Br33) induit respectivement par le champ magnétique (Bel, Be2, Be3) d'émission dans chaque bobine réceptrice (R1, R2, R3), pour fournir une ou plusieurs mesures Ipi pour i allant de 1 à N, avec N = Nr.Ne, où Ne est un premier nombre de bobine émettrice (El, E2, E3), Nr est un deuxième nombre de bobine réceptrice (R1, R2, R3), Ne ≥ 1, Nr ≥ 1,
- une unité (25) de traitement comportant un modèle de champ permettant de calculer une position (P) et/ou une orientation (Q) du récepteur (20) par calcul d'une prédiction Hi de la ou des mesures en fonction d'un critère (C ) calculé en fonction d'une erreur $E_i$, elle-même calculée par rapport à la ou aux mesures Ipi,

l'unité (25) de traitement étant configurée pour que l'erreur Ei soit calculée par itérations successives depuis des valeurs initiales prescrites de la prédiction Hi comme étant la différence entre la ou les mesures Ipi et un modèle perturbé Hpi de la ou des mesures, selon l'équation

$$E_i = Ip_i - Hp_i,$$

le modèle perturbé Hpi de la ou des mesures vérifiant les équations suivantes

$$Hp_i = H_i + P_i,$$

$$P_i = \rho_i \frac{jH_i}{|H_i|} e^{j\alpha_i} ,$$

$$\alpha_i = -\arctan(\beta. \omega_i ),$$

où Pi est une perturbation apportée à la ou aux mesures Ipi par un matériau perturbateur (3) de champ magnétique, $\rho_i$ est l'intensité de la perturbation, $\alpha_i$ est un angle de déphasage provoqué par le matériau perturbateur (3) de champ magnétique, $\beta$ étant un paramètre du matériau perturbateur (3) de champ magnétique,
le paramètre $\beta$ étant identique pour toutes les mesures Ipi,
l'unité (25) de traitement étant configurée pour effectuer le calcul de manière à minimiser le critère (C ),
**caractérisé en ce que**
l'unité (25) de traitement est configurée pour mettre en œuvre les étapes suivantes à chaque itération :

- on initialise la prédiction Hi aux valeurs initiales prescrites,
- puis on calcule le paramètre $\beta$ comme minimisant le vecteur A'.$\beta$+B' sur ses coordonnées, puis les $\Delta_i$ et le modèle perturbé Hpi en fonction de Hi et Ipi selon les équations :

$$\Delta_i = [\mathcal{I}(Ip_i) - \mathcal{I}(H_i)]/[\mathcal{R}(H_i). + \mathcal{I}(H_i).\beta.\omega_i]$$

$$Hp_i = H_i.(1+\Delta_i.(j+ \beta.\omega_i))$$

où A' est un vecteur, dont les coordonnées sont formées de respectivement :

$$\omega_i.[\mathcal{R}(H_i).\mathcal{I}(Ip_i) - \mathcal{R}(Ip_i).\mathcal{I}(H_i)] ,$$

B' est un vecteur, dont les coordonnées sont formées de respectivement:

$$\mathcal{R}(H_i)^2 + \mathcal{I}(H_i)^2 - (\mathcal{R}(H_i).\mathcal{R}(Ip_i)+\mathcal{I}(H_i).(Ip_i)),$$

pour i allant de 1 à N, où

$\mathcal{R}(H_i)$ est la partie réelle de la prédiction Hi,

$\mathcal{I}(H_i)$ est la partie imaginaire de la prédiction Hi,

$\mathcal{R}(Ip_i)$ est la partie réelle de la mesure Ipi,

$\mathcal{I}(Ip_i)$ est la partie imaginaire de la mesure Ipi,

- puis on calcule l'erreur $E_i$,
- puis on calcule le critère C suivant l'équation

$$C = \sum_{i=0}^{N} \left| E_i \right|^2 ,$$

- puis on calcule par le modèle de champ la prédiction Hi qui correspond au critère C,

cette prédiction Hi calculée étant utilisée pour l'itération suivante jusqu'à ce que le critère C calculé devienne inférieur à une borne positive non nulle η, prescrite.

**13.** Localisateur magnétique (1) comprenant :

- au moins un émetteur (10) comprenant au moins une (Ne) bobine émettrice (El, E2, E3) et au moins un générateur (101, 102, 103) apte à générer au moins un signal d'émission, relié à la au moins une bobine émettrice (El, E2, E3), pour que la au moins une bobine émettrice (El, E2, E3) soit apte à émettre au moins un champ magnétique (Bel, Be2, Be3) d'émission à au moins une pulsation déterminée $\omega_i$ en réponse au signal d'émission qui lui est envoyé par le générateur (101, 102, 103),
- au moins un récepteur (20) comprenant au moins une (Nr) bobine réceptrice (R1, R2, R3) et un dispositif (24) de mesure, qui est apte à être relié à la au moins une bobine réceptrice (R1, R2, R3) et qui est apte à fournir au moins une mesure Ipi d'un champ magnétique de réception (Br11, Br21, Br31 ; Br12, Br22, Br32 ; Brl3, Br23, Br33) induit respectivement par le champ magnétique (Bel, Be2, Be3) d'émission dans chaque bobine réceptrice (R1, R2, R3), pour fournir plusieurs mesures Ipi pour i allant de 1 à N, avec N = Nr.Ne, où Ne est un premier nombre de bobine émettrice (El, E2, E3), Nr est un deuxième nombre de bobine réceptrice (R1, R2, R3), Ne ≥ 1, Nr ≥ 1,
- une unité (25) de traitement comportant un modèle de champ permettant de calculer une position (P) et/ou une orientation (Q) du récepteur (20) par calcul d'une prédiction Hi de la ou des mesures en fonction d'un critère (C ) calculé en fonction d'une erreur $E_i$, elle-même calculée par rapport à la ou aux mesures Ipi,

l'unité (25) de traitement étant configurée pour que l'erreur Ei soit calculée par itérations successives depuis des valeurs initiales prescrites de la prédiction Hi comme étant la différence entre la ou les mesures Ipi et un modèle perturbé Hpi de la ou des mesures, selon l'équation

$$E_i = Ip_i - Hp_i,$$

le modèle perturbé Hpi de la ou des mesures vérifiant les équations suivantes

$$Hp_i = H_i + P_i,$$

$$P_i = \rho_i \frac{jH_i}{\left| H_i \right|} e^{j\alpha_i} ,$$

$$\alpha_i = -\arctan(\beta . \omega_i ),$$

où Pi est une perturbation apportée à la ou aux mesures Ipi par un matériau perturbateur (3) de champ magnétique, $\rho_i$ est l'intensité de la perturbation, $\alpha_i$ est un angle de déphasage provoqué par le matériau perturbateur (3) de champ magnétique, β étant un paramètre du matériau perturbateur (3) de champ magnétique,
le paramètre β étant identique pour toutes les mesures Ipi,
l'unité (25) de traitement étant configurée pour effectuer le calcul de manière à minimiser le critère (C ),
**caractérisé en ce que**
l'unité (25) de traitement est configurée pour mettre en œuvre les étapes suivantes à chaque itération :

- on initialise la prédiction Hi aux valeurs initiales prescrites,

- puis on calcule le paramètre $\beta$ puis les $\Delta_i$ et le modèle perturbé Hpi en fonction de Hi et Ipi selon les équations :

$$\beta = \frac{A'^T \cdot B'}{|A'|^2}$$

$$\Delta_i = [\mathscr{I}(Ip_i) - \mathscr{I}(H_i)]/[\mathscr{R}(H_i) + \mathscr{I}(H_i).\beta.\omega_i]$$

$$Hp_i = H_i.(1+\Delta_i.(j+ \beta.\omega_i))$$

où A' est un vecteur, dont les coordonnées sont formées de respectivement :

$$\omega_i.[\mathscr{R}(H_i).\mathscr{I}(Ip_i) - \mathscr{R}(Ip_i).\mathscr{I}(H_i)] \ ,$$

B' est un vecteur, dont les coordonnées sont formées de respectivement:

$$\mathscr{R}(H_i)^2 + \mathscr{I}(H_i)^2 - (\mathscr{R}(H_i).\mathscr{R}(Ip_i)+\mathscr{I}(H_i).(Ip_i)),$$

pour i allant de 1 à N, où

$\mathscr{R}(H_i)$ est la partie réelle de la prédiction Hi,

$\mathscr{I}(H_i)$ est la partie imaginaire de la prédiction Hi,

$\mathscr{R}(Ip_i)$ est la partie réelle de la mesure Ipi,

$\mathscr{I}(Ip_i)$ est la partie imaginaire de la mesure Ipi,

- puis on calcule l'erreur $E_i$,
- puis on calcule le critère C suivant l'équation

$$C = \sum_{i=0}^{N} |E_i|^2 \ ,$$

- puis on calcule par le modèle de champ la prédiction Hi qui correspond au critère C,

cette prédiction $H_i$ calculée étant utilisée pour l'itération suivante jusqu'à ce que le critère C calculé devienne inférieur à une borne positive non nulle $\eta$, prescrite.

14. Localisateur magnétique (1) suivant l'une quelconque des revendications 10 à 13, **caractérisé en ce que** le modèle de champ permettant de calculer une position (P) et/ou une orientation (Q) du récepteur (20) par calcul de la prédiction $H_i$ de la ou des mesures en fonction du critère (C) utilise un algorithme de minimisation de Levenberg-Marquardt.

15. Programme d'ordinateur, comportant des instructions de code qui conduisent le localisateur magnétique selon l'une quelconque des revendications 10-14 à exécuter les étapes du procédé de compensation du localisateur magnétique (1) en présence d'au moins un matériau perturbateur de champ magnétiques suivant l'une quelconque des revendications 1 à 9, lorsque le programme d'ordinateur est exécuté sur un calculateur.

**Patentansprüche**

1. Verfahren zur Kompensation eines magnetischen Lokalisators (1) bei Vorhandensein mindestens eines magnetfeldstörenden Materials, wobei der magnetische Lokalisator (1) umfasst: $\omega$

   - mindestens einen Sender (10), der mindestens eine (Ne) Sendespule (E1, E2, E3) und mindestens einen Generator (101, 102, 103) mindestens eines Sendesignals umfasst, der mit der mindestens einen Sendespule (E1, E2, E3) verbunden ist, damit die mindestens eine Sendespule (E1, E2, E3) als Reaktion auf das Sendesignal, das ihr vom Generator (101, 102, 103) geschickt wird, mindestens ein Sendemagnetfeld (Be1, Be2, Be3) mit mindestens einer bestimmten Pulsation $\omega_i$ sendet,
   - mindestens einen Empfänger (20), der mindestens eine (Nr) Empfangsspule (R1, R2, R3) und eine Messvorrichtung (24) umfasst, die mit der mindestens einen Empfangsspule (R1, R2, R3) verbunden ist und die mindestens eine Messung Ipi eines magnetischen Empfangsfeldes (Br11, Br21, Br31; Br12, Br22, Br32; Br13, Br23, Br33) liefert, das jeweils von dem Sendemagnetfeld (Be1, Be2, Be3) in jeder Empfangsspule (R1, R2, R3) induziert wird, derart, dass eine oder mehrere Messungen Ip$_i$ für i von 1 bis N, mit N = Nr.Ne, geliefert werden, wobei Ne eine erste Sendespulenanzahl (E1, E2, E3) ist und Nr eine zweite Empfangsspulenanzahl (R1, R2, R3) ist, Ne $\geq$ 1, Nr $\geq$ 1,
   - eine Verarbeitungseinheit (25), die ein Feldmodell umfasst, das das Berechnen einer Position (P) und/oder einer Ausrichtung (Q) des Empfängers (20) durch Berechnung einer Voraussage Hi der einen oder mehreren Messungen in Abhängigkeit von einem Kriterium (C) ermöglicht, das in Abhängigkeit von einem Fehler Ei berechnet wird, der selbst in Bezug auf die eine oder mehreren Messungen Ipi berechnet wird,

   wobei der Fehler Ei in aufeinanderfolgenden Iterationen ausgehend von vorgeschriebenen Ausgangswerten der Voraussage H$_i$ als die Differenz zwischen der oder den Messungen Ipi und einem gestörten Modell Hp$_i$ der einen oder mehreren Messungen gemäß der Gleichung E$_i$ = Ip$_i$ - Hp$_i$ berechnet wird,
   wobei das gestörte Modell Hp$_i$ der einen oder mehreren Messungen die folgenden Gleichungen erfüllt:

   $$\mathrm{Hp_i \ = \ H_i \ + \ P_i,}$$

   $$P_i = \rho_i \frac{jH_i}{|H_i|} e^{ja_i},$$

   $$\mathrm{\alpha_i \ = \ -arctan(\beta.\ \omega_i\ ),}$$

   wo P$_i$ die Störung ist, die zu der oder den Messungen Ip$_i$ durch das magnetfeldstörende Material (3) beigetragen wird, $\rho_i$ die Stärke der Störung ist, $\alpha_i$ ein Phasenverschiebungswinkel ist, der von dem magnetfeldstörenden Material (3) verursacht wird, $\beta$ ein Parameter des magnetfeldstörenden Materials (3) ist, wobei der Parameter $\beta$ für alle Messungen Ipi identisch ist, wobei die Berechnung derart durchgeführt wird, dass das Kriterium (C) minimiert wird,
   **dadurch gekennzeichnet, dass**
   bei jeder Iteration

   - die Voraussage H$_i$ auf die vorgeschriebenen Ausgangswerte initialisiert wird,
   - $\Delta_i$, der Parameter $\beta$ als den Vektor A.$\beta$ - B auf seinen Koordinaten minimierend und das gestörte Modell Hpi in Abhängigkeit von Hi und Ipi gemäß den folgenden Gleichungen berechnet werden:

   $$\Delta_\mathrm{i} = [\mathcal{R}(\mathrm{H_i}).\mathcal{I}(\mathrm{Ip_i}) - \mathcal{I}(\mathrm{H_i}).\mathcal{R}(\mathrm{Ip_i})]/[(\mathcal{I}(\mathrm{H_i})^2 + \mathcal{R}(\mathrm{H_i})^2]\ ,$$

   $$\mathrm{Hp_i \ = \ H_i.(1 + \Delta_i.(j + \ \beta.\omega_i)),}$$

   wo A ein Vektor ist, dessen erste und zweite Koordinaten wie folgt gebildet sind:

$$A_{2i} = \Delta_i . \mathcal{R}(H_i) . \omega_i$$

beziehungsweise

$$A_{2i+1} = \Delta_i . \mathcal{I}(H_i) . \omega_i \quad ,$$

B ein Vektor ist, dessen erste und zweite Koordinaten wie folgt gebildet sind:

$$B_{2i} = \mathcal{R}(Ip_i) - \mathcal{R}(H_i) + \Delta_i . \mathcal{I}(H_i)$$

beziehungsweise

$$B_{2i+1} = \mathcal{I}(Ip_i) - \mathcal{I}(H_i) - \Delta_i . \mathcal{R}(H_i),$$

für i von 1 bis N, wo

$\mathcal{R}(H_i)$ der Realteil der Voraussage $H_i$ ist,

$\mathcal{I}(H_i)$ der Imaginärteil der Voraussage $H_i$ ist,

$\mathcal{R}(Ip_i)$ der Realteil der Messung $Ip_i$ ist,

$\mathcal{I}(Ip_i)$ der Imaginärteil der Messung $Ip_i$ ist,

- dann der Fehler $E_i$ berechnet wird,
- dann das Kriterium C gemäß der folgenden Gleichung berechnet wird:

$$C = \sum_{i=0}^{N} |E_i|^2,$$

- dann durch das Feldmodell die Voraussage $H_i$ berechnet wird, die dem Kriterium C entspricht,

wobei diese Voraussage $H_i$ für die folgende Iteration verwendet wird, bis das berechnete Kriterium C kleiner als eine vorgeschriebene positive Grenze $\eta$ von nicht null wird.

2. Verfahren zur Kompensation eines magnetischen Lokalisators (1) beim Vorhandensein mindestens eines magnetfeldstörenden Materials, wobei der magnetische Lokalisator (1) umfasst:

- mindestens einen Sender (10), der mindestens eine (Ne) Sendespule (E1, E2, E3) und mindestens einen Generator (101, 102, 103) mindestens eines Sendesignals umfasst, der mit der mindestens einen Sendespule (E1, E2, E3) verbunden ist, damit die mindestens eine Sendespule (E1, E2, E3) als Reaktion auf das Sendesignal, das ihr vom Generator (101, 102, 103) geschickt wird, mindestens ein Sendemagnetfeld (Be1, Be2, Be3) mit mindestens einer bestimmten Pulsation $\omega_i$ sendet,
- mindestens einen Empfänger (20), der mindestens eine (Nr) Empfangsspule (R1, R2, R3) und eine Messvorrichtung (24) umfasst, die mit der mindestens einen Empfangsspule (R1, R2, R3) verbunden ist und die mindestens eine Messung $Ip_i$ eines magnetischen Empfangsfeldes (Br11, Br21, Br31; Br12, Br22, Br32; Br13, Br23, Br33) liefert, das jeweils von dem Sendemagnetfeld (Be1, Be2, Be3) in jeder Empfangsspule (R1, R2, R3) induziert wird, derart, dass eine oder mehrere Messungen $Ip_i$ für i von 1 bis N, mit N = Nr.Ne, geliefert werden, wobei Ne eine erste Sendespulenanzahl (E1, E2, E3) ist und Nr eine zweite Empfangsspulenanzahl (R1, R2, R3) ist, Ne $\geq$ 1, Nr $\geq$ 1,
- eine Verarbeitungseinheit (25), die ein Feldmodell umfasst, das das Berechnen einer Position (P) und/oder einer Ausrichtung (Q) des Empfängers (20) durch Berechnung einer Voraussage Hi der einen oder mehreren Messungen in Abhängigkeit von einem Kriterium (C) ermöglicht, das in Abhängigkeit von einem Fehler Ei berechnet wird, der selbst in Bezug auf die eine oder mehreren Messungen Ipi berechnet wird,

wobei der Fehler Ei in aufeinanderfolgenden Iterationen ausgehend von vorgeschriebenen Ausgangswerten der Voraussage $H_i$ als die Differenz zwischen der oder den Messungen Ipi und einem gestörten Modell $Hp_i$ der einen oder mehreren Messungen gemäß der Gleichung $E_i = Ip_i - Hp_i$ berechnet wird, wobei das gestörte Modell $Hp_i$ der einen oder mehreren Messungen die folgenden Gleichungen erfüllt:

$$Hp_i = H_i + P_i$$

$$P_i = \rho_i \frac{jH_i}{|H_i|} e^{ja_i},$$

$$\alpha_i = -arctan(\beta. \omega_i),$$

wo $P_i$ die Störung ist, die zu der oder den Messungen $Ip_i$ durch das magnetfeldstörende Material (3) beigetragen wird, $\rho_i$ die Stärke der Störung ist, $\alpha_i$ ein Phasenverschiebungswinkel ist, der von dem magnetfeldstörenden Material (3) verursacht wird, $\beta$ ein Parameter des magnetfeldstörenden Materials (3) ist, wobei der Parameter $\beta$ für alle Messungen Ipi identisch ist, wobei die Berechnung derart durchgeführt wird, dass das Kriterium (C) minimiert wird, **dadurch gekennzeichnet, dass** bei jeder Iteration

- die Voraussage $H_i$ auf die vorgeschriebenen Ausgangswerte initialisiert wird,
- $\Delta_i$, der Parameter $\beta$ und das gestörte Modell Hpi in Abhängigkeit von Hi und Ipi gemäß den folgenden Gleichungen berechnet werden:

$$\Delta_i = [\mathcal{R}(H_i).\mathcal{I}(Ip_i) - \mathcal{I}(H_i).\mathcal{R}(Ip_i)]/[(\mathcal{I}(H_i)^2 + \mathcal{R}(H_i)^2],$$

$$\beta = \frac{A^T \cdot B}{|A|^2}$$

$$Hp_i = H_i.(1+\Delta_i.(j+ \beta.\omega_i)),$$

wo A ein Vektor ist, dessen erste und zweite Koordinaten wie folgt gebildet sind:

$$A_{2i} = \Delta_i.\mathcal{R}(H_i).\omega_i$$

beziehungsweise

$$A_{2i+1} = \Delta_i.\mathcal{I}(H_i).\omega_i,$$

B ein Vektor ist, dessen erste und zweite Koordinaten wie folgt gebildet sind:

$$B_{2i} = \mathcal{R}(Ip_i) - \mathcal{R}(H_i) + \Delta_i.\mathcal{I}(H_i)$$

beziehungsweise

$$B_{2i+1} = \mathcal{I}(Ip_i) - \mathcal{I}(H_i) - \Delta_i.\mathcal{R}(H_i),$$

für i von 1 bis N, wo

$\mathcal{R}(H_i)$ der Realteil der Voraussage Hi ist,

$\mathcal{I}(H_i)$ der Imaginärteil der Voraussage $H_i$ ist,

$\mathcal{R}(Ip_i)$ der Realteil der Messung $Ip_i$ ist,

$\mathcal{I}(Ip_i)$ der Imaginärteil der Messung $Ip_i$ ist,

- dann der Fehler $E_i$ berechnet wird,
- dann das Kriterium C gemäß der folgenden Gleichung berechnet wird:

$$C = \sum_{i=0}^{N} |E_i|^2,$$

- dann durch das Feldmodell die Voraussage $H_i$ berechnet wird, die dem Kriterium C entspricht,

wobei diese Voraussage $H_i$ für die folgende Iteration verwendet wird, bis das berechnete Kriterium C kleiner als eine vorgeschriebene positive Grenze η von nicht null wird.

3. Verfahren zur Kompensation eines magnetischen Lokalisators (1) beim Vorhandensein mindestens eines magnetfeldstörenden Materials, wobei der magnetische Lokalisator (1) umfasst:

- mindestens einen Sender (10), der mindestens eine (Ne) Sendespule (E1, E2, E3) und mindestens einen Generator (101, 102, 103) mindestens eines Sendesignals umfasst, der mit der mindestens einen Sendespule (E1, E2, E3) verbunden ist, damit die mindestens eine Sendespule (E1, E2, E3) als Reaktion auf das Sendesignal, das ihr vom Generator (101, 102, 103) geschickt wird, mindestens ein Sendemagnetfeld (Be1, Be2, Be3) mit mindestens einer bestimmten Pulsation $\omega_i$ sendet,
- mindestens einen Empfänger (20), der mindestens eine (Nr) Empfangsspule (R1, R2, R3) und eine Messvorrichtung (24) umfasst, die mit der mindestens einen Empfangsspule (R1, R2, R3) verbunden ist und die mindestens eine Messung $Ip_i$ eines magnetischen Empfangsfeldes (Br11, Br21, Br31; Br12, Br22, Br32; Br13, Br23, Br33) liefert, das jeweils von dem Sendemagnetfeld (Be1, Be2, Be3) in jeder Empfangsspule (R1, R2, R3) induziert wird, derart, dass eine oder mehrere Messungen $Ip_i$ für i von 1 bis N, mit N = Nr.Ne, geliefert werden, wobei Ne eine erste Sendespulenanzahl (E1, E2, E3) ist und Nr eine zweite Empfangsspulenanzahl (R1, R2, R3) ist, Ne ≥ 1, Nr ≥ 1,
- eine Verarbeitungseinheit (25), die ein Feldmodell umfasst, das das Berechnen einer Position (P) und/oder einer Ausrichtung (Q) des Empfängers (20) durch Berechnung einer Voraussage $H_i$ der einen oder mehreren Messungen in Abhängigkeit von einem Kriterium (C) ermöglicht, das in Abhängigkeit von einem Fehler $E_i$ berechnet wird, der selbst in Bezug auf die eine oder mehreren Messungen $Ip_i$ berechnet wird,

wobei der Fehler $E_i$ in aufeinanderfolgenden Iterationen ausgehend von vorgeschriebenen Ausgangswerten der Voraussage $H_i$ als die Differenz zwischen der oder den Messungen $Ip_i$ und einem gestörten Modell $Hp_i$ der einen oder mehreren Messungen gemäß der Gleichung $E_i = Ip_i - Hp_i$ berechnet wird,
wobei das gestörte Modell $Hp_i$ der einen oder mehreren Messungen die folgenden Gleichungen erfüllt:

$$Hp_i = H_i + P_i$$

$$P_i = \rho_i \frac{jH_i}{|H_i|} e^{ja_i},$$

$$\alpha_i = -\arctan(\beta \cdot \omega_i),$$

wo $P_i$ die Störung ist, die zu der oder den Messungen $Ip_i$ durch das magnetfeldstörende Material (3) beigetragen wird, $\rho_i$ die Stärke der Störung ist, $\alpha_i$ ein Phasenverschiebungswinkel ist, der von dem magnetfeldstörenden Material (3) verursacht wird, $\beta$ ein Parameter des magnetfeldstörenden Materials (3) ist, wobei der Parameter $\beta$ für alle Messungen $Ip_i$ identisch ist,
wobei die Berechnung derart durchgeführt wird, dass das Kriterium (C) minimiert wird,
**dadurch gekennzeichnet, dass**
bei jeder Iteration

- die Voraussage $H_i$ auf die vorgeschriebenen Ausgangswerte initialisiert wird,
- der Parameter $\beta$ als den Vektor $A'.\beta + B'$ auf seinen Koordinaten minimierend, dann die $\Delta_i$ und das gestörte Modell Hpi in Abhängigkeit von $H_i$ und $Ip_i$ gemäß den folgenden Gleichungen berechnet werden:

$$\Delta_i = [\mathcal{I}(Ip_i) - \mathcal{I}(H_i)]/[\mathcal{R}(H_i).+ \mathcal{I}(H_i).\beta.\omega_i]$$

$$Hp_i = H_i.(1+\Delta_i.(j+ \beta.\omega_i))$$

wo A' ein Vektor ist, dessen Koordinaten jeweils wie folgt gebildet werden:

$$\omega_i.[\mathcal{R}(H_i).\mathcal{I}(Ip_i) - \mathcal{R}(Ip_i).\mathcal{I}(H_i)],$$

B' ein Vektor ist, dessen Koordinaten jeweils wie folgt gebildet werden:

$$\mathcal{R}(H_i)^2 + \mathcal{I}(H_i)^2 - (\mathcal{R}(H_i).\mathcal{R}(Ip_i)+\mathcal{I}(H_i).(Ip_i)),$$

für i von 1 bis N, wo

$\mathcal{R}(H_i)$ der Realteil der Voraussage $H_i$ ist,

$\mathcal{I}(H_i)$ der Imaginärteil der Voraussage $H_i$ ist,

$\mathcal{R}(Ip_i)$ der Realteil der Messung $Ip_i$ ist,

$\mathcal{I}(Ip_i)$ der Imaginärteil der Messung $Ip_i$ ist,

- dann der Fehler $E_i$ berechnet wird,
- dann das Kriterium C gemäß der folgenden Gleichung berechnet wird:

$$C = \sum_{i=0}^{N} |E_i|^2,$$

- dann durch das Feldmodell die Voraussage $H_i$ berechnet wird, die dem Kriterium C entspricht,

wobei diese Voraussage $H_i$ für die folgende Iteration verwendet wird, bis das berechnete Kriterium C kleiner als eine vorgeschriebene positive Grenze $\eta$ von nicht null wird.

4. Verfahren zur Kompensation eines magnetischen Lokalisators (1) beim Vorhandensein mindestens eines magnetfeldstörenden Materials, wobei der magnetische Lokalisator (1) umfasst:

- mindestens einen Sender (10), der mindestens eine (Ne) Sendespule (E1, E2, E3) und mindestens einen Generator (101, 102, 103) mindestens eines Sendesignals umfasst, der mit der mindestens einen Sendespule (E1, E2, E3) verbunden ist, damit die mindestens eine Sendespule (E1, E2, E3) als Reaktion auf das Sendesignal, das ihr vom Generator (101, 102, 103) geschickt wird, mindestens ein Sendemagnetfeld (Be1, Be2, Be3) mit mindestens einer bestimmten Pulsation $\omega_i$ sendet,

- mindestens einen Empfänger (20), der mindestens eine (Nr) Empfangsspule (R1, R2, R3) und eine Messvorrichtung (24) umfasst, die mit der mindestens einen Empfangsspule (R1, R2, R3) verbunden ist und die mindestens eine Messung $Ip_i$ eines magnetischen Empfangsfeldes (Br11, Br21, Br31; Br12, Br22, Br32; Br13, Br23, Br33) liefert, das jeweils von dem Sendemagnetfeld (Be1, Be2, Be3) in jeder Empfangsspule (R1, R2, R3) induziert wird, derart, dass eine oder mehrere Messungen $Ip_i$ für i von 1 bis N, mit N = Nr.Ne, geliefert werden, wobei Ne eine erste Sendespulenanzahl (E1, E2, E3) ist und Nr eine zweite Empfangsspulenanzahl (R1, R2, R3) ist, Ne $\geq$ 1, Nr $\geq$ 1,

- eine Verarbeitungseinheit (25), die ein Feldmodell umfasst, das das Berechnen einer Position (P) und/oder einer Ausrichtung (Q) des Empfängers (20) durch Berechnung einer Voraussage $H_i$ der einen oder mehreren Messungen in Abhängigkeit von einem Kriterium (C) ermöglicht, das in Abhängigkeit von einem Fehler $E_i$ berechnet wird, der selbst in Bezug auf die eine oder mehreren Messungen $Ip_i$ berechnet wird,

wobei der Fehler $E_i$ in aufeinanderfolgenden Iterationen ausgehend von vorgeschriebenen Ausgangswerten der Voraussage $H_i$ als die Differenz zwischen der oder den Messungen $Ip_i$ und einem gestörten Modell $Hp_i$ der einen oder mehreren Messungen gemäß der Gleichung $E_i = Ip_i - Hp_i$ berechnet wird,

wobei das gestörte Modell $Hp_i$ der einen oder mehreren Messungen die folgenden Gleichungen erfüllt:

$$Hp_i = H_i + P_i$$

$$P_i = \rho_i \frac{jH_i}{|H_i|} e^{ja_i},$$

$$\alpha_i = -\arctan(\beta. \omega_i),$$

wo $P_i$ die Störung ist, die zu der oder den Messungen $Ip_i$ durch das magnetfeldstörende Material (3) beigetragen wird, $\rho_i$ die Stärke der Störung ist, $\alpha_i$ ein Phasenverschiebungswinkel ist, der von dem magnetfeldstörenden Material (3) verursacht wird, $\beta$ ein Parameter des magnetfeldstörenden Materials (3) ist, wobei der Parameter $\beta$ für alle Messungen $Ip_i$ identisch ist,

wobei die Berechnung derart durchgeführt wird, dass das Kriterium (C) minimiert wird,

**dadurch gekennzeichnet, dass**

bei jeder Iteration

- die Voraussage $H_i$ auf die vorgeschriebenen Ausgangswerte initialisiert wird,
- der Parameter $\beta$, dann die $\Delta_i$ und das gestörte Modell $Hpi$ in Abhängigkeit von $H_i$ und $Ip_i$ gemäß den folgenden Gleichungen berechnet werden:

$$\beta = \frac{A'^T \cdot B'}{|A'|^2}$$

$$\Delta_i = [\mathcal{I}(Ip_i) - \mathcal{I}(H_i)]/[\mathcal{R}(H_i) + \mathcal{I}(H_i).\beta.\omega_i]$$

$$Hp_i = H_i.(1+\Delta_i.(j+ \beta.\omega_i))$$

wo A' ein Vektor ist, dessen Koordinaten jeweils wie folgt gebildet werden:

$$\omega_i.[\mathcal{R}(H_i).\mathcal{I}(Ip_i) - \mathcal{R}(Ip_i).\mathcal{I}(H_i)],$$

B' ein Vektor ist, dessen Koordinaten jeweils wie folgt gebildet werden:

$$\mathcal{R}(H_i)^2 + \mathcal{I}(H_i)^2 - (\mathcal{R}(H_i).\mathcal{R}(Ip_i) + \mathcal{I}(H_i).(Ip_i)),$$

für i von 1 bis N, wo

$\mathcal{R}(H_i)$ der Realteil der Voraussage $H_i$ ist,

$\mathcal{I}(H_i)$ der Imaginärteil der Voraussage $H_i$ ist,

$\mathcal{R}(Ip_i)$ der Realteil der Messung $Ip_i$ ist,

$\mathcal{I}(Ip_i)$ der Imaginärteil der Messung $Ip_i$ ist,

- dann der Fehler $E_i$ berechnet wird,
- dann das Kriterium C gemäß der folgenden Gleichung berechnet wird:

$$C = \sum_{i=0}^{N} |E_i|^2,$$

- dann durch das Feldmodell die Voraussage $H_i$ berechnet wird, die dem Kriterium C entspricht,

wobei diese Voraussage $H_i$ für die folgende Iteration verwendet wird, bis das berechnete Kriterium C kleiner als eine vorgeschriebene positive Grenze $\eta$ von nicht null wird.

5. Kompensationsverfahren nach einem der Ansprüche 1 bis 4, **dadurch gekennzeichnet, dass** das Feldmodell, das das Berechnen einer Position (P) und/oder einer Ausrichtung (Q) des Empfängers (20) durch Berechnung der Voraussage $H_i$ der einen oder mehreren Messungen in Abhängigkeit von dem Kriterium (C) ermöglicht, einen Levenberg-Marquardt-Minimierungsalgorithmus nutzt.

6. Kompensationsverfahren nach einem der vorhergehenden Ansprüche, **dadurch gekennzeichnet, dass** die bestimmten Pulsatione $\omega_i$ voneinander verschieden sind.

7. Kompensationsverfahren nach einem der vorhergehenden Ansprüche, **dadurch gekennzeichnet, dass** der Sender (10) eine Vielzahl Ne von Sendespulen (E1, E2, E3) umfasst, die jeweils eine Vielzahl Ne von Sendemagnetfeldern (Be1, Be2, Be3) senden, wobei Ne ≥ 2,
wobei die Messvorrichtung (24) für jede Empfangsspule (R1, R2, R3) eine Vielzahl Ne von Empfangsmagnetfeldern (Br11, Br21, Br31; Br12, Br22, Br32; Br13, Br23, Br33) misst, die jeweils von der Vielzahl Ne von Sendemagnetfeldern (Be1, Be2, Be3) in der Empfangsspule (R1, R2, R3) induziert werden und die Messungen $Ip_i$ für i von 1 bis N bilden.

8. Kompensationsverfahren nach einem der vorhergehenden Ansprüche, **dadurch gekennzeichnet, dass** der Lokalisator (1) eine Sendespule (E1, E2, E3), die K unterschiedliche Pulsatione $\omega_{jk}$ emittiert, und J Empfangsspulen (R1, R2, R3) umfasst, wobei die Messvorrichtung (24) die Messungen $Ip_{jk}$ für den Index j von 1 bis J und den Index k von 1 bis K liefert,

wobei die Verarbeitungseinheit (25) berechnet:

$$\rho_j = \frac{\sum_{k=1}^{K} u_{jk} \cdot \left( \left| H_{jk} \right|^2 + \left| Ip_{jk} \right|^2 - 2 \cdot v_{jk} \right)}{\sum_{k=1}^{K} u_{jk}^2}$$

und

$$\beta = \frac{\sum_{j=1}^{J} \sum_{k=1}^{K} u_{jk} \cdot \left( v_{jk} - \left| H_{jk} \right|^2 \right)}{\sum_{j=1}^{J} \sum_{k=1}^{K} u_{jk}^2}$$

mit

$$v_{jk} = \Re(H_{jk}) \cdot \Re(Ip_{jk}) + \Im(H_{jk}) \cdot \Im(Ip_{jk})$$

$$u_{jk} = \left( \Re(H_{jk}) \cdot \Im(Ip_{jk}) - \Im(H_{jk}) \cdot \Re(Ip_{jk}) \right) \cdot \omega_{jk}$$

$$\mathrm{Ip_{jk} = H_{jk}.(1 + \Delta_{jk}.(j + \beta.\omega_{jk}))}$$

$$\Delta_{jk} = \rho_j \frac{\omega_{jk}}{1 + \left( \beta \cdot \omega_{jk} \right)^2}$$

wo $H_{jk}$ die Voraussage ist, $\rho_j$ die Stärke der Störung ist, $Hp_{jk}$ das gestörte Modell ist und $Hp_{jk} - Ip_{jk} = 0$.

9. Kompensationsverfahren nach einem der vorhergehenden Ansprüche, **dadurch gekennzeichnet, dass** der Lokalisator (1) L Sendespulen (E1, E2, E3) oder L Sender (10), die K unterschiedliche Pulsatione $\omega_{jkl}$ senden, und J Empfangsspulen (R1, R2, R3) umfasst, wobei die Messvorrichtung (24) die Messungen $Ip_{jkl}$ für den Index j von 1 bis J, den Index k von 1 bis K und den Index I von 1 bis L liefert,

wobei die Verarbeitungseinheit (25) berechnet:

$$\rho_{jl} = \frac{\sum_{k=1}^{K} u_{jkl} \cdot \left( \left| H_{jkl} \right|^2 + \left| Ip_{jkl} \right|^2 - 2 \cdot v_{jkl} \right)}{\sum_{k=1}^{K} u_{jkl}^2}$$

und

$$\beta_l = \frac{\sum_{j=1}^{J} \sum_{k=1}^{K} u_{jkl} \cdot \left( v_{jkl} - \left| H_{jkl} \right|^2 \right)}{\sum_{j=1}^{J} \sum_{k=1}^{K} u_{jkl}^2}$$

mit

$$v_{jkl} = \Re(H_{jkl}) \cdot \Re(Ip_{jkl}) + \Im(H_{jkl}) \cdot \Im(Ip_{jkl})$$

$$u_{jkl} = \left(\Re(H_{jkl}) \cdot \Im(Ip_{jkl}) - \Im(H_{jkl}) \cdot \Re(Ip_{jkl})\right) \cdot \omega_{jkl}$$

$$Ip_{jkl} = H_{jkl}.(1+\Delta_{jkl}.(j+ \beta.\omega_{jkl}))$$

$$\Delta_{jkl} = \rho_j \frac{\omega_{jkl}}{1+\left(\beta \cdot \omega_{jkl}\right)^2}$$

wo $H_{jkl}$ die Voraussage ist, $\rho_{jl}$ die Stärke der Störung ist, $Hp_{jkl}$ das gestörte Modell ist und $Hp_{jkl} - Ip_{jkl} = 0$.

**10.** Magnetischer Lokalisator (1), der umfasst:

- mindestens einen Sender (10), der mindestens eine (Ne) Sendespule (E1, E2, E3) und mindestens einen Generator (101, 102, 103) umfasst, der geeignet ist, mindestens ein Sendesignal zu erzeugen und der mit der mindestens einen Sendespule (E1, E2, E3) verbunden ist, damit die mindestens eine Sendespule (E1, E2, E3) geeignet ist, als Reaktion auf das Sendesignal, das ihr von dem Generator (101, 102, 103) geschickt wird, mindestens ein Sendemagnetfeld (Be1, Be2, Be3) mit mindestens einer bestimmten Pulsation $\omega_j$ zu senden,
- mindestens einen Empfänger (20), der mindestens eine (Nr) Empfangsspule (R1, R2, R3) und eine Messvorrichtung (24) umfasst, die geeignet ist, mit der mindestens einen Empfangsspule (R1, R2, R3) verbunden zu sein und die geeignet ist, mindestens eine Messung $Ip_i$ eines Empfangsmagnetfelds (Br11, Br21, Br31; Br12, Br22, Br32; Br13, Br23, Br33) zu liefern, das jeweils von dem Sendemagnetfeld (Be1, Be2, Be3) in jeder Empfangsspule (R1, R2, R3) induziert wird, um eine oder mehrere Messungen $Ip_i$ für i von 1 bis N zu liefern, mit N = Nr.Ne, wobei Ne eine erste Sendespulenanzahl (E1, E2, E3) ist und Nr eine zweite Empfangsspulenanzahl (R1, R2, R3), Ne $\geq$ 1, Nr $\geq$ 1 ist,
- eine Verarbeitungseinheit (25), die ein Feldmodell umfasst, das das Berechnen einer Position (P) und/oder einer Ausrichtung (Q) des Empfängers (20) durch Berechnung einer Voraussage $H_i$ der einen oder mehreren Messungen in Abhängigkeit von einem Kriterium (C) ermöglicht, das in Abhängigkeit von einem Fehler $E_i$ berechnet wird, der selber in Bezug auf die eine oder mehreren Messungen $Ip_i$ berechnet wird,

wobei die Verarbeitungseinheit (25) dazu ausgestaltet ist, den Fehler $E_i$ in aufeinanderfolgenden Iterationen ausgehend von vorgeschriebenen Ausgangswerten der Voraussage $H_i$ als die Differenz zwischen der oder den Messungen $Ip_i$ und einem gestörten Modell $Hp_i$ der einen oder mehreren Messungen gemäß der folgenden Gleichung zu berechnen:

$$E_i = Ip_i - Hp_i,$$

wobei das gestörte Modell $Hp_i$ der einen oder mehreren Messungen die folgenden Gleichungen erfüllt:

$$Hp_i = H_i + P_i$$

$$P_i = \rho_i \frac{jH_i}{|H_i|} e^{j\alpha_i},$$

$$\alpha_i = -\arctan(\beta.\ \omega_i),$$

wo $P_i$ eine Störung ist, die zu der oder den Messungen $Ip_i$ durch das magnetfeldstörende Material (3)

beigetragen wird, $\rho_i$ die Stärke der Störung ist, $\alpha_i$ ein Phasenverschiebungswinkel ist, der von dem magnetfeldstörenden Material (3) verursacht wird, $\beta$ ein Parameter des magnetfeldstörenden Materials (3) ist, wobei der Parameter $\beta$ für alle Messungen $Ip_i$ identisch ist,

wobei die Verarbeitungseinheit (25) dazu ausgestaltet ist, die Berechnung derart durchzuführen, dass das Kriterium (C) minimiert wird,

**dadurch gekennzeichnet, dass**

die Verarbeitungseinheit (25) dazu ausgestaltet ist, bei jeder Iteration die folgenden Schritte durchzuführen:

- Initialisieren der Voraussage $H_i$ auf die vorgeschriebenen Werte,
- dann Berechnen von $\Delta_i$, der Parameter $\beta$ als den Vektor A. $\beta$ — B auf seinen Koordinaten minimierend und des gestörten Modells $Hp_i$ in Abhängigkeit von $H_i$ und $Ip_i$ gemäß den folgenden Gleichungen:

$$\Delta_i = [\mathcal{R}(H_i).\mathcal{I}(Ip_i) - \mathcal{I}(H_i).\mathcal{R}(Ip_i)]/[(\mathcal{I}(H_i)^2 + \mathcal{R}(H_i)^2)] \, ,$$

$$Hp_i = H_i.(1 + \Delta_i.(j + \beta.\omega_i)),$$

wo A ein Vektor ist, dessen erste und zweite Koordinaten wie folgt gebildet sind:

$$A_{2i} = \Delta_i.\mathcal{R}(H_i).\omega_i$$

beziehungsweise

$$A_{2i+1} = \Delta_i.\mathcal{I}(H_i).\omega_i \, ,$$

B ein Vektor ist, dessen erste und zweite Koordinaten wie folgt gebildet sind:

$$B_{2i} = \mathcal{R}(Ip_i) - \mathcal{R}(H_i) + \Delta_i.\mathcal{I}(H_i)$$

beziehungsweise

$$B_{2i+1} = \mathcal{I}(Ip_i) - \mathcal{I}(H_i) - \Delta_i.\mathcal{R}(H_i),$$

für i von 1 bis N, wo

$\mathcal{R}(H_i)$ der Realteil der Voraussage $H_i$ ist,

$\mathcal{I}(H_i)$ der Imaginärteil der Voraussage $H_i$ ist,

$\mathcal{R}(Ip_i)$ der Realteil der Messung $Ip_i$ ist,

$\mathcal{I}(Ip_i)$ der Imaginärteil der Messung $Ip_i$ ist,

- dann der Fehler $E_i$ berechnet wird,
- dann das Kriterium C gemäß der folgenden Gleichung berechnet wird:

$$C = \sum_{i=0}^{N} |E_i|^2,$$

- dann durch das Feldmodell die Voraussage $H_i$ berechnet wird, die dem Kriterium C entspricht,

wobei diese Voraussage $H_i$ für die folgende Iteration verwendet wird, bis das berechnete Kriterium C kleiner als eine vorgeschriebene positive Grenze $\eta$ von nicht null wird.

11. Magnetischer Lokalisator (1), der umfasst:

- mindestens einen Sender (10), der mindestens eine (Ne) Sendespule (E1, E2, E3) und mindestens einen Generator (101, 102, 103) umfasst, der geeignet ist, mindestens ein Sendesignal zu erzeugen und der mit der mindestens einen Sendespule (E1, E2, E3) verbunden ist, damit die mindestens eine Sendespule (E1, E2, E3) geeignet ist, als Reaktion auf das Sendesignal, das ihr von dem Generator (101, 102, 103) geschickt wird, mindestens ein Sendemagnetfeld (Be1, Be2, Be3) mit mindestens einer bestimmten Pulsation $\omega_i$ zu senden,
- mindestens einen Empfänger (20), der mindestens eine (Nr) Empfangsspule (R1, R2, R3) und eine Messvorrichtung (24) umfasst, die geeignet ist, mit der mindestens einen Empfangsspule (R1, R2, R3) verbunden zu sein und die geeignet ist, mindestens eine Messung $Ip_i$ eines Empfangsmagnetfelds (Br11, Br21, Br31; Br12, Br22, Br32; Br13, Br23, Br33) zu liefern, das jeweils von dem Sendemagnetfeld (Be1, Be2, Be3) in jeder Empfangsspule (R1, R2, R3) induziert wird, um eine oder mehrere Messungen $Ip_i$ für i von 1 bis N zu liefern, mit N = Nr.Ne, wobei Ne eine erste Sendespulenanzahl (E1, E2, E3) ist und Nr eine zweite Empfangsspulenanzahl (R1, R2, R3), Ne $\geq$ 1, Nr $\geq$ 1 ist,
- eine Verarbeitungseinheit (25), die ein Feldmodell umfasst, das das Berechnen einer Position (P) und/oder einer Ausrichtung (Q) des Empfängers (20) durch Berechnung einer Voraussage $H_i$ der einen oder mehreren Messungen in Abhängigkeit von einem Kriterium (C) ermöglicht, das in Abhängigkeit von einem Fehler $E_i$ berechnet wird, der selber in Bezug auf die eine oder mehreren Messungen $Ip_i$ berechnet wird,

wobei die Verarbeitungseinheit (25) dazu ausgestaltet ist, den Fehler $E_i$ in aufeinanderfolgenden Iterationen ausgehend von vorgeschriebenen Ausgangswerten der Voraussage $H_i$ als die Differenz zwischen der oder den Messungen $Ip_i$ und einem gestörten Modell $Hp_i$ der einen oder mehreren Messungen gemäß der folgenden Gleichung zu berechnen:

$$E_i = Ip_i - Hp_i,$$

wobei das gestörte Modell $Hp_i$ der einen oder mehreren Messungen die folgenden Gleichungen erfüllt:

$$Hp_i = H_i + P_i$$

$$P_i = \rho_i \frac{jH_i}{|H_i|} e^{ja_i},$$

$$\alpha_i = -\arctan(\beta.\ \omega_i\ ),$$

wo $P_i$ die Störung ist, die zu der oder den Messungen $Ip_i$ durch das magnetfeldstörende Material (3) bei-getragen wird, $\rho_i$ die Stärke der Störung ist, $\alpha_i$ ein Phasenverschiebungswinkel ist, der von dem magnetfeldstörenden Material (3) verursacht wird, $\beta$ ein Parameter des magnetfeldstörenden Materials (3) ist, wobei der Parameter $\beta$ für alle Messungen $Ip_i$ identisch ist,
**dadurch gekennzeichnet, dass**
die Verarbeitungseinheit (25) dazu ausgestaltet ist, die Berechnung derart durchzuführen, dass das Kriterium (C) minimiert wird,
die Verarbeitungseinheit (25) dazu ausgestaltet ist, bei jeder Iteration die folgenden Schritte durchzuführen:

- Initialisieren der Voraussage $H_i$ auf die vorgeschriebenen Werte,
- dann Berechnen von $\Delta_i$, der Parameter $\beta$ und des gestörten Modells Hpi in Abhängigkeit von $H_i$ und $Ip_i$ gemäß den folgenden Gleichungen:

$$\Delta_i = [\mathcal{R}(H_i).\mathcal{I}(Ip_i) - \mathcal{I}(H_i).\mathcal{R}(Ip_i)]/[(\mathcal{I}(H_i)^2 + \mathcal{R}(H_i)^2]$$

$$\beta = \frac{A^T \cdot B}{|A|^2}$$

$$Hp_i = H_i.(1+\Delta_i.(j+ \beta.\omega_i)),$$

wo A ein Vektor ist, dessen erste und zweite Koordinaten wie folgt gebildet sind:

$$A_{2i} = \Delta_i.\mathcal{R}(H_i).\omega_i$$

beziehungsweise

$$A_{2i+1} = \Delta_i.\mathcal{I}(H_i).\omega_i,$$

B ein Vektor ist, dessen erste und zweite Koordinaten wie folgt gebildet sind:

$$B_{2i} = \mathcal{R}(Ip_i) - \mathcal{R}(H_i) + \Delta_i.\mathcal{I}(H_i)$$

beziehungsweise

$$B_{2i+1} = \mathcal{I}(Ip_i) - \mathcal{I}(H_i) - \Delta_i.\mathcal{R}(H_i),$$

für i von 1 bis N, wo

$\mathcal{R}(H_i)$ der Realteil der Voraussage $H_i$ ist,

$\mathcal{I}(H_i)$ der Imaginärteil der Voraussage $H_i$ ist,

$\mathcal{R}(Ip_i)$ der Realteil der Messung $Ip_i$ ist,

$\mathcal{I}(Ip_i)$ der Imaginärteil der Messung $Ip_i$ ist,

- dann der Fehler $E_i$ berechnet wird,
- dann das Kriterium C gemäß der folgenden Gleichung berechnet wird:

$$C = \sum_{i=0}^{N}|E_i|^2,$$

- dann durch das Feldmodell die Voraussage $H_i$ berechnet wird, die dem Kriterium C entspricht,

wobei diese Voraussage $H_i$ für die folgende Iteration verwendet wird, bis das berechnete Kriterium C kleiner als eine vorgeschriebene positive Grenze $\eta$ von nicht null wird.

12. Magnetischer Lokalisator (1), der umfasst:

- mindestens einen Sender (10), der mindestens eine (Ne) Sendespule (E1, E2, E3) und mindestens einen Generator (101, 102, 103) umfasst, der geeignet ist, mindestens ein Sendesignal zu erzeugen und der mit der mindestens einen Sendespule (E1, E2, E3) verbunden ist, damit die mindestens eine Sendespule (E1, E2, E3) geeignet ist, als Reaktion auf das Sendesignal, das ihr von dem Generator (101, 102, 103) geschickt wird, mindestens ein Sendemagnetfeld (Be1, Be2, Be3) mit mindestens einer bestimmten Pulsation $\omega_i$ zu senden,
- mindestens einen Empfänger (20), der mindestens eine (Nr) Empfangsspule (R1, R2, R3) und eine Messvorrichtung (24) umfasst, die geeignet ist, mit der mindestens einen Empfangsspule (R1, R2, R3) verbunden zu sein und die geeignet ist, mindestens eine Messung $Ip_i$ eines Empfangsmagnetfelds (Br11, Br21, Br31; Br12, Br22, Br32; Br13, Br23, Br33) zu liefern, das jeweils von dem Sendemagnetfeld (Be1, Be2, Be3) in jeder Empfangsspule (R1, R2, R3) induziert wird, um eine oder mehrere Messungen $Ip_i$ für i von 1 bis N zu liefern, mit N = Nr.Ne, wobei Ne eine erste Sendespulenanzahl (E1, E2, E3) ist und Nr eine zweite Empfangsspulenanzahl (R1, R2, R3), Ne ≥ 1, Nr ≥ 1 ist,
- eine Verarbeitungseinheit (25), die ein Feldmodell umfasst, das das Berechnen einer Position (P) und/oder einer Ausrichtung (Q) des Empfängers (20) durch Berechnung einer Voraussage $H_i$ der einen oder mehreren Messungen in Abhängigkeit von einem Kriterium (C) ermöglicht, das in Abhängigkeit von einem Fehler $E_i$ berechnet wird, der selber in Bezug auf die eine oder mehreren Messungen $Ip_i$ berechnet wird,

wobei die Verarbeitungseinheit (25) dazu ausgestaltet ist, den Fehler $E_i$ in aufeinanderfolgenden Iterationen ausgehend von vorgeschriebenen Ausgangswerten der Voraussage $H_i$ als die Differenz zwischen der oder den Messungen $Ip_i$ und dem gestörten Modell $Hp_i$ der einen oder mehreren Messungen gemäß der folgenden Gleichung zu berechnen:

$$E_i = Ip_i - Hp_i,$$

wobei das gestörte Modell $Hp_i$ der einen oder mehreren Messungen die folgenden Gleichungen erfüllt:

$$Hp_i = H_i + P_i$$

$$P_i = \rho_i \frac{jH_i}{|H_i|} e^{ja_i},$$

$$\alpha_i = -arctan(\beta.\ \omega_i),$$

wo $P_i$ eine Störung ist, die zu der oder den Messungen $Ip_i$ durch das magnetfeldstörende Material (3) beigetragen wird, $\rho_i$ die Stärke der Störung ist, $\alpha_i$ ein Phasenverschiebungswinkel ist, der von dem magnetfeldstörenden Material (3) verursacht wird, $\beta$ ein Parameter des magnetfeldstörenden Materials (3) ist, wobei der Parameter $\beta$ für alle Messungen $Ip_i$ identisch ist,
wobei die Verarbeitungseinheit (25) dazu ausgestaltet ist, die Berechnung derart durchzuführen, dass das Kriterium (C) minimiert wird,
**dadurch gekennzeichnet, dass**
die Verarbeitungseinheit (25) dazu ausgestaltet ist, bei jeder Iteration die folgenden Schritte durchzuführen:

- Initialisieren der Voraussage $H_i$ auf die vorgeschriebenen Werte,
- dann Berechnen des Parameters $\beta$ als den Vektor A'.β + B' auf seinen Koordinaten minimierend, dann der $\Delta_i$ und des gestörten Modells $Hp_i$ in Abhängigkeit von $H_i$ und $Ip_i$ gemäß den folgenden Gleichungen:

$$\Delta_i = [\mathcal{I}(Ip_i) - \mathcal{I}(H_i)]/[\mathcal{R}(H_i). + \mathcal{I}(H_i).\beta.\omega_i]$$

$$Hp_i = H_i.(1+\Delta_i.(j+ \beta.\omega_i))$$

wobei A' ein Vektor ist, dessen Koordinaten jeweils wie folgt gebildet sind:

$$\omega_i.[\mathcal{R}(H_i).\mathcal{I}(Ip_i) - \mathcal{R}(Ip_i).\mathcal{I}(H_i)] \ ,$$

B' ein Vektor ist, dessen Koordinaten jeweils wie folgt gebildet sind:

$$\mathcal{R}(H_i)^2 + \mathcal{I}(H_i)^2 - (\mathcal{R}(H_i).\mathcal{R}(Ip_i) + \mathcal{I}(H_i).(Ip_i)),$$

für i von 1 bis N, wobei

$\mathcal{R}(H_i)$ der Realteil der Voraussage $H_i$ ist,

$\mathcal{I}(H_i)$ der Imaginärteil der Voraussage $H_i$ ist,

$\mathcal{R}(Ip_i)$ der Realteil der Messung $Ip_i$ ist,

$\mathcal{I}(Ip_i)$ der Imaginärteil der Messung $Ip_i$ ist,

- dann der Fehler $E_i$ berechnet wird,
- dann das Kriterium C gemäß der folgenden Gleichung berechnet wird:

$$C = \sum_{i=0}^{N} |E_i|^2,$$

- dann durch das Feldmodell die Voraussage $H_i$ berechnet wird, die dem Kriterium C entspricht,

wobei diese Voraussage $H_i$ für die folgende Iteration verwendet wird, bis das berechnete Kriterium C kleiner als eine vorgeschriebene positive Grenze η von nicht null wird.

13. Magnetischer Lokalisator (1), der umfasst:

- mindestens einen Sender (10), der mindestens eine (Ne) Sendespule (E1, E2, E3) und mindestens einen Generator (101, 102, 103) umfasst, der geeignet ist, mindestens ein Sendesignal zu erzeugen und der mit der mindestens einen Sendespule (E1, E2, E3) verbunden ist, damit die mindestens eine Sendespule (E1, E2, E3) geeignet ist, als Reaktion auf das Sendesignal, das ihr von dem Generator (101, 102, 103) geschickt wird, mindestens ein Sendemagnetfeld (Be1, Be2, Be3) mit mindestens einer bestimmten Pulsation $\omega_i$ zu senden,
- mindestens einen Empfänger (20), der mindestens eine (Nr) Empfangsspule (R1, R2, R3) und eine Messvorrichtung (24) umfasst, die geeignet ist, mit der mindestens einen Empfangsspule (R1, R2, R3) verbunden zu sein und die geeignet ist, mindestens eine Messung $Ip_i$ eines Empfangsmagnetfelds (Br11, Br21, Br31; Br12, Br22, Br32; Br13, Br23, Br33) zu liefern, das jeweils von dem Sendemagnetfeld (Be1, Be2, Be3) in jeder Empfangsspule (R1, R2, R3) induziert wird, um mehrere Messungen $Ip_i$ für i von 1 bis N zu liefern, mit N = Nr.Ne, wobei Ne eine erste Sendespulenanzahl (E1, E2, E3) ist und Nr eine zweite Empfangsspulenanzahl (R1, R2, R3), Ne ≥ 1, Nr ≥ 1 ist,
- eine Verarbeitungseinheit (25), die ein Feldmodell umfasst, das das Berechnen einer Position (P) und/oder einer Ausrichtung (Q) des Empfängers (20) durch Berechnung einer Voraussage $H_i$ der einen oder mehreren Messungen in Abhängigkeit von einem Kriterium (C) ermöglicht, das in Abhängigkeit von einem Fehler $E_i$ berechnet wird, der selber in Bezug auf die eine oder mehreren Messungen $Ip_i$ berechnet wird,

wobei die Verarbeitungseinheit (25) dazu ausgestaltet ist, den Fehler $E_i$ in aufeinanderfolgenden Iterationen ausgehend von vorgeschriebenen Ausgangswerten der Voraussage $H_i$ als die Differenz zwischen der oder den Messungen $Ip_i$ und dem gestörten Modell $Hp_i$ der einen oder mehreren Messungen gemäß der folgenden Gleichung zu berechnen:

$$E_i \ = \ Ip_i \ - \ Hp_i,$$

wobei das gestörte Modell $Hp_i$ der einen oder mehreren Messungen die folgenden Gleichungen erfüllt:

$$Hp_i = H_i + P_i$$

$$P_i = \rho_i \frac{jH_i}{|H_i|} e^{ja_i},$$

$$\alpha_i = -\arctan(\beta \cdot \omega_i),$$

wo $P_i$ eine Störung ist, die zu der oder den Messungen $Ip_i$ durch ein magnetfeldstörendes Material (3) beigetragen wird, $\rho_i$ die Stärke der Störung ist, $\alpha_i$ ein Phasenverschiebungswinkel ist, der von dem magnetfeldstörenden Material (3) verursacht wird, $\beta$ ein Parameter des magnetfeldstörenden Materials (3) ist, wobei der Parameter $\beta$ für alle Messungen $Ip_i$ identisch ist,
wobei die Verarbeitungseinheit (25) dazu ausgestaltet ist, die Berechnung derart durchzuführen, dass das Kriterium (C) minimiert wird,
**dadurch gekennzeichnet, dass**
die Verarbeitungseinheit (25) dazu ausgestaltet ist, bei jeder Iteration die folgenden Schritte durchzuführen:

- Initialisieren der Voraussage $H_i$ auf die vorgeschriebenen Werte,
- dann Berechnen der Parameter $\beta$, dann der $\Delta_i$ und des gestörten Modells Hpi in Abhängigkeit von $H_i$ und $Ip_i$ gemäß den folgenden Gleichungen:

$$\beta = \frac{A'^T \cdot B'}{|A'|^2}$$

$$\Delta_i = [\mathcal{I}(Ip_i) - \mathcal{I}(H_i)]/[\mathcal{R}(H_i) + \mathcal{I}(H_i).\beta.\omega i]$$

$$Hp_i = H_i.(1+\Delta_i.(j+ \beta.\omega_i))$$

wobei A' ein Vektor ist, dessen Koordinaten jeweils wie folgt gebildet sind:

$$\omega_i.[\mathcal{R}(H_i).\mathcal{I}(Ip_i) - \mathcal{R}(Ip_i).\mathcal{I}(H_i)] ,$$

B' ein Vektor ist, dessen Koordinaten jeweils wie folgt gebildet sind:

$$\mathcal{R}(H_i)^2 + \mathcal{I}(H_i)^2 - (\mathcal{R}(H_i).\mathcal{R}(Ip_i)+\mathcal{I}(H_i).(Ip_i)),$$

für i von 1 bis N, wobei

$\mathcal{R}(H_i)$ der Realteil der Voraussage $H_i$ ist,

$\mathcal{I}(H_i)$ der Imaginärteil der Voraussage $H_i$ ist,

$\mathcal{R}(Ip_i)$ der Realteil der Messung $Ip_i$ ist,

$\mathcal{I}(Ip_i)$ der Imaginärteil der Messung $Ip_i$ ist,

- dann der Fehler $E_i$ berechnet wird,
- dann das Kriterium C gemäß der folgenden Gleichung berechnet wird:

$$C = \sum_{i=0}^{N} |E_i|^2,$$

- dann durch das Feldmodell die Voraussage $H_i$ berechnet wird, die dem Kriterium C entspricht,

wobei diese Voraussage $H_i$ für die folgende Iteration verwendet wird, bis das berechnete Kriterium C kleiner als eine vorgeschriebene positive Grenze $\eta$ von nicht null wird.

14. Magnetischer Lokalisator (1) nach einem der Ansprüche 10 bis 13, **dadurch gekennzeichnet, dass** das Feldmodell, das das Berechnen einer Position (P) und/oder einer Ausrichtung (Q) des Empfängers (20) durch Berechnung der Voraussage $H_i$ der einen oder mehreren Messungen in Abhängigkeit von dem Kriterium (C) ermöglicht, einen Levenberg-Marquardt-Minimierungsalgorithmus nutzt.

15. Computerprogramm, das Codeanweisungen umfasst, die bewirken, dass der magnetische Lokalisator nach einem der Ansprüche 10 bis 14 die Schritte des Verfahrens zur Kompensation des magnetischen Lokalisators (1) bei Vorhandensein eines magnetfeldstörenden Materials nach einem der Ansprüche 1 bis 9 ausführt, wenn das Computerprogrammprodukt auf einem Rechner ausgeführt wird.

**Claims**

1. A method for compensating a magnetic locator (1) in the presence of at least one magnetic-field-disrupting material, said magnetic locator (1) comprising:

    - at least one transmitter (10) comprising at least one (Ne) transmitter coil (E1, E2, E3) and at least one generator (101, 102, 103) of at least one transmission signal, connected to the at least one transmitter coil (E1, E2, E3), so that the at least one transmitter coil (E1, E2, E3) transmits at least one transmitting magnetic field (Bel, Be2, Be3) at at least one determined pulsation $\omega_i$ in response to the transmission signal that is sent to it by the generator (101, 102, 103),
    - at least one receiver (20) comprising at least one (Nr) receiver coil (R1, R2, R3) and a measuring device (24), which is connected to the at least one receiver coil (R1, R2, R3) and which supplies at least one measurement $Ip_i$ of a receiving magnetic field (Br11, Br21, Br31; Br12, Br22, Br32; Br13, Br23, Br33) respectively induced by the transmitting magnetic field (Bel, Be2, Be3) in each receiver coil (R1, R2, R3), in such a way as to supply one or several measurements $Ip_i$ for i ranging from 1 to N, with N=Ne.Nr, where Ne is a first number of transmitter coil (E1, E2, E3), Nr is a second number of receiver coil (R1, R2, R3), Ne ≥ 1, Nr ≥ 1,
    - a processing unit (25) comprising a field model making it possible to compute a position (P) and/or an orientation (Q) of the receiver (20) by computing a prediction $H_i$ of the measurement or of the measurements as a function of a criterion (C ) computed as a function of an error $E_i$, itself computed with respect to the measurement or the measurements $Ip_i$,

    wherein the error $E_i$ is computed by successive iterations from initial prescribed values of the prediction $H_i$ as being the difference between the measurement or the measurements $Ip_i$ and a disrupted model $Hp_i$ of the measurement or of the measurements, according to the equation

$$E_i = Ip_i - Hp_i,$$

    the disrupted model $Hp_i$ of the the measurement or of the measurements verifying the following equations

$$Hp_i = H_i + P_i,$$

$$P_i = \rho_i \frac{jH_i}{|H_i|} e^{j\alpha_i} \quad,$$

$$\alpha_i = -\arctan(\beta.\,\omega_i)\,,$$

where $P_i$ is the disruption made to the measurement or the measurements $Ip_i$ by the magnetic-field-disrupting material (3), $\rho_i$ is the intensity of the disruption, $\alpha_i$ is a phase shift angle caused by the magnetic-field-disrupting material (3), $\beta$ being a parameter of the magnetic-field-disrupting material (3),
the parameter $\beta$ being identical for all the measurements $Ip_i$,
the computation being carried out in such a way as to minimize the criterion (C ),
**characterized in that**
at each iteration

- the prediction $H_i$ is initialized at the prescribed initial values,
- then are computed $\Delta_i$, the parameter $\beta$ so as to minimize the vector A.$\beta$ — B over its coordinates, and the disrupted model $Hp_i$ as a function of $H_i$ and $Ip_i$ according to the equations

$$\Delta_i = [\Re(H_i).\Im(Ip_i) - \Im(H_i).\Re(Ip_i)]/[(\Im(H_i)^2 + \Re(H_i)^2] \,,$$

$$Hp_i = H_i.(1+\Delta_i.(j+ \beta.\omega_i)) \,,$$

where A is a vector, the first and second coordinates of which are respectively formed by:

$$A_{2i} = \Delta_i.\Re(H_i).\omega_i$$

and

$$A_{2i+1} = \Delta_i.\Im(H_i).\omega_i \,,$$

B is a vector, the first and second coordinates of which are respectively formed by:

$$B_{2i} = \Re(Ip_i) - \Re(H_i) + \Delta_i.\Im(H_i)$$

and

$$B_{2i+1} = \Im(Ip_i) - \Im(H_i) - \Delta_i.\Re(H_i),$$

for i ranging from 1 to N, where

$\Re(H_i)$ is the real part of the prediction $H_i$,

$\Im(H_i)$ is the imaginary part of the prediction $H_i$,

$\Re(Ip_i)$ is the real part of the measurement $Ip_i$,

$\Im(Ip_i)$ is the imaginary part of the measurement $Ip_i$,

- then the error $E_i$ is computed,
- then the criterion C is computed according to the equation

$$C = \sum_{i=0}^{N} \left| E_i \right|^2 \, ,$$

- then the prediction Hi corresponding to the criterion C is computed by the field model,

this computed prediction $H_i$ being used for the following iteration until the computed criterion C becomes less than a prescribed non-zero positive bound $\eta$.

2. A method for compensating a magnetic locator (1) in the presence of at least one magnetic-field-disrupting material, said magnetic locator (1) comprising:

- at least one transmitter (10) comprising at least one (Ne) transmitter coil (E1, E2, E3) and at least one generator (101, 102, 103) of at least one transmission signal, connected to the at least one transmitter coil (E1, E2, E3), so that the at least one transmitter coil (E1, E2, E3) transmits at least one transmitting magnetic field (Bel, Be2, Be3) at at least one determined pulsation $\omega_i$ in response to the transmission signal that is sent to it by the generator (101, 102, 103),
- at least one receiver (20) comprising at least one (Nr) receiver coil (R1, R2, R3) and a measuring device (24), which is connected to the at least one receiver coil (R1, R2, R3) and which supplies at least one measurement $Ip_i$ of a receiving magnetic field (Br11, Br21, Br31; Br12, Br22, Br32; Br13, Br23, Br33) respectively induced by the transmitting magnetic field (Bel, Be2, Be3) in each receiver coil (R1, R2, R3), in such a way as to supply one or several measurements $Ip_i$ for i ranging from 1 to N, with N=Ne.Nr, where Ne is a first number of transmitter coil (E1, E2, E3), Nr is a second number of receiver coil (R1, R2, R3), Ne $\geq$ 1, Nr $\geq$ 1,
- a processing unit (25) comprising a field model making it possible to compute a position (P) and/or an orientation (Q) of the receiver (20) by computing a prediction $H_i$ of the measurement or of the measurements as a function of a criterion (C ) computed as a function of an error $E_i$, itself computed with respect to the measurement or the measurements $Ip_i$,

wherein the error $E_i$ is computed by successive iterations from initial prescribed values of the prediction $H_i$ as being the difference between the measurements $Ip_i$ and a disrupted model $Hp_i$ of the measurement or of the measurements, according to the equation

$$E_i = Ip_i - Hp_i,$$

the disrupted model $Hp_i$ of the measurement or of the measurements verifying the following equations

$$Hp_i = H_i + P_i,$$

$$P_i = \rho_i \frac{jH_i}{\left|H_i\right|} e^{j\alpha_i} \, ,$$

$$\alpha_i = -\arctan(\beta. \omega_i),$$

where $P_i$ is the disruption made to the measurement or the measurements $Ip_i$ by the magnetic-field-disrupting material (3), $\rho_i$ is the intensity of the disruption, $\alpha_i$ is a phase shift angle caused by the magnetic-field-disrupting material (3), $\beta$ being a parameter of the magnetic-field-disrupting material (3), the parameter $\beta$ being identical for all the measurements $Ip_i$, the computation being carried out in such a way as to minimize the criterion (C ),
**characterized in that**
at each iteration

- the prediction $H_i$ is initialized at the prescribed initial values,

- then are computed $\Delta_i$, the parameter $\beta$ and the disrupted model $Hp_i$ as a function of $H_i$ and $Ip_i$ according to the equations

$$\Delta_i = [\Re(H_i).\Im(Ip_i) - \Im(H_i).\Re(Ip_i)]/[(\Im(H_i)^2 + \Re(H_i)^2] ,$$

$$\beta = \frac{A^T \cdot B}{\left| A \right|^2}$$

$$Hp_i = H_i.(1+\Delta_i.(j+ \beta.\omega_i)) ,$$

where A is a vector, the first and second coordinates of which are respectively formed by:

$$A_{2i} =\Delta_i.\Re(H_i).\omega_i$$

and

$$A_{2i+1}=\Delta_i.\Im(H_i).\omega_i ,$$

B is a vector, the first and second coordinates of which are respectively formed by:

$$B_{2i} = \Re(Ip_i) - \Re(H_i) + \Delta_i.\Im(H_i)$$

and

$$B_{2i+1} = \Im(Ip_i) - \Im(H_i) - \Delta_i.\Re(H_i),$$

for i ranging from 1 to N, where

$\Re(H_i)$ is the real part of the prediction $H_i$,

$\Im(H_i)$ is the imaginary part of the prediction $H_i$,

$\Re(Ip_i)$ is the real part of the measurement $Ip_i$,

$\Im(Ip_i)$ is the imaginary part of the measurement $Ip_i$.

- then the error $E_i$ is computed,
- then the criterion C is computed according to the equation

$$C = \sum_{i=0}^{N} \left| E_i \right|^2 ,$$

- then the prediction Hi corresponding to the criterion C is computed by the field model,

this computed prediction $H_i$ being used for the following iteration until the computed criterion C becomes less than a prescribed non-zero positive bound $\eta$.

3. A method for compensating a magnetic locator (1) in the presence of at least one magnetic-field-disrupting material, said magnetic locator (1) comprising:

- at least one transmitter (10) comprising at least one (Ne) transmitter coil (E1, E2, E3,) and at least one generator (101, 102, 103) of at least one transmission signal, connected to the at least one transmitter coil (E1, E2, E3), so that the at least one transmitter coil (E1, E2, E3) transmits at least one transmitting magnetic field (Bel, Be2, Be3) at at least one determined pulsation $\omega_i$ in response to the transmission signal that is sent to it by the generator (101, 102, 103),

- at least one receiver (20) comprising at least one (Nr) receiver coil (R1, R2, R3) and a measuring device (24), which is connected to the at least one receiver coil (R1, R2, R3) and which supplies at least one measurement $Ip_i$ of a receiving magnetic field (Br11, Br21, Br31; Br12, Br22, Br32; Br13, Br23, Br33) respectively induced by the transmitting magnetic field (Bel, Be2, Be3) in each receiver coil (R1, R2, R3), in such a way as to supply one or several measurements $Ip_i$ for i ranging from 1 to N, with N=Ne.Nr, where Ne is a first number of transmitter coil (E1, E2, E3), Nr is a second number of receiver coil (R1, R2, R3), Ne $\geq$ 1, Nr $\geq$ 1,

- a processing unit (25) comprising a field model making it possible to compute a position (P) and/or an orientation (Q) of the receiver (20) by computing a prediction $H_i$ of the measurement or of the measurements as a function of a criterion (C ) computed as a function of an error $E_i$, itself computed with respect to the measurement or the the measurements $Ip_i$,

wherein the error $E_i$ is computed by successive iterations from initial prescribed values of the prediction $H_i$ as being the difference between the measurement or the measurements $Ip_i$ and a disrupted model $Hp_i$ of the measurement or of the measurements, according to the equation

$$E_i = Ip_i - Hp_i,$$

the disrupted model $Hp_i$ of the measurement or the measurements verifying the following equations

$$Hp_i = H_i + P_i,$$

$$P_i = \rho_i \frac{jH_i}{|H_i|} e^{j\alpha_i} \ ,$$

$$\alpha_i = -\arctan(\beta. \omega_i ),$$

where $P_i$ is the disruption made to the measurement or the measurements $Ip_i$ by the magnetic-field-disrupting material (3), $\rho_i$ is the intensity of the disruption, $\alpha_i$ is a phase shift angle caused by the magnetic-field-disrupting material (3), $\beta$ being a parameter of the magnetic-field-disrupting material (3), the parameter $\beta$ being identical for all the measurements $Ip_i$, the computation being carried out in such a way as to minimize the criterion (C ), **characterized in that** at each iteration

- the prediction $H_i$ is initialized at the prescribed initial values,
- then are computed the parameter $\beta$ so as to minimize the vector A'.$\beta$+B' over its coordinates, then the $\Delta_i$ and the disrupted model $Hp_i$ as a function of $H_i$ and $Ip_i$ according to the equations:

$$\Delta_i = (\Im(Ip_i) - \Im(H_i))/( \Re(H_i) + \Im(H_i)\beta\omega_i)$$

$$Hp_i = H_i.(1 + \Delta_i.(j + \beta.\omega_i))$$

where A' is a vector, the coordinates of which are respectively formed by:

$$\omega_i.[\Re(H_i).\Im(Ip_i) - \Re(Ip_i).\Im(H_i)] ,$$

B' is a vector, the coordinates of which are respectively formed by:

$$\Re(H_i)^2 + \Im(H_i)^2 - (\Re(H_i).\Re(Ip_i)+\Im(H_i).(Ip_i)),$$

for i ranging from 1 to N, where

$\Re(H_i)$ is the real part of the prediction $H_i$,

$\Im(H_i)$ is the imaginary part of the prediction $H_i$,

$\Re(Ip_i)$ is the real part of the measurement $Ip_i$,

$\Im(Ip_i)$ is the imaginary part of the measurement $Ip_i$,

- then the error $E_i$ is computed,
- then the criterion C is computed according to the equation

$$C = \sum_{i=0}^{N} |E_i|^2 ,$$

- then the prediction $H_i$ corresponding to the criterion C is computed by the field model,

this computed prediction $H_i$ being used for the following iteration until the computed criterion C becomes less than a prescribed non-zero positive bound $\eta$.

4. A method for compensating a magnetic locator (1) in the presence of at least one magnetic-field-disrupting material, said magnetic locator (1) comprising:

- at least one transmitter (10) comprising at least one (Ne) transmitter coil (E1, E2, E3,) and at least one generator (101, 102, 103) of at least one transmission signal, connected to the at least one transmitter coil (E1, E2, E3), so that the at least one transmitter coil (E1, E2, E3) transmits at least one transmitting magnetic field (Bel, Be2, Be3) at at least one determined pulsation $\omega_i$ in response to the transmission signal that is sent to it by the generator (101, 102, 103),
- at least one receiver (20) comprising at least one (Nr) receiver coil (R1, R2, R3) and a measuring device (24), which is connected to the at least one receiver coil (R1, R2, R3) and which supplies at least one measurement $Ip_i$ of a receiving magnetic field (Br11, Br21, Br31; Br12, Br22, Br32; Br13, Br23, Br33) respectively induced by the transmitting magnetic field (Bel, Be2, Be3) in each receiver coil (R1, R2, R3), in such a way as to supply one or several measurements $Ip_i$ for i ranging from 1 to N, with N=Ne.Nr, where Ne is a first number of transmitter coil (E1, E2, E3), Nr is a second number of receiver coil (R1, R2, R3), Ne $\geq$ 1, Nr $\geq$ 1,
- a processing unit (25) comprising a field model making it possible to compute a position (P) and/or an orientation (Q) of the receiver (20) by computing a prediction $H_i$ of the measurement or the measurements as a function of a criterion (C ) computed as a function of an error $E_i$, itself computed with respect to the measurement or the measurements $Ip_i$,

wherein the error $E_i$ is computed by successive iterations from initial prescribed values of the prediction $H_i$ as being the difference between the measurement or the measurements $Ip_i$ and a disrupted model $Hp_i$ of the measurement or of the measurements, according to the equation

$$E_i = Ip_i - Hp_i,$$

the disrupted model Hpi of the measurement or of the measurements verifying the following equations

$$Hp_i = H_i + P_i,$$

$$P_i = \rho_i \frac{jH_i}{|H_i|} e^{j\alpha_i} \quad,$$

$$\alpha_i = -\arctan(\beta.\,\omega_i\,),$$

where $P_i$ is the disruption made to the measurement or the measurements $Ip_i$ by the magnetic-field-disrupting material (3), $\rho_i$ is the intensity of the disruption, $\alpha_i$ is a phase shift angle caused by the magnetic-field-disrupting material (3), $\beta$ being a parameter of the magnetic-field-disrupting material (3),
the parameter $\beta$ being identical for all the measurements $Ip_i$,
the computation being carried out in such a way as to minimize the criterion (C ),
**characterized in that**
at each iteration

- the prediction $H_i$ is initialized at the prescribed initial values,
- then are computed the parameter $\beta$ then the $\Delta_i$ and the disrupted model $Hp_i$ as a function of $H_i$ and $Ip_i$ according to the equations:

$$\beta = \frac{A'^T \cdot B'}{|A'|^2}$$

$$\Delta_i = (\Im(Ip_i) - \Im(H_i))/(\,\Re(H_i) + \Im(H_i)\beta\omega_i)$$

$$Hp_i = H_i.(1+\Delta_i.(j+ \beta.\omega_i))$$

where A' is a vector, the coordinates of which are respectively formed by:

$$\omega_i.[\Re(H_i).\Im(Ip_i) - \Re(Ip_i).\Im(H_i)]\ ,$$

B' is a vector, the coordinates of which are respectively formed by:

$$\Re(H_i)^2 + \Im(H_i)^2 - (\Re(H_i).\Re(Ip_i)+\Im(H_i).(Ip_i)),$$

for i ranging from 1 to N, where

$\Re(H_i)$ is the real part of the prediction $H_i$,

$\Im(H_i)$ is the imaginary part of the prediction $H_i$,

$\Re(Ip_i)$ is the real part of the measurement $Ip_i$,

$\Im(Ip_i)$ is the imaginary part of the measurement $Ip_i$,

- then the error $E_i$ is computed,
- then the criterion C is computed according to the equation

$$C = \sum_{i=0}^{N} \left| E_i \right|^2 ,$$

- then the prediction Hi corresponding to the criterion C is computed by the field model,

this computed prediction $H_i$ being used for the following iteration until the computed criterion C becomes less than a prescribed non-zero positive bound $\eta$.

5. The compensating method as claimed in any one of claims 1 to 4, **characterized in that** the field model making it possible to compute a position (P) and/or an orientation (Q) of the receiver (20) by computing the prediction $H_i$ of the measurement or of the measurements as a function of the criterion (C ) uses a Levenberg-Marquardt minimization algorithm.

6. The compensating method as claimed in any one of the preceding claims, **characterized in that** the determined pulsations $\omega_i$ are separate from one another.

7. The compensating method as claimed in any one of the preceding claims, **characterized in that** the transmitter (10) comprises a plurality Ne of transmitter coils (E1, E2, E3) respectively transmitting a plurality Ne of transmitting magnetic fields (Bel, Be2, Be3), with Ne $\geq$ 2,
the measuring device (24) measuring for each receiver coil (R1, R2, R3) a plurality Ne of receiving magnetic fields (Br11, Br21, Br31; Br12, Br22, Br32; Br13, Br23, Br33) respectively induced by the plurality Ne of transmitting magnetic fields (Bel, Be2, Be3) in the receiver coil (R1, R2, R3) and forming the measurements $Ip_i$ for i ranging from 1 N.

8. The compensating method as claimed in any one of the preceding claims, **characterized in that** the locator (1) comprises a transmitter coil (E1, E2, E3) transmitting at K different pulsations $\omega_{jk}$ and J receiver coils (R1, R2, R3), the measuring device (24) supplying the measurements $Ip_{jk}$ for the index j ranging from 1 to J and the index k ranging from 1 to K,

the processing unit (25) computes

$$\rho_j = \frac{\sum_{k=1}^{K} u_{jk} \cdot \left( \left| H_{jk} \right|^2 + \left| Ip_{jk} \right|^2 - 2 \cdot v_{jk} \right)}{\sum_{k=1}^{K} u_{jk}^2}$$

and

$$\beta = \frac{\sum_{j=1}^{J} \sum_{k=1}^{K} u_{jk} \cdot \left( v_{jk} - \left| H_{jk} \right|^2 \right)}{\sum_{j=1}^{J} \sum_{k=1}^{K} u_{jk}^2}$$

with

$$v_{jk} = \Re(H_{jk}).\Re(Ip_{jk}) + \Im(H_{jk}) \, \Im(Ip_{jk})$$

$$u_{jk} = (\Re(H_{jk}).\Im(Ip_{jk}) - \Im(H_{jk}).\Re(Ip_{jk})).\omega_{jk}$$

$$Ip_{jk} = H_{jk}.(1+\Delta_{jk}.(j+ \beta.\omega_{jk}))$$

$$\Delta_{jk} = \rho_j \frac{\omega_{jk}}{1+\left(\beta \cdot \omega_{jk}\right)^2}$$

where $H_{jk}$ is the prediction, $\rho_j$ is the intensity of the disruption, $Hp_{jk}$ is the disrupted model and $Hp_{jk} - Ipjk = 0$.

9. The compensating method as claimed in any one of the preceding claims, **characterized in that** the locator (1) comprises L transmitter coils (E1, E2, E3) or L transmitters (10), which transmit at K different pulsations $\omega_{jkl}$, and J receiver coils (R1, R2, R3), the measuring device (24) supplying the measurements $Ip_{jkl}$ for the index j ranging from 1 to J, the index k ranging from 1 to K and the index 1 ranging from 1 to L,

the processing unit (25) computes

$$\rho_{jl} = \frac{\sum_{k=1}^{K} u_{jkl} \cdot \left(\left|H_{jkl}\right|^2 + \left|Ip_{jkl}\right|^2 - 2 \cdot v_{jkl}\right)}{\sum_{k=1}^{K} u_{jkl}^2}$$

and

$$\beta_l = \frac{\sum_{j=1}^{J}\sum_{k=1}^{K} u_{jkl} \cdot \left(v_{jkl} - \left|H_{jkl}\right|^2\right)}{\sum_{j=1}^{J}\sum_{k=1}^{K} u_{jkl}^2}$$

with

$$v_{jkl} = \Re(H_{jkl}).\Re(Ip_{jkl}) + \Im(H_{jkl}) \Im(Ip_{jkl})$$

$$u_{jkl} = (\Re(H_{jkl}).\Im(Ip_{jkl}) - \Im(H_{jkl}).\Re(Ip_{jkl})).\omega_{jkl}$$

$$Ip_{jkl} = H_{jkl}.(1+\Delta_{jkl}.(j+ \beta.\omega_{jkl}))$$

$$\Delta_{jkl} = \rho_j \frac{\omega_{jkl}}{1+\left(\beta \cdot \omega_{jkl}\right)^2}$$

where $H_{jkl}$ is the prediction, $\rho_{jl}$ is the intensity of the disruption, $Hp_{jkl}$ is the disrupted model and $Hp_{jkl} - Ip_{jkl} = 0$.

10. A magnetic locator (1) comprising:

- at least one transmitter (10) comprising at least one (Ne) transmitter coil (E1, E2, E3) and at least one generator (101, 102, 103) able to generate at least one transmission signal, connected to the at least one transmitter coil (E1, E2, E3), so that the at least one transmitter coil (E1, E2, E3) is able to transmit at least one transmitting magnetic field (Bel, Be2, Be3) at at least one determined pulsation $\omega_i$ in response to the transmission signal that is sent to it by the generator (101, 102, 103),

- at least one receiver (20) comprising at least one (Nr) receiver coil (R1, R2, R3) and a measuring device (24), which is able to be connected to the at least one receiver coil (R1, R2, R3) and which is able to supply at least one measurement Ipi of a receiving magnetic field (Br11, Br21, Br31; Br12, Br22, Br32; Br13, Br23, Br33) respectively induced by the transmitting magnetic field (Bel, Be2, Be3) in each receiver coil (R1, R2, R3), to supply one or several measurements $Ip_i$ for i ranging from 1 to N, with N=Ne.Nr, where Ne is a first number of transmitter coil (E1, E2, E3), Nr is a second number of receiver coil (R1, R2, R3), Ne $\geq$ 1, Nr $\geq$ 1,

- a processing unit (25) comprising a field model making it possible to compute a position (P) and/or an orientation (Q) of the receiver (20) by computing a prediction $H_i$ of the measurement or of the measurements as a function of a criterion (C ) computed as a function of an error $E_i$, itself computed with respect to the measurement or the measurements $Ip_i$,

wherein the processing unit (25) is configured so that the error $E_i$ is computed by successive iterations from initial prescribed values of the prediction $H_i$ as being the difference between the measurement or the measurements $Ip_i$ and a disrupted model $Hp_i$ of the measurement or of the measurements, according to the equation

$$E_i = Ip_i - Hp_i,$$

the disrupted model Hpi of the measurements verifying the following equations

$$Hp_i= H_i+P_i,$$

$$P_i = \rho_i \frac{jH_i}{|H_i|} e^{j\alpha_i} \ ,$$

$$\alpha_i = -\arctan(\beta. \omega_i ),$$

where $P_i$ is a disruption made to the measurement or the measurements $Ip_i$ by a magnetic-field-disrupting material (3), $\rho_i$ is the intensity of the disruption, $\alpha_i$ is a phase shift angle caused by the magnetic-field-disrupting material (3), $\beta$ being a parameter of the magnetic-field-disrupting material (3),

the parameter $\beta$ being identical for all the measurements $Ip_i$,

the processing unit (25) being configured to carry out the computation in such a way as to minimize the criterion (C ),

**characterized in that**

the processing unit (25) is configured to implement the following steps at each iteration:

- the prediction $H_i$ is initialized at the prescribed initial values,
- then are computed $\Delta_i$, the parameter $\beta$ so as to minimize the vector A.$\beta$ — B over its coordinates, and the disrupted model $Hp_i$ as a function of $H_i$ and $Ip_i$ according to the equations:

$$\Delta_i = [\Re(H_i).\Im(Ip_i) - \Im(H_i).\Re(Ip_i)]/[(\Im(H_i)^2 + \Re(H_i)^2] \ ,$$

$$Hp_i = H_i.(1+\Delta_i.(j+ \beta.\omega_i)) \ ,$$

where A is a vector, the first and second coordinates of which are respectively formed by:

$$A_{2i} =\Delta_i.\Re(H_i).\omega_i \ and \ A_{2i+1}=\Delta_i.\Im(H_i).\omega_i \ ,$$

B is a vector, the first and second coordinates of which are respectively formed by:

$$B_{2i} = \Re(Ip_i) - \Re(H_i) + \Delta_i.\Im(H_i)$$

and

$$B_{2i+1} = \Im(Ip_i) - \Im(H_i) - \Delta_i.\Re(H_i),$$

for i ranging from 1 to N, where

$\Re(H_i)$ is the real part of the prediction $H_i$,

$\Im(H_i)$ is the imaginary part of the prediction $H_i$,

$\Re(Ip_i)$ is the real part of the measurement $Ip_i$,

$\Im(Ip_i)$ is the imaginary part of the measurement $Ip_i$,

- then the error $E_i$ is computed,
- then the criterion C is computed according to the equation

$$C = \sum_{i=0}^{N} |E_i|^2 \;,$$

- then the prediction $H_i$ corresponding to the criterion C is computed by the field model,

this computed prediction $H_i$ being used for the following iteration until the computed criterion C becomes less than a prescribed non-zero positive bound $\eta$.

11. A magnetic locator (1) comprising:

- at least one transmitter (10) comprising at least one (Ne) transmitter coil (E1, E2, E3) and at least one generator (101, 102, 103) able to generate at least one transmission signal, connected to the at least one transmitter coil (E1, E2, E3), so that the at least one transmitter coil (E1, E2, E3) is able to transmit at least one transmitting magnetic field (Bel, Be2, Be3) at at least one determined pulsation $\omega_i$ in response to the transmission signal that is sent to it by the generator (101, 102, 103),
- at least one receiver (20) comprising at least one (Nr) receiver coil (R1, R2, R3) and a measuring device (24), which is able to be connected to the at least one receiver coil (R1, R2, R3) and which is able to supply at least one measurement $Ip_i$ of a receiving magnetic field (Br11, Br21, Br31; Br12, Br22, Br32; Br13, Br23, Br33) respectively induced by the transmitting magnetic field (Bel, Be2, Be3) in each receiver coil (R1, R2, R3), to supply one or several measurements $Ip_i$ for i ranging from 1 to N, with N=Ne.Nr, where Ne is a first number of transmitter coil (E1, E2, E3), Nr is a second number of receiver coil (R1, R2, R3), Ne $\geq$ 1, Nr $\geq$ 1,
- a processing unit (25) comprising a field model making it possible to compute a position (P) and/or an orientation (Q) of the receiver (20) by computing a prediction $H_i$ of the measurement or of the measurements as a function of a criterion (C) computed as a function of an error $E_i$, itself computed with respect to the measurement or the measurements $Ip_i$,

wherein the processing unit (25) is configured so that the error $E_i$ is computed by successive iterations from prescribed initial values of the prediction $H_i$ as being the difference between the measurement or the measurements $Ip_i$ and a disrupted model $Hp_i$ of the measurement or of the measurements, according to the equation

$$E_i = Ip_i - Hp_i,$$

the disrupted model Hpi of the measurement or of the measurements verifying the following equations

$$Hp_i = H_i + P_i,$$

$$P_i = \rho_i \frac{jH_i}{|H_i|} e^{j\alpha_i} \quad ,$$

$$\alpha_i = -\arctan(\beta . \omega_i),$$

where $P_i$ is a disruption made to the measurement or the measurements $Ip_i$ by a magnetic-field-disrupting material (3), $\rho_i$ is the intensity of the disruption, $\alpha_i$ is a phase shift angle caused by the magnetic-field-disrupting material (3), $\beta$ being a parameter of the magnetic-field-disrupting material (3),
the parameter $\beta$ being identical for all the measurements $Ip_i$,
**characterized in that**
the processing unit (25) is configured to carry out the computation in such a way as to minimize the criterion (C),
the processing unit (25) being configured to implement the following steps at each iteration:

- the prediction $H_i$ is initialized at the prescribed initial values,
- then are computed $\Delta_i$, the parameter $\beta$ and the disrupted model $Hp_i$ as a function of $H_i$ and $Ip_i$ according to the equations

$$\Delta_i = [\Re(H_i).\Im(Ip_i) - \Im(H_i).\Re(Ip_i)]/[(\Im(H_i)^2 + \Re(H_i)^2] ,$$

$$\beta = \frac{A^T \cdot B}{|A|^2}$$

$$Hp_i = H_i.(1+\Delta_i.(j+ \beta.\omega_i)) \quad ,$$

where A is a vector, the first and second coordinates of which are respectively formed by:

$$A_{2i} = \Delta_i.\Re(H_i).\omega_i$$

and

$$A_{2i+1} = \Delta_i.\Im(H_i).\omega_i ,$$

B is a vector, the first and second coordinates of which are respectively formed by:

$$B_{2i} = \Re(Ip_i) - \Re(H_i) + \Delta_i.\Im(H_i)$$

and

$$B_{2i+1} = \mathfrak{I}(Ip_i) - \mathfrak{I}(H_i) - \Delta_i.\mathfrak{R}(H_i),$$

for i ranging from 1 to N, where

$\mathfrak{R}(H_i)$ is the real part of the prediction $H_i$,

$\mathfrak{I}(H_i)$ is the imaginary part of the prediction $H_i$,

$\mathfrak{R}(Ip_i)$ is the real part of the measurement $Ip_i$,

$\mathfrak{I}(Ip_i)$ is the imaginary part of the measurement $Ip_i$.

- then the error $E_i$ is computed,
- then the criterion C is computed according to the equation

$$C = \sum_{i=0}^{N} \left| E_i \right|^2,$$

- then the prediction $H_i$ corresponding to the criterion C is computed by the field model,

this computed prediction $H_i$ being used for the following iteration until the computed criterion C becomes less than a prescribed non-zero positive bound $\eta$.

12. A magnetic locator (1) comprising:

- at least one transmitter (10) comprising at least one (Ne) transmitter coil (E1, E2, E3) and at least one generator (101, 102, 103) able to generate at least one transmission signal, connected to the at least one transmitter coil (E1, E2, E3), so that the at least one transmitter coil (E1, E2, E3) is able to transmit at least one transmitting magnetic field (Bel, Be2, Be3) at at least one determined pulsation $\omega_i$ in response to the transmission signal that is sent to it by the generator (101, 102, 103),
- at least one receiver (20) comprising at least one (Nr) receiver coil (R1, R2, R3) and a measuring device (24), which is able to be connected to the at least one receiver coil (R1, R2, R3) and which is able to supply at least one measurement $Ip_i$ of a receiving magnetic field (Br11, Br21, Br31; Br12, Br22, Br32; Br13, Br23, Br33) respectively induced by the transmitting magnetic field (Bel, Be2, Be3) in each receiver coil (R1, R2, R3), to supply one or several measurements $Ip_i$ for i ranging from 1 to N, with N=Ne.Nr, where Ne is a first number of transmitter coil (E1, E2, E3), Nr is a second number of receiver coil (R1, R2, R3), Ne $\geq$ 1, Nr $\geq$ 1,
- a processing unit (25) comprising a field model making it possible to compute a position (P) and/or an orientation (Q) of the receiver (20) by computing a prediction $H_i$ of the measurement or of the measurements as a function of a criterion (C) computed as a function of an error $E_i$, itself computed with respect to the measurement or the measurements $Ip_i$,

wherein the processing unit (25) is configured so that the error $E_i$ is computed by successive iterations from prescribed initial values of the prediction $H_i$ as being the difference between the measurement or the measurements $Ip_i$ and a disrupted model $Hp_i$ of the measurement or of the measurements, according to the equation

$$E_i = Ip_i - Hp_i,$$

the disrupted model Hpi of the measurement or of the measurements verifying the following equations

$$Hp_i = H_i + P_i,$$

$$P_i = \rho_i \frac{jH_i}{|H_i|} e^{j\alpha_i} \quad,$$

$$\alpha_i = -\arctan(\beta. \omega_i ),$$

where $P_i$ is a disruption made to the measurement or the measurements $Ip_i$ by a magnetic-field-disrupting material (3), $\rho_i$ is the intensity of the disruption, $\alpha_i$ is a phase shift angle caused by the magnetic-field-disrupting material (3), $\beta$ being a parameter of the magnetic-field-disrupting material (3), the parameter $\beta$ being identical for all the measurements $Ip_i$,
**characterized in that**
the processing unit (25) is configured to carry out the computation in such a way as to minimize the criterion (C ),
the processing unit (25) being configured to implement the following steps at each iteration:

- the prediction $H_i$ is initialized at the prescribed initial values,
- then are computed the parameter $\beta$ so as to minimize the vector A'.$\beta$+B' over its coordinates, then the $\Delta_i$ and the disrupted model $Hp_i$ as a function of $H_i$ and $Ip_i$ according to the equations:

$$\Delta_i = (\Im(Ip_i) - \Im(H_i))/( \Re(H_i) + \Im(H_i)\beta\omega_i)$$

$$Hp_i = H_i.(1+\Delta_i.(j+ \beta.\omega_i))$$

where A' is a vector, the coordinates of which are respectively formed by:

$$\omega_i.[\Re(H_i).\Im(Ip_i) - \Re(Ip_i).\Im(H_i)] \ ,$$

B' is a vector, the coordinates of which are respectively formed by:

$$\Re(H_i)^2 + \Im(H_i)^2 - (\Re(H_i).\Re(Ip_i)+\Im(H_i).(Ip_i)),$$

for i ranging from 1 to N, where

$\Re(H_i)$ is the real part of the prediction $H_i$,

$\Im(H_i)$ is the imaginary part of the prediction $H_i$,

$\Re(Ip_i)$ is the real part of the measurement $Ip_i$,

$\Im(Ip_i)$ is the imaginary part of the measurement $Ip_i$,

- then the error $E_i$ is computed,
- then the criterion C is computed according to the equation

$$C = \sum_{i=0}^{N} |E_i|^2 \ ,$$

- then the prediction $H_i$ corresponding to the criterion C is computed by the field model,

this computed prediction $H_i$ being used for the following iteration until the computed criterion C becomes less than a prescribed non-zero positive bound η.

13. A magnetic locator (1) comprising:

- at least one transmitter (10) comprising at least one (Ne) transmitter coil (E1, E2, E3) and at least one generator (101, 102, 103) able to generate at least one transmission signal, connected to the at least one transmitter coil (E1, E2, E3), so that the at least one transmitter coil (E1, E2, E3) is able to transmit at least one transmitting magnetic field (Bel, Be2, Be3) at at least one determined pulsation $\omega_i$ in response to the transmission signal that is sent to it by the generator (101, 102, 103),

- at least one receiver (20) comprising at least one (Nr) receiver coil (R1, R2, R3) and a measuring device (24), which is able to be connected to the at least one receiver coil (R1, R2, R3) and which is able to supply at least one measurement $Ip_i$ of a receiving magnetic field (Br11, Br21, Br31; Br12, Br22, Br32; Br13, Br23, Br33) respectively induced by the transmitting magnetic field (Bel, Be2, Be3) in each receiver coil (R1, R2, R3), to supply several measurements $Ip_i$ for i ranging from 1 to N, with N=Ne.Nr, where Ne is a first number of transmitter coil (E1, E2, E3), Nr is a second number of receiver coil (R1, R2, R3), Ne ≥ 1, Nr ≥ 1,

- a processing unit (25) comprising a field model making it possible to compute a position (P) and/or an orientation (Q) of the receiver (20) by computing a prediction $H_i$ of the measurement or of the measurements as a function of a criterion (C ) computed as a function of an error $E_i$, itself computed with respect to the measurement or the measurements $Ip_i$,

wherein the processing unit (25) is configured so that the error $E_i$ is computed by successive iterations from prescribed initial values of the prediction $H_i$ as being the difference between the measurement or the measurements $Ip_i$ and a disrupted model $Hp_i$ of the measurement or of the measurements, according to the equation

$$E_i = Ip_i - Hp_i,$$

the disrupted model $Hp_i$ of the measurement or of the measurements verifying the following equations

$$Hp_i = H_i + P_i,$$

$$P_i = \rho_i \frac{jH_i}{|H_i|} e^{j\alpha_i} \quad ,$$

$$\alpha_i = -\arctan(\beta . \omega_i ),$$

where $P_i$ is a disruption made to the measurement or the measurements $Ip_i$ by a magnetic-field-disrupting material (3), $\rho_i$ is the intensity of the disruption, $\alpha_i$ is a phase shift angle caused by the magnetic-field-disrupting material (3), β being a parameter of the magnetic-field-disrupting material (3),

the parameter β being identical for all the measurements $Ip_i$,

the processing unit (25) being configured to carry out the computation in such a way as to minimize the criterion (C ),

**characterized in that**

the processing unit (25) is configured to implement the following steps at each iteration:

- the prediction $H_i$ is initialized at the prescribed initial values,

- then are computed the parameter β then the $\Delta_i$ and the disrupted model $Hp_i$ as a function of $H_i$ and $Ip_i$ according to the equations:

$$\beta = \frac{A'^{T} \cdot B'}{|A'|^2}$$

$$\Delta_i = (\mathfrak{J}(Ip_i) - \mathfrak{J}(H_i))/(\mathfrak{R}(H_i) + \mathfrak{J}(H_i)\beta\omega_i)$$

$$Hp_i = H_i.(1+\Delta_i.(j+ \beta.\omega_i))$$

where A' is a vector, the coordinates of which are respectively formed by:

$$\omega_i.[\mathfrak{R}(H_i).\mathfrak{J}(Ip_i) - \mathfrak{R}(Ip_i).\mathfrak{J}(H_i)] \ ,$$

B' is a vector, the coordinates of which are respectively formed by:

$$\mathfrak{R}(H_i)^2 + \mathfrak{J}(H_i)^2 - (\mathfrak{R}(H_i).\mathfrak{R}(Ip_i)+\mathfrak{J}(H_i).(Ip_i)),$$

for i ranging from 1 to N, where

$\mathfrak{R}(H_i)$ is the real part of the prediction $H_i$,

$\mathfrak{J}(H_i)$ is the imaginary part of the prediction $H_i$,

$\mathfrak{R}(Ip_i)$ is the real part of the measurement $Ip_i$,

$\mathfrak{J}(Ip_i)$ is the imaginary part of the measurement $Ip_i$,

- then the error $E_i$ is computed,
- then the criterion C is computed according to the equation

$$C = \sum_{i=0}^{N} |E_i|^2 \ ,$$

- then the prediction $H_i$ corresponding to the criterion C is computed by the field model,

this computed prediction $H_i$ being used for the following iteration until the computed criterion C becomes less than a prescribed non-zero positive bound $\eta$.

**14.** The magnetic locator (1) as claimed in any one of claims 10 to 13, **characterized in that** the field model making it possible to compute a position (P) and/or an orientation (Q) of the receiver (20) by computing the prediction $H_i$ of the measurement or of the measurements as a function of the criterion (C ) uses a Levenberg-Marquardt minimization algorithm.

**15.** A computer program, comprising code instructions which cause the magnetic locator as claimed in any one of claims 10-14 for implementing the method for compensating the magnetic locator (1) in the presence of at least one magnetic-field-disrupting material as claimed in any one of claims 1 to 9, when the computer program is executed on a calculator.

FIG. 1

# FIG. 2

10

20

CF

3

EP 3 710 869 B1

FIG. 3

Im

Bc

Bp

Re

Ferro-magnétique

FIG. 4

Im

Bp

Bc

Bs

Re

Courants de Foucault

FIG. 5

Im

B'p

Bp

Bc

Bs

Re

Ferro-magnétique
+
courants de Foucault

EP 3 710 869 B1

# FIG. 6

# FIG. 7

## FIG. 8

1

10,20

$$Syst\grave{e}me\ EM$$

26 $\eta$ 29 24

$$Mod\grave{e}le\ de\ champ\ P, Q$$

$$Optimisation\ C = \sum_{i=0}^{N} |E_i|^2$$

$$Dispositif\ de\ mesure$$

$$H_i$$

$$Calcul\ de\ l'erreur\ E_i = Ip_i - Hp_i$$ 27

$$Ip_i$$ 25

28

$$Calcul\ de\ la\ projection\ \beta,\ Hp_i$$

# FIG. 9

Etat du capteur
$P, Q$

26

$\downarrow \eta$

Modèle
de champ

Optimisation
$C = \sum_{i=0}^{N} |E_i|^2$

29

24

Dispositif
de mesure

$H_i$

Calcul de l'erreur
$E_i = Ip_i - Hp_i$

27

$Ip_i$

Calcul de la projection

$$\beta = \frac{A^T B}{|A|}$$

$$\Delta_i = \frac{\Re(H_i) \cdot \Im(Ip_i) - \Im(H_i) \cdot \Re(Ip_i)}{\Im(H_i)^2 + \Re(H_i)^2}$$

28

$$Hp_i = H_i(1 + \Delta_i(j + \beta \cdot \omega_i))$$

## FIG. 10

$$\textit{Etat du capteur}$$
$$P, Q$$

26

$$\eta$$

$$\textit{Modèle}$$
$$\textit{de champ}$$

29

$$\textit{Optimisation}$$
$$C = \sum_{i=0}^{N} |E_i|^2$$

24

$$\textit{Dispositif}$$
$$\textit{de mesure}$$

$$H_i$$

$$\textit{Calcul de l'erreur}$$
$$E_i = Ip_i - Hp_i$$

27

$$Ip_i$$

$$\textit{Calcul de la projection}$$

$$\beta = \frac{A'^{T} B'}{|A'|}$$

$$\Delta_i = \frac{\Im(Ip_i) - \Im(H_i)}{(\Re(H_i) + \Im(H_i)\beta \cdot \omega_i)}$$

$$Hp_i = H_i(1 + \Delta_i(j + \beta \cdot \omega_i))$$

28

## FIG. 11

```
                    ┌─────────────────────┐
                    │   Etat du capteur   │
                    │      P, Q, β        │
                    └─────────────────────┘
```

$$Etat\ du\ capteur$$
$$P, Q, \beta$$

26

$\downarrow \eta$

29

24

**Modèle de champ**

**Optimisation**
$$C = \sum_{i=0}^{N} |E_i|^2$$

**Dispositif de mesure**

$H_i$

**Calcul de l'erreur**
$$E_i = Ip_i - Hp_i$$

27

$Ip_i$

**Calcul de la projection**

$$\Delta = C^+ D$$

$$Hp_i = H_i(1 + \Delta_i(j + \beta \cdot \omega_i))$$

28

70

**EP 3 710 869 B1**

## RÉFÉRENCES CITÉES DANS LA DESCRIPTION

**Documents brevets cités dans la description**

- US 2011004430 A1